# EUROPÄISCHE PATENTSCHRIFT

(11) **EP 0 796 855 B1**
(45) Veröffentlichungstag und Bekanntmachung des Hinweises auf die Patenterteilung: **06.02.2002**
(21) Anmeldenummer: 97103712.2
(22) Anmeldetag: 06.03.1997
(51) Int. Cl.: C07D 403/12, A61K 31/415, A61K 31/41, C07D 233/76, A61K 31/505, C07D 471/04, C07D 491/04

(54) **Inhibitoren der Knochenresorption und Vitronectinrezeptor-Antagonisten**
Inhibitors of bone resorption and vitronectin receptor antagonists
Inhibiteurs de la résorption osseuse et antagonistes du récepteur de la vitronectine

(30) Priorität: 20.03.1996 DE 19610919; 03.07.1996 DE 19626701; 02.09.1996 DE 19635522
(43) Veröffentlichungstag der Anmeldung: 24.09.1997
(73) Patentinhaber: HOECHST AKTIENGESELLSCHAFT, 65929 Frankfurt am Main (DE); Genentech Inc., South San Francisco, CA 94080-4990 (US)
(72) Erfinder: Wehner, Volkmar, Dr., 97657 Sandberg (DE); Knolle, Jochen, Dr., 65830 Kriftel (DE); Stilz, Hans Ulrich, Dr., 65929 Frankfurt (DE); Carniato, Denis, Dr., 91460 Marcoussis (FR); Gourvest, Jean-François, 77410 Claye Souilly (FR); Gadek, Tom, Dr., Oakland, CA 94611 (US); McDowell, Robert, Dr., San Francisco, CA 94114 (US)
(74) Vertreter: Rousseau, Pierrick Edouard

(56) Entgegenhaltungen:
- EP-A- 0 449 079
- EP-A- 0 528 586
- EP-A- 0 528 587
- EP-A- 0 530 505
- EP-A- 0 566 919
- EP-A- 0 584 694
- WO-A-93/18057
- WO-A-95/14008
- WO-A-95/28426
- WO-A-96/00574
- DE-A- 4 301 747
- US-A- 5 424 293

## Beschreibung

Gegenstand der vorliegenden Erfindung sind Verbindungen der Formel I sowie deren physiologisch verträglichen Salze und solche Verbindungen enthaltende pharmazeutische Zubereitungen, deren Herstellung und Verwendung als Heilmittel, insbesondere als Inhibitoren der Knochenresorption durch Osteoclasten, als Inhibitoren von Tumorwachstum und Tumormetastasierung, als Entzündungshemmer, zur Behandlung oder Prophylaxe von cardiovaskulären Erkrankungen wie Arteriosklerose oder Restenose, zur Behandlung oder Prophylaxe von Nephropatien und Retinopathien, wie z.B. diabetischer Retinopathie, sowie als Vitronectinrezeptor-Antagonisten zur Behandlung und Prophylaxe von Krankheiten, die auf der Wechselwirkung zwischen Vitronectinrezeptoren und deren Liganden bei Zell-Zell- oder Zell-Matrix-Interaktionsprozessen beruhen. Die Erfindung betrifft ferner die Verwendung der Verbindungen der Formel I sowie deren physiologisch verträglichen Salze und solche Verbindungen enthaltende pharmazeutische Zubereitungen als Heilmittel zur Linderung oder Heilung von Krankheiten, die zumindest teilweise durch ein unerwünschtes Maß an Knochenresorption, Angiogenese, oder Proliferation von Zellen der glatten Gefäßmuskulatur bedingt sind.

Die menschlichen Knochen unterliegen einem fortwährenden dynamischen Umbauprozeß, der Knochenresorption und Knochenaufbau beinhaltet. Diese Prozesse werden von dafür spezialisierten Zelltypen gesteuert. Knochenaufbau beruht auf der Ablagerung von Knochenmatrix durch Osteoblasten, Knochenresorption beruht auf dem Abbau von Knochenmatrix durch Osteoclasten. Die Mehrzahl der Knochenerkrankungen beruhen auf einem gestörten Gleichgewicht zwischen Knochenbildung und Knochenresorption.

Osteoporose ist charakterisiert durch einen Verlust an Knochenmatrix. Aktivierte Osteoclasten sind vielkernige Zellen mit einem Durchmesser bis zu 400 µm, die Knochenmatrix abtragen. Aktivierte Osteoclasten lagern sich an die Oberfläche der Knochenmatrix an und sezernieren proteolytische Enzyme und Säuren in die sogenannte "sealing zone", dem Bereich zwischen ihrer Zellmembran und der Knochenmatrix. Die saure Umgebung und die Proteasen bewirken den Abbau des Knochens.

Die erfindungsgemäßen Verbindungen der Formel I inhibieren die Knochenresorption durch Osteoclasten. Knochenkrankheiten, gegen die die erfindungsgemäßen Verbindungen eingesetzt werden können, sind vor allem Osteoporose, Hypercalcämie, Osteopenie, z.B. hervorgerufen durch Metastasen, Zahnerkrankungen, Hyperparathyroidismus, periarticulare Erosionen bei rheumathoider Arthritis und Paget Krankheit.
Ferner können die Verbindungen der Formel I zur Linderung, Vermeidung oder Therapie von Knochenerkrankungen, die durch eine Glucocortikoid-, Sterorid- oder Corticosteroid-Therapie oder durch einen Mangel an Sexualhormon(en) hervorgerufen werden, eingesetzt werden. Alle diese Erkrankungen sind durch Knochenverlust gekennzeichnet, der auf dem Ungleichgewicht zwischen Knochenaufbau und Knochenabbau beruht.

Studien haben gezeigt, daß die Anlagerung von Osteoclasten an den Knochen durch Integrin-Rezeptoren auf der Zelloberfläche von Osteoclasten gesteuert wird.

Integrine sind eine Superfamilie von Rezeptoren zu denen unter anderen der Fibrinogenrezeptor α_{IIb}β₃ auf den Blutplättchen und der Vitronectinrezeptor αᵥβ₃ gehören. Der Vitronectinrezeptor αᵥβ₃ ist ein membranständiges Glycoprotein, das auf der Zelloberfläche einer Reihe von Zellen wie Endothelzellen, Zellen der glatten Gefäßmuskulatur, Osteoclasten und Tumorzellen exprimiert wird. Der Vitronectinrezeptor α_{V}β₃, der auf der Osteoclastenmembran exprimiert wird, steuert den Prozeß der Anlagerung an den Knochen und der Knochenresorption und trägt somit zur Osteoporose bei.

α_{V}β₃ bindet hierbei an Knochenmatrixproteine wie Osteopontin, Knochensialoprotein und Thrombospontin, die das Tripeptidmotif Arg-Gly-Asp (oder RGD) enthalten.

Horton und Mitarbeiter beschreiben RGD-Peptide und einen anti-Vitronectinrezeptor Antikörper (23C6), die den Zahnabbau durch Osteoclasten und das Wandern von Osteoclasten inhibieren (Horton et al., Exp. Cell. Res. 1991, 195, 368). Sato et al. beschreiben in J. Cell Biol. 1990, 111, 1713 Echistatin, ein RGD-Peptid aus Schlangengift, als potenten Inhibitor der Knochenresorption in einer Gewebekultur und als Hemmstoff der Osteoclasten-Anheftung an den Knochen. Fischer et al. (Endocrinology, 1993, 132, 1411) konnten an der Ratte zeigen, daß Echistatin die Knochenresorption auch in vivo hemmt.

Der Vitronectinrezeptor α_{V}β₃ auf humanen Zellen der glatten Gefäßmuskulatur der Aorta stimuliert die Wanderung dieser Zellen in das Neointima, was schließlich zu Arteriosklerose und Restenose nach Angioplastie führt (Brown et al., Cardiovascular Res. 1994, 28, 1815).

Die Verbindungen der Formel I können ferner als Träger von Wirkstoffen dienen, um den Wirkstoff gezielt an den Wirkort zu transportieren (= Drug Targeting, siehe z.B. Targeted Drug Detivery, R.C. Juliano, Handbook of Experimental Pharmacology Vol. 100, Ed. Born, G.V.R. et al., Springer Verlag). Bei den Wirkstoffen handelt es sich um solche, die zur Behandlung der obengenannten Krankheiten verwendet werden können.

Brooks et al. (Cell 1994, 79, 1157) zeigten, daß Antikörper gegen α_{V}β₃ oder α_{V}β₃-Antagonisten eine Schrumpfung von Tumoren bewirken können, indem sie die Apoptose von Blutgefäßzellen während der Angiogenese induzieren. Chersh et al. (Science 1995, 270, 1500) beschreiben anti α_{V}β₃-Antikörper oder α_{V}β₃-Antagonisten, die bFGF induzierte Angiogeneseprozesse im Rattenauge inhibieren was therapeutisch bei der Behandlung von Retinopathien nützlich sein könnte.

In der EP-A 449 079, der EP-A 530 505, der EP-A 566 919, der WO 93/18057 sind Hydantoinderivate, in der WO 95/14008 substituierte 5-Ring-Heterocyclen beschrieben, die thrombozytenaggregationshemmende Wirkungen aufweisen.

In der Patentanmeldung WO 94/12181 werden substituierte aromatische oder nichtaromatische Ringsysteme, in WO 94/08577 substituierte Heterocyclen als Fibrinogenrezeptor-Antagonisten und Inhibitoren der Plättchenaggregation beschrieben. Aus EP-A-528 586 und EP-A-528 587 sind Aminoalkyl- oder Heterocyclyl-substituierte Phenylalanin-Derivate, aus WO 95/32710 Arylderivate als Hemmstoffe der Knochenresorption durch Osteoclasten bekannt. In WO 95/28426 werden RGD-Peptide als Inhibitoren der Knochenresorption, Angiogenese und Restenose beschrieben. Die WO 96/00574 beschreibt Benzodiazepine, die WO 96/00730 Fibrinogenrezeptorantagonisten-Template, insbesondere Benzodiazepine, die an einen Stickstoff tragenden 5-Ring geknüpft sind, als Vitronectinrezeptor-Antagonisten.

Gegenstand der vorliegenden Erfindung sind 5-Ring-Heterocyclen der allgemeinen Formel I, 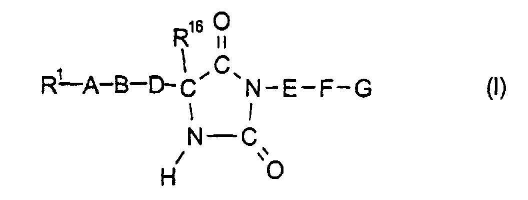 worin bedeuten:
- A: eine direkte Bindung, (C₁-C₄)-Alkandiyl, -NR²-N=CR²-, -NR²-C(O)-NR²-, -NR²-C(O)O-, -NR²-S(O)ₙ-, -NR²-S(O)ₙ-NR²-, -NR²-CO-, -NR²- oder -N=CR², die jeweils durch NH und/oder einfach oder zweifach durch (C₁-C₄)-Alkandiyl substituiert sein können;
- B: eine direkte Bindung, (C₁-C₄)-Alkandiyl, Phenylen, einen zweiwertigen Rest des Pyridins, Thiophens oder Furans, Cyclohexandiyl, -C≡C-, NR²-C(O)-,-C(O)-NR²-, -CR²=CR³-, die jeweils ein- oder zweifach durch (C₁-C₄)-Alkandiyl substituiert sein können,
- D: eine direkte Bindung, (C₁-C₄)-Alkandiyl, Phenylen, -O-, -NR²-, -NR²-CO-, -NR²-C(O)-NR²-, -NR²-S(O)₂-NR²-, -NR²-S(O)-, -NR²-S(O)₂-, -N=CR², -R²C=N-, die jeweils ein- oder zweifach durch (C₁-C₄)-Alkandiyl,
- E: eine direkte Bindung oder (C₁-C₄)-Alkandiyl;
- F: eine direkte Bindung, (C₁-C₆)-Alkandiyl, -O-, -CO-NR²-, -NR²-CO-, -NR²-C(O)-NR²-, -S(O)₂-NR²-, -NR²-S(O)₂-, -CR²=CR³-, -C≡C-, -N=CR²- oder -R²C=N-, die jeweils einfach oder zweifach durch (C₁-C₄)-Alkandiyl substituiert sein können;
- G: 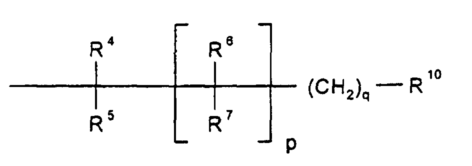
- R¹: R²R³N-C(= NR²)-, 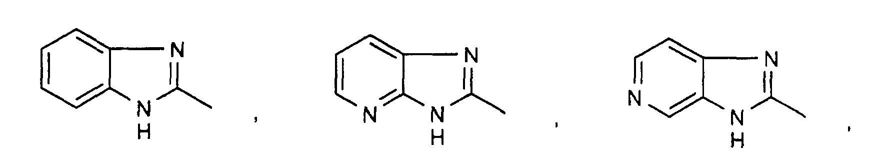 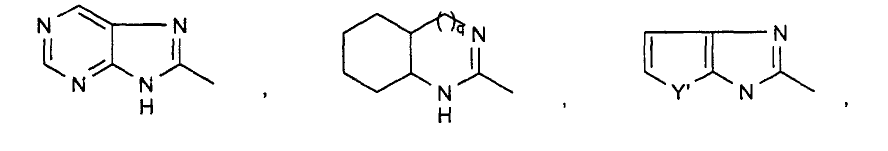 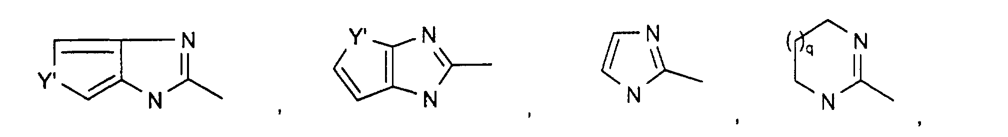 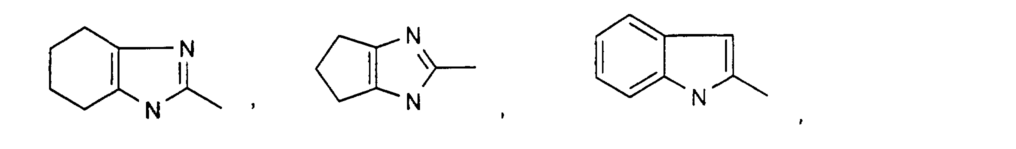 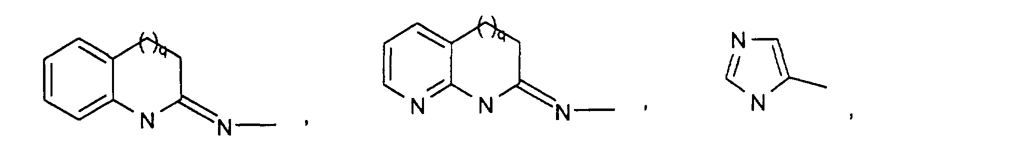 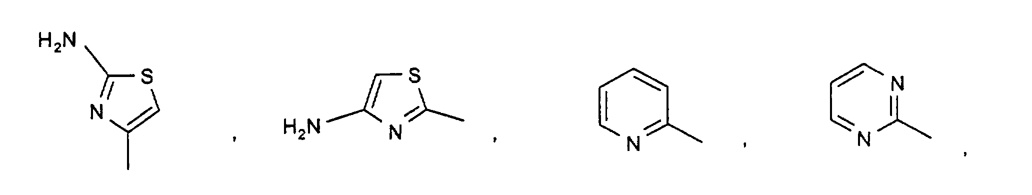 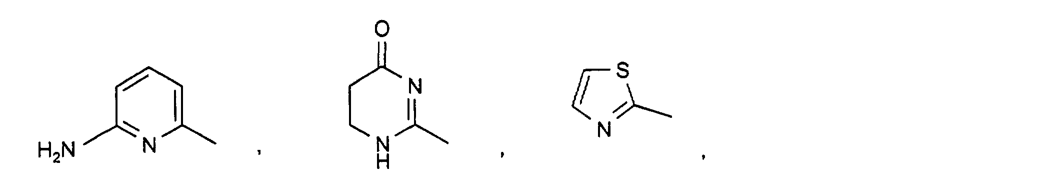 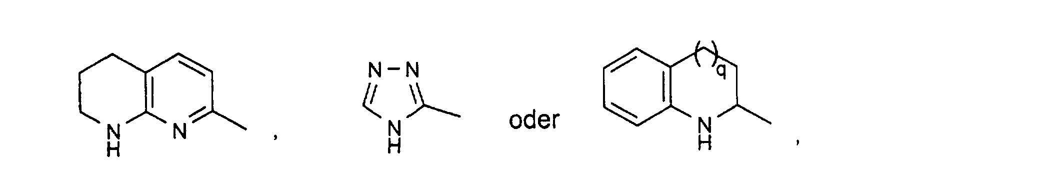 wobei Y' NH, O oder S bedeutet.
- R², R³: unabhängig voneinander H, (C₁-C₆)-Alkyl, Trifluormethyl, Pentafluorethyl, (C₅-C₆)-Cycloalkyl, (C₅-C₆)-Cycloalkyl-(C₁-C₂)-Alkyl, Phenyl, Benzyl, H₂N, R⁸OR⁹, R⁸-(C₅-C₁₀)-ArylR⁹, R⁸NHR⁹, R⁸R⁸NR⁹, R⁸NHC(O)R⁹, H₂N-C(=NH) oder H₂N-C(=NH)-NH-;
- R⁴, R⁵, R⁶, R⁷: unabhängig voneinander H, Fluor, OH, (C₁-C₆)-Alkyl, (C₁₀-C₁₂)-Cycloalkyl, (C₁₀-C₁₂)-Cycloalkyl-(C₁-C₆)-alkyl, R⁸OR⁹, R⁸-(C₅-C₁₀)-ArylR⁹, R⁸R⁸NR⁹, R⁸NHC(O)OR⁹, R⁸S(O)ₙNHR⁹, R⁸OC(O)NHR⁹ oder R⁸C(O)NHR⁹;
- R⁸: H, (C₁-C₆)-Alkyl, (C₁₀-C₁₂)-Cycloalkyl, (C₁₀-C₁₂)-Cycloalkyl-(C₁-C₂)-Alkyl, (C₅-C₁₀)-Aryl oder (C₅-C₁₀)-Aryl-(C₁-C₂)-alkyl;
- R⁹: eine direkte Bindung oder (C₁-C₆)-Alkandiyl;
- R¹⁰: C(O)R¹¹;
- R¹¹: OH, (C₁-C₆)-Alkoxy, Phenoxy, Benzyloxy, (C₁-C₄)-Alkylcarbonyloxy-(C₁-C₄)-alkoxy, NH₂, Mono- oder Di-(C₁-C₆-alkyl)-amino;
- R¹⁶: H, (C₁-C₄)-Alkyl, Trifluormethyl, Pentafluorethyl, (C₅-C₆)-Cycloalkyl, (C₅-C₆)-Cycloalkyl-(C₁-C₂)-alkyl, Phenyl oder Benzyl;
- n: 1 oder 2; und
- p, q: unabhängig voneinander 0 oder 1;
und ihre physiologisch verträgliche Salze,
wobei Verbindungen ausgenommen sind, in denen R¹-A-B-D-C(R¹⁶) gleich R¹-K-C(R¹⁶) ist, wobei hier
- R¹: für X-NH-C(= NH)-(CH₂)ₚ, X¹-NH-(CH₂)ₚ oder 4-Imidazolyl-CH₂- steht, wobei p für eine ganze Zahl von 0 bis 3 stehen kann,
- X: Wasserstoff, (C₁-C₆)-Alkyl, Hydroxy, (C₁-C₆)-Alkoxy, (C₆-C₁₄)-Aryl-(C₁-C₆)-alkoxy oder Amino bedeutet, wobei die Arylgruppen in X reine, gegebenenfalls einfach oder mehrfach substituierte, Carbocyclen darstellen,
- X¹: (C₄-C₁₄)-Aryl-(C₁-C₆)-alkoxy oder R'-NH-C(=N-R") bedeutet, wobei R' und R" unabhängig voneinander die Bedeutungen von X haben und wobei die Arylgruppen in X¹ reine, gegebenenfalls einfach oder mehrfach substituierte, Carbocyclen darstellen,
- K: (C₁-C₆)-Alkandiyl, Cyclohexandiyl, Phenylen, Phenylen-(C₁-C₆)-Alkandiyl, (C₁-C₆)-Alkandiyl-Phenylen, Phenylen-(C₂-C₆)-Alkendiyl oder einen zweiwertigen Rest eines 6-gliedrigen gesättigten oder ungesättigten Ringes, der 1 Stickstoffatom enthalten und einfach oder zweifach durch (C₁-C₆)-Alkyl oder doppelt gebundenen Sauerstoff oder Schwefel substituiert sein kann, bedeutet.

Die in den Substituenten auftretenden Alkylreste können geradkettig oder verzweigt, gesättigt oder einfach oder mehrfach ungesättigt sein. Entsprechendes gilt für davon abgeleitete Reste, wie z.B. Alkoxy. Cycloalkylreste können mono-, bi- oder tricyclisch sein.

Monocyclische Cycloalkylreste sind insbesondere Cyclopropyl, Cyclobutyl, Cyclopentyl, Cyclohexyl, Cycloheptyl und Cyclooctyl, die aber auch durch beispielsweise (C₁-C₄)-Alkyl substituiert sein können. Als Beispiele für substituierte Cycloalkylreste seien 4-Methylcyclohexyl und 2,3-Dimethylcyclopentyl genannt.

Bicyclische und tricyclische Cycloalkylreste können unsubstituiert sein oder in beliebigen geeigneten Positionen durch eine oder mehrere Oxogruppen und/oder eine oder mehrere gleiche oder verschiedene (C₁-C₄)-Alkylgruppen, z.B. Methyl- oder Isopropylgruppen, bevorzugt Methylgruppen, substituiert sein. Die freie Bindung des bi- oder tricyclischen Restes kann sich in einer beliebigen Position des Moleküls befinden, der Rest kann also über ein Brückenkopfatom oder ein Atom in einer Brücke gebunden sein. Die freie Bindung kann sich auch in einer beliebigen stereochemischen Position befinden, beispielsweise in einer exo- oder einer endo-Position.

Beispiele für Grundkörper bicyclischer Ringsysteme sind das Norbornan (= Bicyclo[2.2.1]heptan), das Bicyclo[2.2.2]octan und das Bicyclo[3.2.1]octan. Ein Beispiel für ein mit einer Oxogruppe substituiertes System ist der Campher (= 1,7,7-trimethyl-2-oxobicyclo[2.2.1]heptan).

Beispiele für Grundkörper tricyclischer Systeme sind das Twistan (= Tricyclo[4.4.0.0^{3,8}]decan, das Adamantan ( = Tricyclo[3.3.1.1^{3,7}]decan), das Noradamantan (= Tricyclo[3.3.1.0^{3,7}]-nonan), das Tricyclo[2.2.1.0^{2,6}]heptan, das Tricyclo[5.3.2.0^{4,9}]dodecan, das Tricyclo[5.4.0.0^{2,9}]undecan oder das Tricyclo[5.5.1.0^{3,11}]tridecan.

Aryl sind beispielsweise Phenyl, Naphthyl, Biphenylyl, Anthryl oder Fluorenyl, wobei 1-Naphthyl, 2-Naphthyl und insbesondere Phenyl bevorzugt sind. Arylreste, insbesondere Phenylreste, können ein- oder mehrfach, bevorzugt ein-, zwei oder dreifach, durch gleiche oder verschiedene Reste aus der Reihe (C₁-C₈)-Alkyl, insbesondere (C₁-C₄)-Alkyl, (C₁-C₈)-Alkoxy, insbesondere (C₁-C₄)-Alkoxy, Halogen, wie Fluor, Chlor und Brom, Nitro, Amino, Trifluormethyl, Hydroxy, Methylendioxy, Ethylendioxy, -OC(CH₃)₂O-, Cyano, Hydroxycarbonyl, Aminocarbonyl, (C₁-C₄)-Alkoxycarbonyl, Phenyl, Phenoxy, Benzyloxy, (R¹⁷O)₂P(O), (R¹⁷O)₂P(O)-O-, mit R¹⁷ = H, (C₁-C₁₈)-Alkyl, (C₆-C₁₄)-Aryl oder (C₆-C₁₄)-Aryl-(C₁-C₈)-alkyl oder Tetrazolyl substituiert sein.

In monosubstituierten Phenylresten kann sich der Substituent in der 2-, der 3- oder der 4-Position befinden, wobei die 3- und die 4-Position bevorzugt sind. Ist Phenyl zweifach substituiert, können die Substituenten in 1,2-, 1,3- oder 1,4-Position zueinander stehen. Bevorzugt sind in zweifach substituierten Phenylresten die beiden Substituenten in der 3- und der 4-Position, bezogen auf die Verknüpfungsstelle, angeordnet.

Arylgruppen können ferner mono- oder polycyclische aromatische Ringsysteme darstellen, worin 1 bis 5 C-Atome durch 1 bis 5 Heteroatome ersetzt sein können, wie z.B. 2-Pyridyl, 3-Pyridyl, 4-Pyridyl, Pyrrolyl, Furyl, Thienyl, Imidazolyl, Pyrazolyl, Oxazolyl, Isoxazolyl, Thiazolyl, Isothiazolyl, Tetrazolyl, Pyridyl, Pyrazinyl, Pyrimidinyl, Indolyl, Isoindolyl, Indazolyl, Phthalazinyl, Chinolyl, Isochinolyl, Chinoxalinyl, Chinazolinyl, Cinnolinyl, β-Carbolinyl, oder ein benz-anelliertes, cyclopenta-, cyclohexa- oder cyclohepta-anelliertes Derivat dieser Reste.
Diese Heterocyclen können mit den gleichen Substituenten wie die vorstehend genannten carbocyclischen Arylsysteme substituiert sein.

In der Reihe dieser Arylgruppen sind bevorzugt mono- oder bicyclische aromatische Ringsysteme mit 1 - 3 Heteroatome aus der Reihe N, O, S, die mit 1 - 3 Substituenten aus der Reihe (C₁-C₆)-Alkyl, (C₁-C₆)-Alkoxy, Fluor, Cl, NO₂, NH₂, Trifluormethyl, OH, (C₁-C₄)-Alkoxycarbonyl, Phenyl, Phenoxy, Benzyloxy oder Benzyl substituiert sein können.

Besonders bevorzugt sind hierbei mono- oder bicyclische aromatische 5 - 10 gliedrige Ringsysteme mit 1 - 3 Heteroatomen aus der Reihe N, O, S, die mit 1 - 2 Substituenten aus der Reihe (C₁-C₄)-Alkyl, (C₁-C₄)-Alkoxy, Phenyl, Phenoxy, Benzyl oder Benzyloxy substituiert sein können.

Das Vorgenannte gilt auch für die von Alkyl, Cycloalkyl und Aryl abgeleiteten bivalenten Reste wie Alkandiyl, Alkendiyl, Alkindiyl, Cycloalkandiyl und Arylen.

Bevorzugt sind auch Verbindungen der Formel I, die einen lipophilen Rest R⁴, R⁵, R⁶ oder R⁷ wie z.B. Benzyloxycarbonylamino, Cyclohexylmethylcarbonylamino etc. tragen.

Physiologisch verträgliche Salze der Verbindungen der allgemeinen Formel I sind insbesondere pharmazeutisch verwendbare oder nicht-toxische Salze.
Solche Salze werden beispielsweise von Verbindungen der allgemeinen Formel I, welche saure Gruppen, z.B. Carboxy, enthalten, mit Alkali- oder Erdalkalimetallen gebildet, wie z.B. Na, K, Mg und Ca, sowie mit physiologisch verträglichen organischen Aminen, wie z.B. Triethylamin, Ethanolamin oder Tris-(2-hydroxyethyl)-amin.
Verbindungen der allgemeinen Formel I, welche basische Gruppen, z.B. eine Aminogruppe, eine Amidinogruppe oder eine Guanidinogruppe enthalten, bilden mit anorganischen Säuren, wie z.B. Salzsäure, Schwefelsäure oder Phosphorsäure, und mit organischen Carbon- oder Sulfonsäuren, wie z.B. Essigsäure, Citronensäure, Benzoesäure, Maleinsäure, Fumarsäure, Weinsäure, Methansulfonsäure oder p-Toluolsulfonsäure Salze.

Die erfindungsgemäßen Verbindungen der allgemeinen Formel I können optisch aktive Kohlenstoffatome, die unabhängig voneinander R- oder S-Konfigurationen haben können, enthalten und somit in Form reiner Enantiomerer oder reiner Diastereomerer oder in Form von Enantiomerengemischen oder Diastereomerengemischen vorliegen. Sowohl reine Enantiomere und Enantiomerengemische als auch Diastereomere und Diastereomerengemische sind Gegenstand der vorliegenden Erfindung.

Die erfindungsgemäßen Verbindungen der allgemeinen Formel I können darüber hinaus bewegliche Wasserstoffatome enthalten, also in verschiedenen tautomeren Formen vorliegen. Auch diese Tautomeren sind Gegenstand der vorliegenden Erfindung.

Die erfindungsgemäßen Verbindungen der Formel I können, falls A, D oder F unabhängig voneinander -CR²=CR³-, -NR²-N=CR²-, -N=CR²- oder -R²C=N- und/oder B -CR²=CR³- sind, und/oder W R¹-A-B-D-C(R¹⁶)=C bzw. 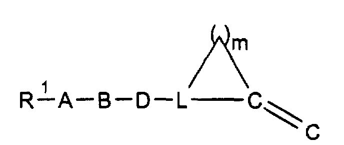 ist, als E/2-Isomerengemische vorliegen. Gegenstand der vorliegenden Erfindung sind sowohl reine E- bzw. Z-lsomere als auch E/Z-lsomerengemische. Diastereomere, einschließlich E/Z-Isomere können durch Chromatographie in die Einzelisomeren aufgetrennt werden. Racemate können entweder durch Chromatographie an chiralen Phasen oder durch Racematspaltung in die beiden Enantiomere aufgetrennt werden.

Bevorzugt sind Verbindungen der Formel I, in der bedeuten:
- F: ist CO-NR²-;
sowie deren physiologisch verträglichen Salze.

Besonders bevorzugt sind Verbindungen der Formel I, in der bedeuten: 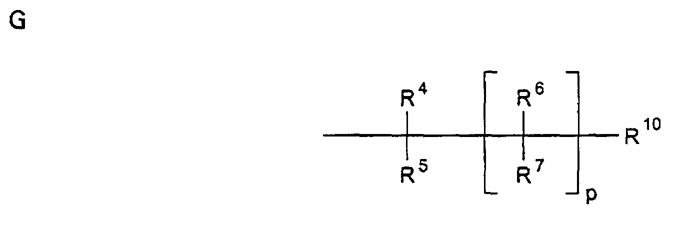 sowie deren physiologisch verträglichen Salze.

Verwendete Abkürzungen:
- Boc :: t-Butoxycarbonyl
- DCCI :: Dicyclohexylcarbodiimid
- DMF :: Dimethylformamid
- HOOBt:: 3-Hydroxy-4-oxo-3,4-dihydro-1,2,3-benzotriazin
- THF :: Tetrahydrofuran
- HOBt:: 1-Hydroxy-benzotriazol
- TOTU: O-[Cyan(ethoxycarbonyl)methylenamino]-1,1,3,3-tetramethyluronium-tetrafluoroborat
- DIPEA:: Diisopropyl-ethylamin
- RT :: Raumtemperatur
- Z :: Benzyloxycarbonyl

Verbindungen der Formel I können generell, beispielsweise im Zuge einer konvergenten Synthese, durch Verknüpfung zweier oder mehrerer Fragmente, die sich retrosynthetisch aus der allgemeinen Formel I ableiten lassen, hergestellt werden. Bei der Herstellung der Verbindungen der Formel I kann es generell im Laufe der Synthese nötig sein, funktionelle Gruppen, die im jeweiligen Syntheseschritt zu unerwünschten Reaktionen oder Nebenreaktionen führen könnten, durch eine dem Syntheseproblem angepaßte Schutzgruppenstrategie temporär zu blockieren, was dem Fachmann bekannt ist. Die Methode der Fragmentverknüpfung ist nicht auf die nachfolgenden Beispiele beschränkt, sondern allgemein für Synthesen der Verbindungen der Formel I anwendbar.

Beispielsweise können Verbindungen der Formel I mit F = C(O)NR² durch Kondensation einer Verbindung der Formel II 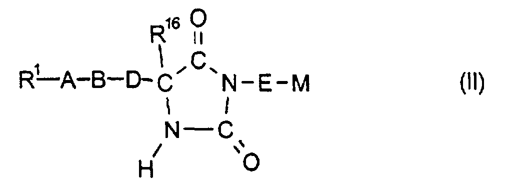 wobei M für Hydroxycarbonyl, (C₁-C₆)-Alkoxycarbonyl, aktivierte Carbonsäurederivate wie Säurechloride, Aktivester oder gemischte Anhydride steht, mit HNR²-G hergestellt werden.

Zur Kondensation zweier Fragmente unter Bildung einer Amidbindung verwendet man vorteilhafterweise die an sich bekannten Kupplungsmethoden der Peptidchemie (siehe z.B. Houben-Weyl, Methoden der Organischen Chemie, Band 15/1 und 15/2, Georg Thieme Verlag, Stuttgart, 1974). Dazu ist es in der Regel nötig, daß vorhandene, nicht reagierende Aminogruppen durch reversible Schutzgruppen während der Kondensation geschützt werden. Gleiches gilt für nicht an der Reaktion beteiligte Carboxylgruppen, die bevorzugt als (C₁-C₆)-Alkyl-, Benzyl- oder tert.-Butylester eingesetzt werden. Ein Aminogruppen-Schutz erübrigt sich, wenn die zu generierenden Aminogruppen noch als Nitro- oder Cyanogruppen vorliegen und erst nach der Kupplung durch Hydrierung gebildet werden. Nach der Kupplung werden die vorhandenen Schutzgruppen in geeigneter Weise abgespalten. Beispielsweise können NO₂-Gruppen (Guanidinoschutz), Benzyloxycarbonylgruppen und Benzylester abhydriert werden. Die Schutzgruppen vom tert.-Butyltyp werden sauer abgespalten, während der 9-Fluorenylmethyloxycarbonylrest durch sekundäre Amine entfernt wird.

Verbindungen der Formel I, in denen 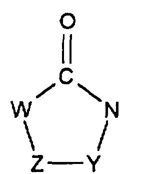 einen Dioxo- oder Thioxo-oxo substituierten Imidazolidinring darstellt, in dem W für R¹-A-B-D-C(R¹⁶) steht können beispielsweise erhalten werden:
durch Reaktion von α-Aminosäuren oder N-substituierten α-Aminosäuren oder bevorzugt deren Ester, z.B. der Methyl-, Ethyl-, tert.-Butyl- oder Benzylester, beispielsweise einer Verbindung der allgemeinen Formel III 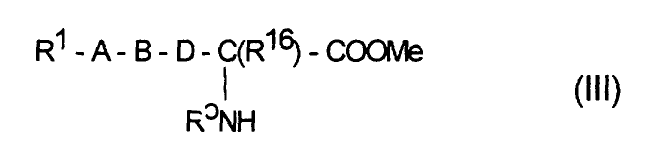 mit einem Isocyanat oder Isothiocyanat, beispielsweise der allgemeinen Formel U - E - F - G mit U gleich Isocyanato, Isothiocyanato oder Trichlormethylcarbonylamino, wobei man Harnstoff- oder Thioharnstoffderivate der allgemeinen Formel IV erhält, 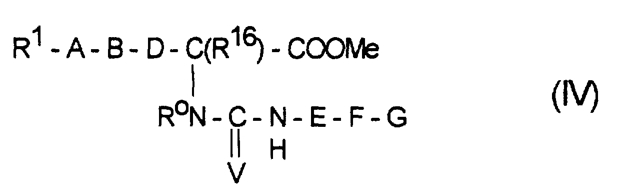 in der V Sauerstoff oder Schwefel bedeutet, die durch Erhitzen mit Säure unter Verseifung der Esterfunktion zu Verbindungen der Formel I des Typs 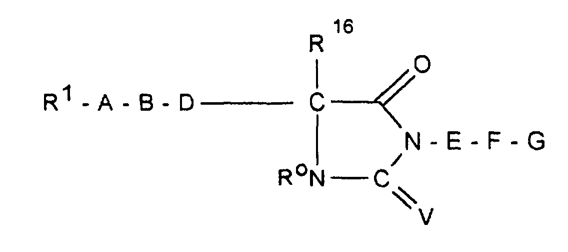 cyclisiert werden.

Eine weitere Methode zur Herstellung von Verbindungen der allgemeinen Formel I in der Y C=O oder C = S und W R¹-A-B-D-C(R¹⁶) bedeuten, ist beispielsweise die Umsetzung von Verbindungen der Formel V 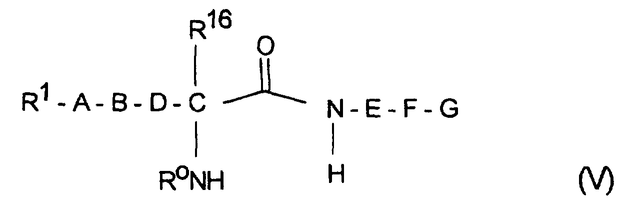 mit Phosgen, Thiophosgen oder entsprechenden Äquivalenten (analog S. Goldschmidt und M. Wick, Liebigs Ann. Chem. 575 (1952), 217-231 und C. Tropp, Chem. Ber. 61 (1928), 1431-1439).

Verbindungen der Formel I, in denen 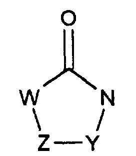 einen Heterocyclus des Typs 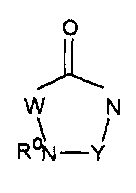 darstellt, in denen Y C=O oder C=S und W R¹-A-B-D-C(R¹⁶)=C bedeuten, werden beispielsweise nach folgendem Schema 1 hergestellt: 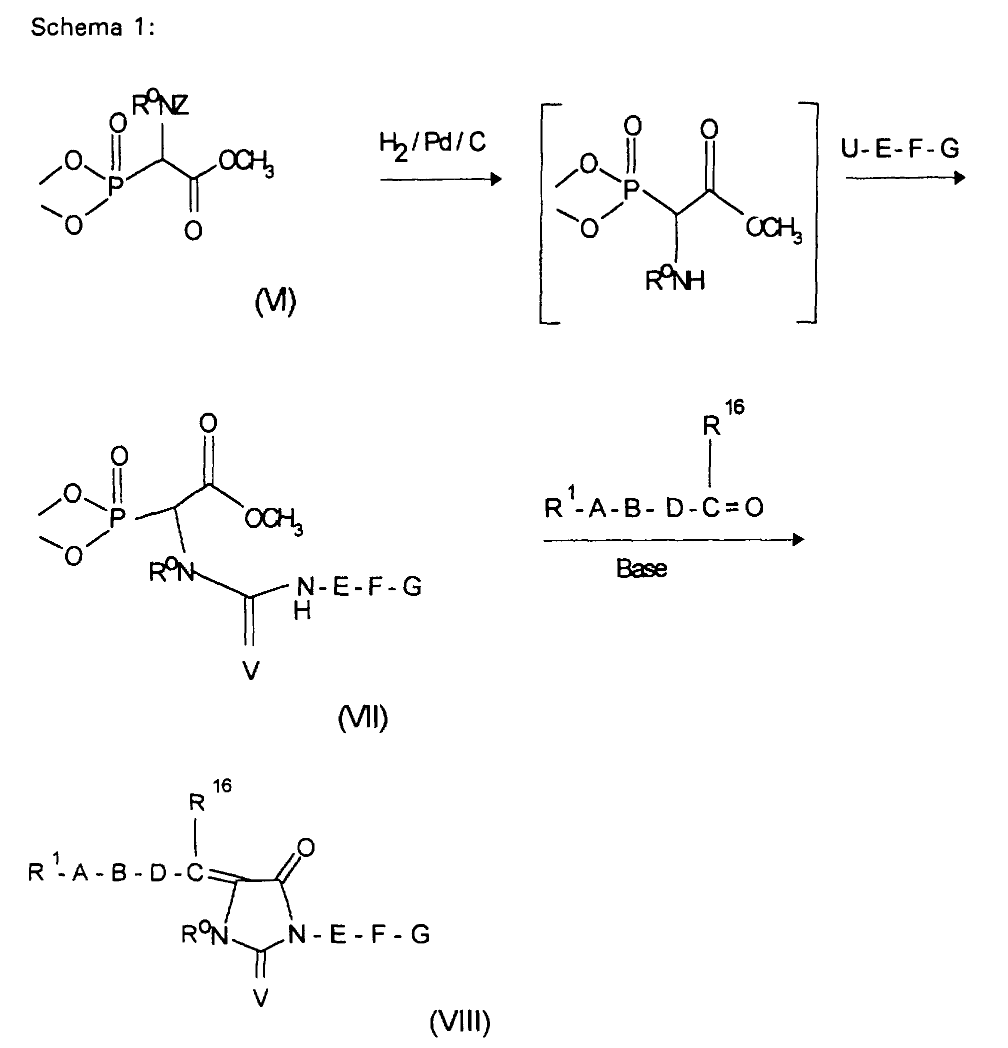 U bedeutet -NCO oder -NCS; V bedeutet 0, S.

Die Umsetzung von Verbindungen der Formel VI zu Verbindungen der Formel VII und Verbindungen der Formel VII zu Verbindungen der Formel VIII kann beispielsweise analog S. Chung-gi et al., Tetrahedron Lett 1987, 28 (33), 3827 oder U. Schmidt et al. Angew. Chemie 1984, 53 durchgeführt werden.

Eine weitere Möglichkeit zur Herstellung von Verbindungen der Formel VIII besteht beispielsweise darin, zunächst Verbindungen der Formel VII unter Einfluß von Säure zu Verbindungen der Formel XII 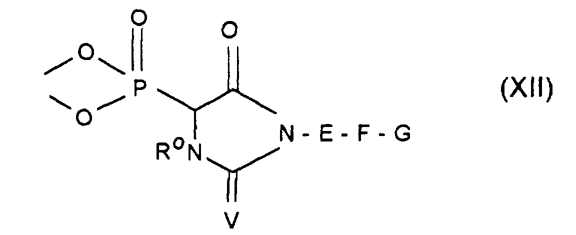 zu cyclisieren und anschließend Verbindungen der Formel XII in einer Horner-Emmons-Reaktion mit 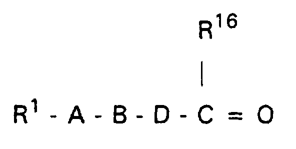 zu Verbindungen der Formel VIII umzusetzen.

Verbindungen der Formel I, in denen 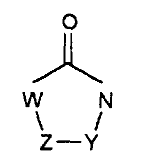 einen Heterocyclus des Typs 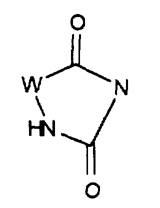 darstellt, in dem W 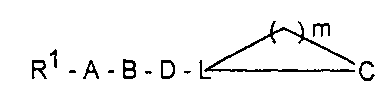 ist, können beispielsweise nach folgendem Schema 2 hergestellt werden:

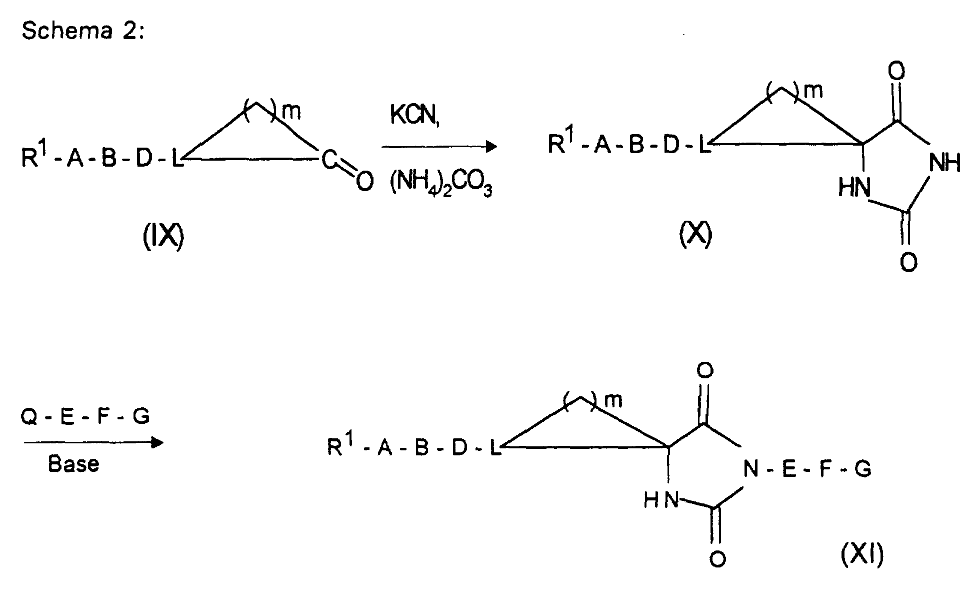

Q bedeutet eine nukleophil substituierbare Abgangsgruppe wie z.B. Halogen, Mesylat, Tosylat etc.

Die Umsetzung von Verbindungen der Formel IX zu Verbindungen der Formel X kann beispielsweise analog E. Marinez et al., Helv. Chim. Acta 1983, 66 (1), 338 oder E.W. Logusch et al., J. Org. Chem. 1988, 53 (17), 4069 durchgeführt werden.

Verbindungen der Formel I, in denen 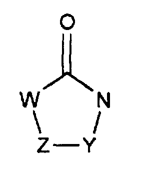 einen Heterocyclus des Typs 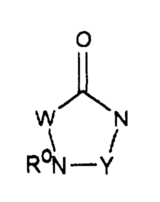 darstellt, in dem Y C=O oder C=S und W 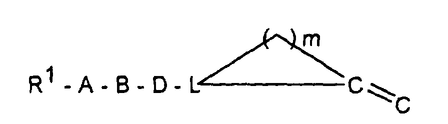 bedeuten, können beispielsweise nach folgendem Schema 3 hergestellt werden: 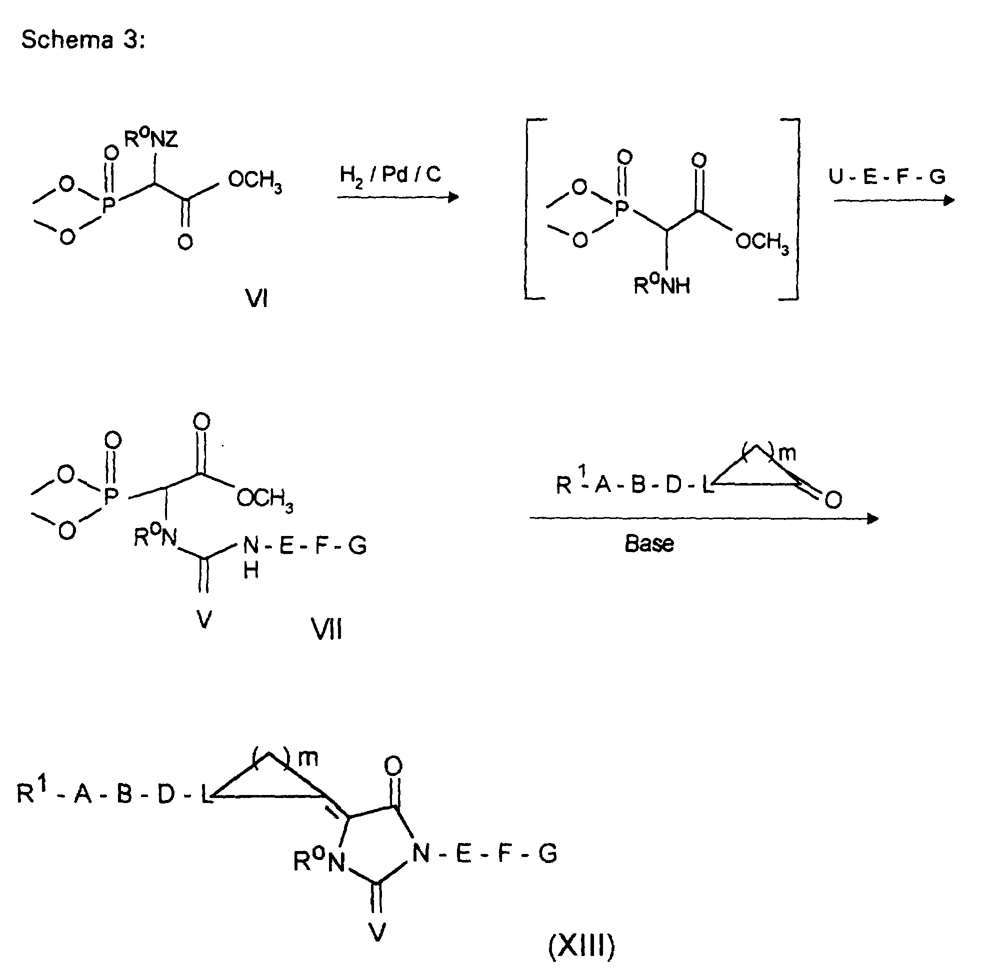 U bedeutet - NCO oder -NCS; V bedeutet 0, S.

Eine andere Möglichkeit zur Herstellung von Verbindungen der Formel XIII besteht beispielsweise darin Verbindungen der Formel VII unter Einfluß von Säure zu Verbindungen der Formel XII zu cyclisieren und anschließend Verbindungen der Formel XII in einer Horner-Emmons-Reaktion mit 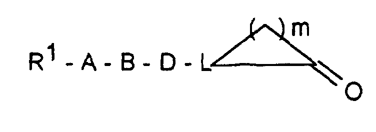 zu Verbindungen der Formel XIII umzusetzen.

Im Zuge einer konvergenten Synthese kann es allerdings vorteilhaft sein, je nach Bedeutung der einzelnen Substituenten R¹, A, B, D etc. zunächst das heterocyclische Ringsystem, das nur einen Teil der Substituenten trägt, aufzubauen und anschließend die restlichen Substituenten beispielsweise im Zuge einer Fragmentverknüpfung einzuführen. Als Beispiel sei hier die Synthese von Beispiel 1 erwähnt:

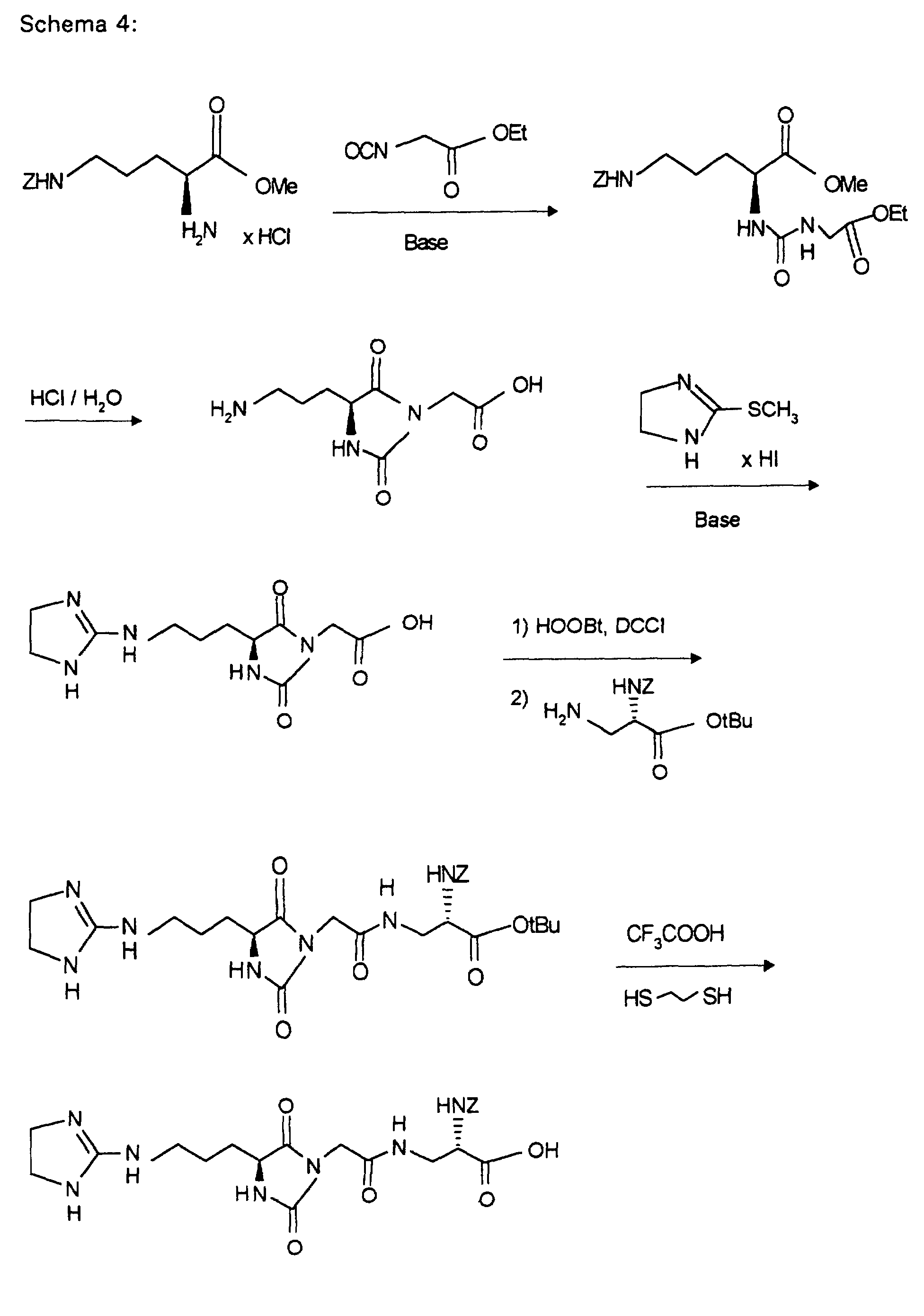

Dieses generelle Prinzip ist allerdings nicht auf dieses eine Beispiel beschränkt, sondern allgemein anwendbar.

Verbindungen der Formel I, in denen R¹-A 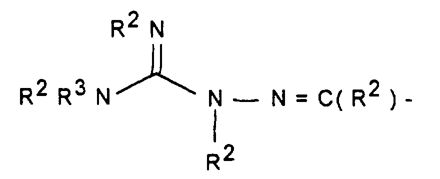 oder cyclische Guanylhydrazone des Typs 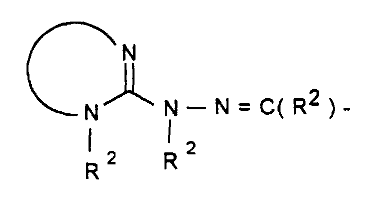 bedeuten, werden beispielsweise durch Kondensation von 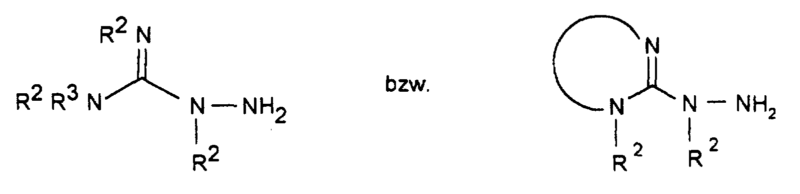 mit Ketonen oder Aldehyden des Typs O=C(R²)- oder entsprechender Acetale oder Ketale nach gängigen Literaturverfahren, beispielsweise analog N. Desideri et al., Arch. Pharm. 325 (1992) 773-777 oder A. Alves et al., Eur. J. Med. Chem. Chim. Ther. 21 (1986) 297-304 hergestellt.

Obige Guanylhydrazone können gegebenenfalls als E/Z-Isomerengemische anfallen, die nach gängigen Chromatographieverfahren getrennt werden können.

Verbindungen der Formel I, in denen R¹-A R²-C(=NR²)NR²-N=C(R²)- oder ein einen Mono- oder Polycyclus enthaltendes System des Typs 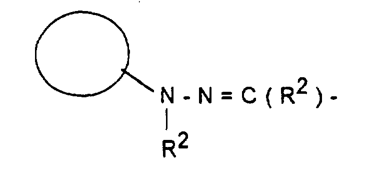 darstellt, können analog erhalten werden.

Verbindungen der Formel I mit R¹⁰=SO₂R¹¹ werden beispielsweise hergestellt, in dem man Verbindungen der Formel I mit R¹⁰=SH nach literaturbekannten Verfahren (vgl. Houben-Weyl, Methoden der Organischen Chemie, Bd. E12/2, Georg Thieme Verlag, Stuttgart 1985, S. 1058ff) zu Verbindungen der Formel I mit R¹⁰=SO₃H oxidiert, aus denen dann direkt oder über entsprechende Sulfonsäurehalogenide durch Veresterung oder Knüpfung einer Amidbindung die Verbindungen der Formel I mit R¹⁰=SO₂R¹¹ (R¹¹ ≠ OH) hergestellt werden. Oxidationsempfindliche Gruppen im Molekül, wie z.B. Amino-, Amidino- oder Guanidinogruppen werden, falls erforderlich, vor Durchführung der Oxidation durch geeignete Schutzgruppen geschützt.

Verbindungen der Formel I mit R¹⁰=S(O)R¹¹ werden beispielsweise hergestellt, in dem man Verbindungen der Formel I mit R¹⁰=SH in das entsprechende Sulfid (R¹⁰=S^{⊖}) überführt und anschließend mit meta-Chlorperbenzoesäure zu den Sulfinsäuren (R¹⁰= SO₂H) oxidiert (vgl. Houben-Weyl, Methoden der Organischen Chemie, Bd. E11/1, Georg Thieme Verlag, Stuttgart 1985, S. 618f), aus denen nach literaturbekannten Methoden die entsprechenden Sulfinsäureester oder -amide R¹⁰=S(O)R¹¹ (R¹¹ ≠ OH) hergestellt werden können. Generell können auch andere literaturbekannte Methoden zur Herstellung von Verbindungen der Formel I mit R¹⁰ = S(O)ₙR¹¹ (n =1,2) Anwendung finden (vgl. Houben-Weyl, Methoden der Organischen Chemie, Bd. E11/1, Georg Thieme Verlag, Stuttgart 1985, S. 618ff oder Bd. E11/2, Stuttgart 1985, S. 1055ff).

Verbindungen der Formel I mit R¹⁰ =P(O)Rₙ¹¹ (n =1,2) werden nach literaturbekannten Verfahren (vgl. Houben-Weyl, Methoden der Organischen Chemie, Bd. E1 und E2, Georg Thieme Verlag, Stuttgart 1982) aus geeigneten Vorstufen aufgebaut, wobei die gewählte Synthesemethode dem Zielmolekül anzupassen ist.

Verbindungen der Formel I mit R¹⁰=C(S)R¹¹ können nach Literaturverfahren hergestellt werden (vgl. Houben-Weyl, Methoden der Organischen Chemie, Bd. E5/1 und E5/2, Georg Thieme Verlag, Stuttgart 1985).

Verbindungen der Formel I mit R¹⁰=S(O)ₙR¹¹ (n=1,2), P(O)R¹¹ₙ (n=1,2) oder C(S)R¹¹ können natürlich auch durch Fragmentverknüpfung, wie vorstehend beschrieben, hergestellt werden, was beispielsweise ratsam ist, wenn in E-F-G der Formel I z.B. eine (käufliche) Aminosulfonsäure, Aminosulfinsäure, Aminophosphonsäure oder Aminophosphinsäure oder daraus abgeleitete Derivate, wie Ester oder Amide, enthalten sind.

Verbindungen der Formel I in denen R¹-A 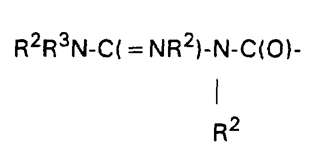 oder cyclische Acylguanidine des Typs 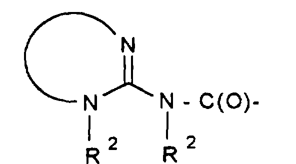 bedeutet, können beispielsweise hergestellt werden, indem man eine Verbindung der Formel I, in der W Q(O)C-B-D-C(R¹⁶)- oder Q(O)C-B-D-C(R¹⁶)= C oder 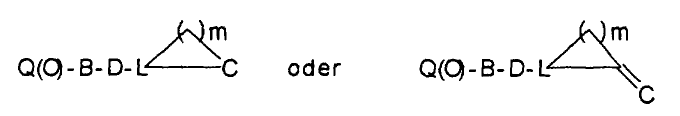 ist und Q für eine leicht nukleophil substituierbare Abgangsgruppe steht, mit dem entsprechenden Guanidin(derivat) des Typs 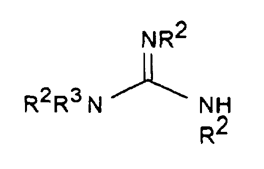 oder cyclischen Guanidin(derivat) 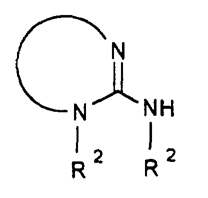 umsetzt.

Die vorstehenden aktivierten Säurederivate des Typs Q(O)C, worin Q eine Alkoxy-, vorzugsweise eine Methoxygruppe, eine Phenoxygruppe, Phenylthio-, Methylthio-, 2-Pyridylthiogruppe, einen Stickstoffheterocyclus, vorzugsweise 1-Imidazolyl, bedeutet, erhält man vorteilhaft in an sich bekannter Weise aus den zugrundeliegenden Carbonsäurechloriden Q=Cl, die man ihrerseits wiederum in an sich bekannter Weise aus den zugrundeliegenden Carbonsäuren Q=OH beispielsweise mit Thionylchlorid herstellen kann.

Neben den Carbonsäurechloriden (Q=Cl) lassen sich auch weitere aktivierte Säurederivate des Typs Q(O)C- in an sich bekannter Weise direkt aus den zugrundeliegenden Carbonsäuren (Q=OH) herstellen, wie beispielsweise die Methylester (Q=OCH₃) durch Behandeln mit gasförmigem HCI in Methanol, die Imidazolide (Q= 1-Imidazolyl) durch Behandeln mit Carbonyldiimidazol [vgl. Staab, Angew. Chem. Int. Ed. Engl. 1, 351-367 (1962)], die gemischten Anhydride (Q=C₂H₅OC(O)O bzw. TosO) mit Cl-COOC₂H₅ bzw. Tosylchlorid in Gegenwart von Triethylamin in einem inerten Lösungsmittel. Die Aktivierung der Carbonsäuren kann auch mit Dicyclohexylcarbodiimid (DCCI) oder mit O-[(Cyano(ethoxycarbonyl)methylen)amino]-1,1,3,3-tetramethyluroniumtetrafluorborat ("TOTU") [Weiss und Krommer, Chemiker Zeitung 98, 817 (1974)] und anderer in der Peptidchemie gebräuchlichen Aktivierungs-Reagentien erfolgen. Eine Reihe geeigneter Methoden zur Herstellung von aktivierten Carbonsäurederivaten der Formel II sind unter Angabe von Quellenliteratur in J. March, Advanced Organic Chemistry, Third Edition (John Wiley & Sons, 1985), S. 350 angegeben.

Die Umsetzung eines aktivierten Carbonsäurederivates desTyps Q(O)C- mit dem jeweiligen Guanidin(derivat) erfolgt in an sich bekannter Weise in einem protischen oder aprotischen polaren aber inerten organischen Lösungsmittel. Dabei haben sich bei der Umsetzung der Methylester (Q=OMe) mit den jeweiligen Guanidinen Methanol, Isopropanol oder THF von 20°C bis zur Siedetemperatur dieser Lösungsmittel bewährt. Bei den meisten Umsetzungen von Verbindungen des Typs Q(O)C- mit salzfreien Guanidinen wird vorteilhaft in aprotischen inerten Lösungsmitteln wie THF, Dimethoxyethan, Dioxan gearbeitet. Aber auch Wasser kann unter Gebrauch einer Base (wie beispielsweise NaOH) als Lösungsmittel bei der Umsetzung von Q(O)C- mit Guanidinen verwendet werden.
Wenn Q=Cl bedeutet, arbeitet man vorteilhaft unter Zusatz eines Säurefängers, z.B. in Form von überschüssigem Guanidin(derivat) zur Abbindung der Halogenwasserstoffsäure.

Verbindungen der Formel I, in denen 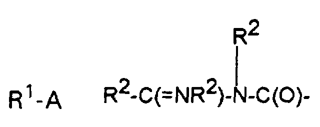 oder ein einen Mono- oder Polycyclus enthaltendes System des Typs 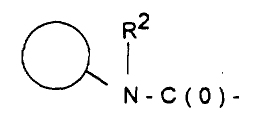 darstellt, können analog erhalten werden.

Verbindungen der Formel I, in denen R¹-A ein Sulfonyl- oder Sulfoxylguanidin des Typs R²R³N-C(=NR²)-NR²-S(O)ₙ (n =1,2) bzw. 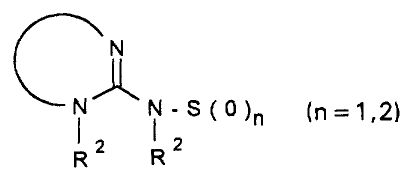 darstellt, werden nach literaturbekannten Verfahren durch Reaktion von R²R³N-C(=NR³)NR²H bzw. 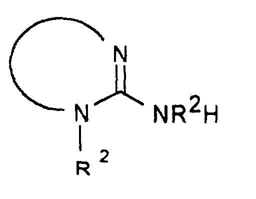 mit Sulfin- oder Sulfonsäurederivaten der Formel I in der W Q-S(O)ₙ-B-D-C(R¹⁶)- oder Q-S(O)ₙ-B-D-C(R¹⁶)=C oder 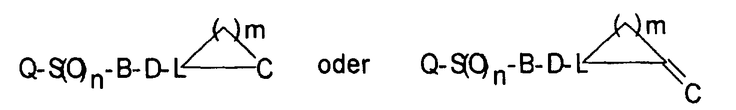 und Q z.B. gleich Cl oder NH₂ bedeuten, analog S. Birtwell et al., J. Chem. Soc. (1946) 491 oder Houben Weyl, Methoden der Organischen Chemie, Bd. E4, Georg Thieme Verlag, Stuttgart 1983; S. 620 ff, hergestellt.

Verbindungen der Formel I, in denen R¹A R²-C(=NR²)NR²-S(O)ₙ (n=1,2) oder ein einen Mono- oder Polycyclus enthaltendes System des Typs 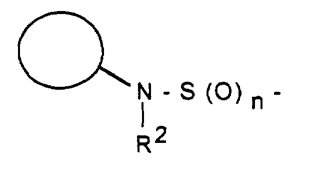 (n = 1,2) bedeutet, können analog erhalten werden.

Verbindungen der Formel I, in denen A -NR²-C(O)-NR²-, -NR²-C(O)O-, -NR²-C(O)S- und R¹ R²R³N-C(=NR²), R²-C(=NR²) oder ein 4-14 gliedriges mono- oder polycyclisches, aromatisches oder nicht aromatisches Ringsystem, das wie auf Seite 6 beschrieben, spezifiziert ist und wie dort beschrieben substituiert sein kann, bedeutet, werden z.B. hergestellt, indem man eine Verbindung der Formel I, in der W Q-B-D-C(R¹⁶)- oder Q-B-D-C(R¹⁶)=C oder 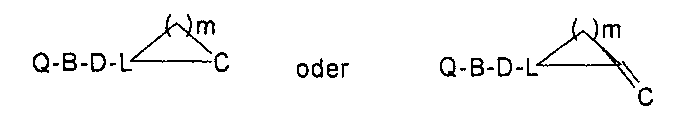 und Q HNR²-, HO- oder HS- bedeuten, mit einem geeigneten Kohlensäurederivat, bevorzugt Phosgen, Diphosgen (Chlorameisensäuretrichlormethylester), Triphosgen (Kohlensäure-bis-trichlormethylester), Chlorameisensäureethylester, Chlorameisensäure-i-butylester, Bis-(1-hydroxy-1-H-benzotriazolyl)carbonat oder N,N'-Carbonyldiimidazol, in einem gegenüber den verwendeten Reagentien inerten Lösungsmittel, bevorzugt DMF, THF oder Toluol, bei einer Temperatur zwischen -20°C und dem Siedepunkt des Lösungsmittels, bevorzugt zwischen 0°C und 60°C, zunächst zu einem substituierten Kohlensäurederivat der Formel I, in denen W 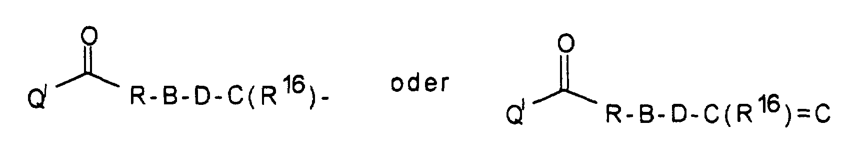 oder 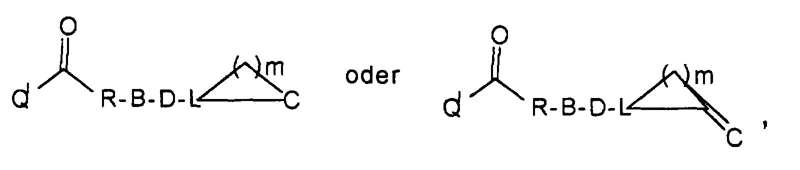 R -NR²-, -O- oder -S- und Q' je nach verwendetem Kohlensäurederivat Chlor, Ethoxy, Isobutoxy, Benzotriazol-1-oxy oder 1-Imidazolyl bedeuten, umsetzt.

Die Umsetzung dieser Derivate mit R²R³N-C(=NR²)-NR²H oder R²-C(= NR²)-NR²H oder mit den einen Mono- oder Polycyclus enthaltenden Systemen des Typs 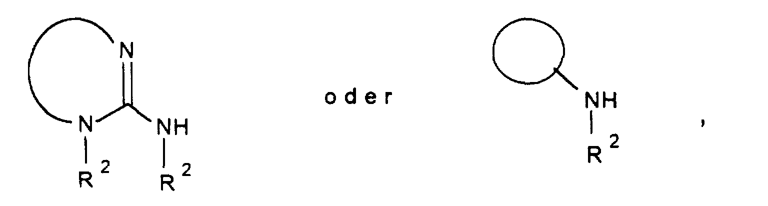 erfolgt wie vorstehend bei der Herstellung von Acylguanidin(derivaten) beschrieben.

Verbindungen der Formel I, in denen F R²N-C(O)-NR² oder R²N-C(S)-NR² ist, werden beispielsweise hergestellt, indem man eine Verbindung des Typs 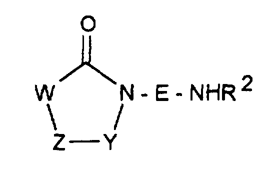 mit einem Isocyanat OCN-G oder Isothiocyanat SCN-G nach literaturbekannten Verfahren umsetzt.

Verbindungen der Formel I, in denen F C(O)NR², -SO₂NR²- oder -C(O)O- ist, können z.B. durch Umsetzung von 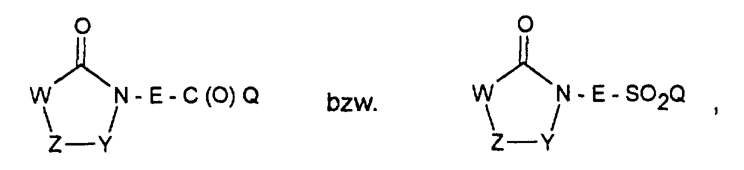 (Q ist eine leicht nukleophil substituierbare Abgangsgruppe, wie z.B. OH, Cl, OMe etc.) mit HR²N-G bzw. HO-G nach Literaturverfahren erhalten werden.

Verbindungen der Formel I, in denen R¹-A einen Mono- oder Polycyclus des Typs 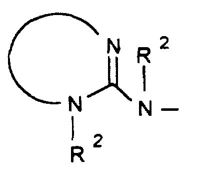 beinhaltet, können beispielsweise hergestellt werden, indem man eine Verbindung der Formel I in der W HR²N-B-D-C(R¹⁶)- oder HR²N-B-D-C(R¹⁶)=C oder 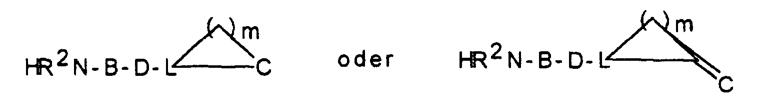 bedeutet mit einem Mono- oder Polycyclus des Typs 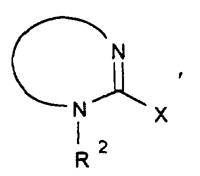 in der X eine nukleophil substituierbare Abgangsgruppe wie z.B. Halogen oder SH, SCH₃, SOCH₃, SO₂CH₃, SO₃H oder HN-NO₂ darstellt, nach literaturbekannten Verfahren (siehe z.B. A.F. Mckay et al., J. Med. Chem. 6 (1963) 587, M.N. Buchman et al., J. Am. Chem. Soc. 71 (1949), 766, F. Jung et al., J. Med. Chem. 34 (1991) 1110 oder G. Sorba et al., Eur. J. Med. Chem. 21 (1986), 391) umsetzt.

Verbindungen der Formel I, in denen R¹A einen Mono- oder Polycyclus des Typs 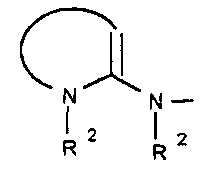 beinhaltet, können beispielsweise hergestellt werden, indem man eine Verbindung der Formel I in der W HR²N-B-D-C(R¹⁶)- oder HR²N-B-D-C(R¹⁶)=C oder 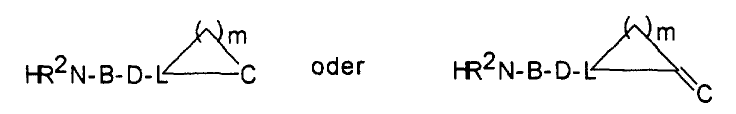 bedeutet mit einer Verbindung des Typs 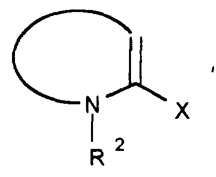 in der X eine Abgangsgruppe, wie z.B. -SCH3 darstellt, nach literaturbekannten Verfahren (vgl. z.B. T. Hiroki et al., Synthesis (1984) 703 oder M. Purkayastha et al., Indian J. Chem. Sect. B 30 (1991) 646) umsetzt.

Verbindungen der Formel I, in denen R¹A einen Bis-Aminotriazol- oder einen Bis-Amino-oxadiazolrest darstellt, können beispielsweise nach P.J. Garrett et al., Tetrahedron 49 (1993) 165 oder R. Lee Webb et al., J. Heterocyclic Chem. 24 (1987) 275 gemäß folgender Reaktionssequenz hergestellt werden: 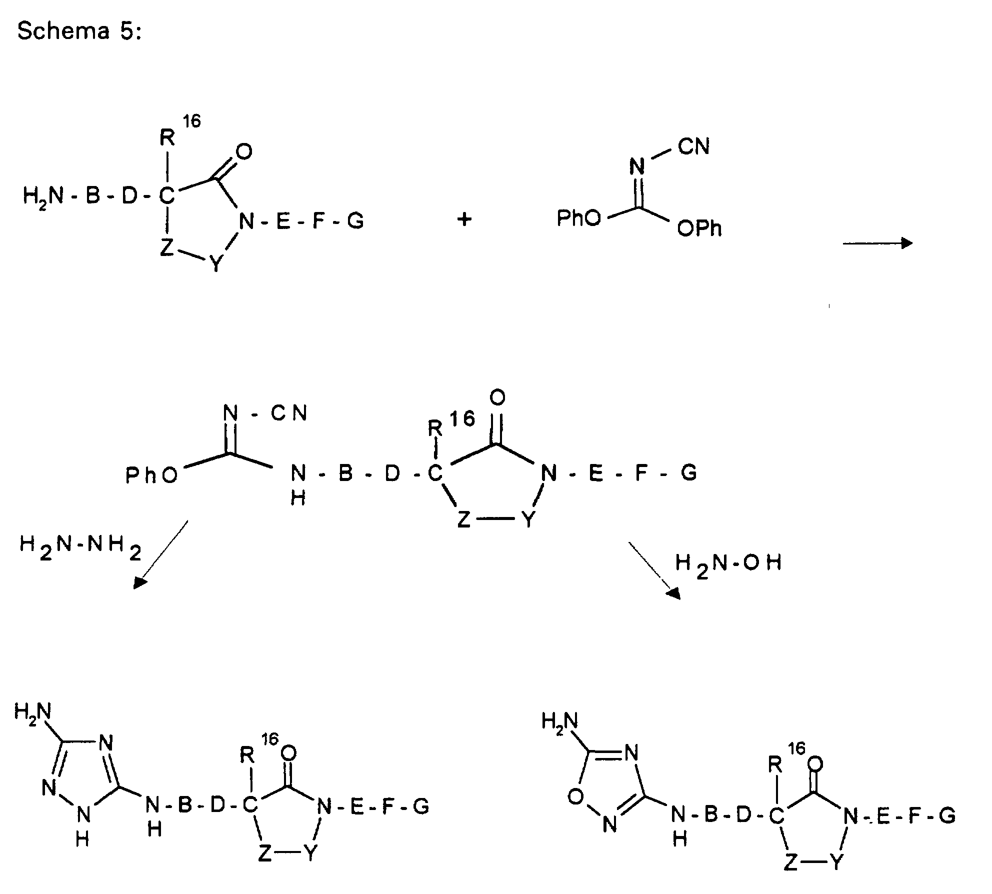 Literaturbekannte Herstellungsverfahren sind z.B. in J. March, Advanced Organic Chemistry, Third Edition (John Wiley & Sons, 1985) beschrieben.

Die Verbindungen der allgemeinen Formel I und ihre physiologisch verträglichen Salze können am Tier, bevorzugt am Säugetier, und insbesondere am Menschen als Heilmittel für sich allein, in Mischungen untereinander oder in Form von pharmazeutischen Zubereitungen verabreicht werden, die eine enterale oder parenterale Anwendung gestatten und die als aktiven Bestandteil eine wirksame Dosis mindestens einer Verbindung der allgemeinen Formel I oder eines Salzes davon, neben üblichen pharmazeutisch einwandfreien Träger- und Zusatzstoffen enthalten. Die Zubereitungen enthalten normalerweise etwa 0,5 bis 90 Gew.-% der therapeutisch wirksamen Verbindung.

Die Heilmittel können oral, z.B. in Form von Pillen, Tabletten, Lacktabletten, Dragees, Granulaten, Hart- und Weichgelatinekapseln, Lösungen, Sirupen, Emulsionen, Suspensionen oder Aerosolmischungen verabreicht werden. Die Verabreichung kann aber auch rektal, z.B. in Form von Suppositorien, oder parenteral, z.B. in Form von Injektions- oder Infusionslösungen, Mikrokapseln oder Rods, perkutan, z.B. in Form von Salben oder Tinkturen, oder nasal, z.B. in Form von Nasalsprays, erfolgen.

Die Herstellung der pharmazeutischen Präparate erfolgt in an sich bekannter Weise, wobei pharmazeutisch inerte anorganische oder organische Trägerstoffe verwendet werden. Für die Herstellung von Pillen, Tabletten, Dragees und Hartgelatinekapseln kann man z.B. Lactose, Maisstärke oder Derivate davon, Talk, Stearinsäure oder deren Salze etc. verwenden. Trägerstoffe für Weichgelatinekapseln und Suppositorien sind z.B. Fette, Wachse, halbfeste und flüssige Polyole, natürliche oder gehärtete Öle etc. Als Trägerstoffe für die Herstellung von Lösungen und Sirupen eignen sich z.B.Wasser, Saccharose, Invertzucker, Glukose, Polyole etc. Als Trägerstoffe für die Herstellung von Injektionslösungen eignen sich Wasser, Alkohole, Glycerin, Polyole, pflanzliche Öle etc. Als Trägerstoffe für Mikrokapseln, Implantate oder Rods eignen sich Mischpolymerisate aus Glykolsäure und Milchsäure.

Die pharmazeutischen Präparate können neben den Wirk- und Trägerstoffen noch Zusatzstoffe, wie z.B. Füllstoffe, Streck-, Spreng-, Binde-, Gleit-, Netz-, Stabilisierungs-, Emulgier-, Konservierungs-, Süß-, Färbe-, Geschmacks- oder Aromatisierungs-, Dickungs-, Verdünnungsmittel, Puffersubstanzen, ferner Lösungsmittel oder Lösungsvermittler oder Mittel zur Erzielung eines Depoteffekts, sowie Salze zur Veränderung des osmotischen Drucks, Überzugsmittel oder Antioxidantien enthalten. Sie können auch zwei oder mehrere Verbindungen der allgemeinen Formel I oder deren physiologisch verträglichen Salze enthalten; ferner neben mindestens einer Verbindung der Formel I noch einen oder mehrere andere therapeutisch wirksame Stoffe.

Die Dosis kann innerhalb weiter Grenzen variieren und ist in jedem einzelnen Fall den individuellen Gegebenheiten anzupassen.

Bei oraler Verabreichung kann die Tagesdosis zwischen 0,01 bis 50 mg/kg, vorzugsweise 0,1 bis 5 mg/kg, vorzugsweise 0,3 bis 0,5 mg/kg Körpergewicht zur Erzielung wirksamer Ergebnisse, betragen, bei intravenöser Applikation beträgt die Tagesdosis im allgemeinen etwa 0,01 bis 100 mg/kg, vorzugsweise 0,05 bis 10 mg/kg Körpergewicht. Die Tagesdosis kann, insbesondere bei der Applikation größerer Mengen, in mehrere, z.B. 2, 3 oder 4 Teilverabreichungen aufgeteilt werden. Gegebenenfalls kann es, je nach individuellem Verhalten, erforderlich werden, von der angegebenen Tagesdosis nach oben oder nach unten abzuweichen.

### Beispiele

Die Produkte wurden durch Massenspektren und/oder NMR-Spektren charakterisiert.

### Beispiel 1

### (2S)-Benzoylcarbonylamino-3-[2-((4S)-(3-(4,5-dihydro-1H-imidazol-2-ylamino)-propyl)-2,5-dioxo-imidazolidin-1-yl)-acetylamino]-propionsäure (1.8)

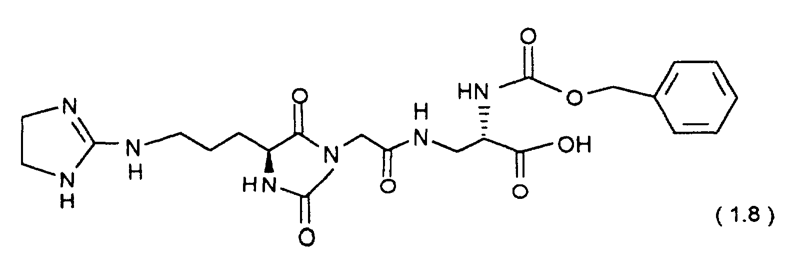

Die Synthese erfolgte nach folgender Reaktionssequenz:

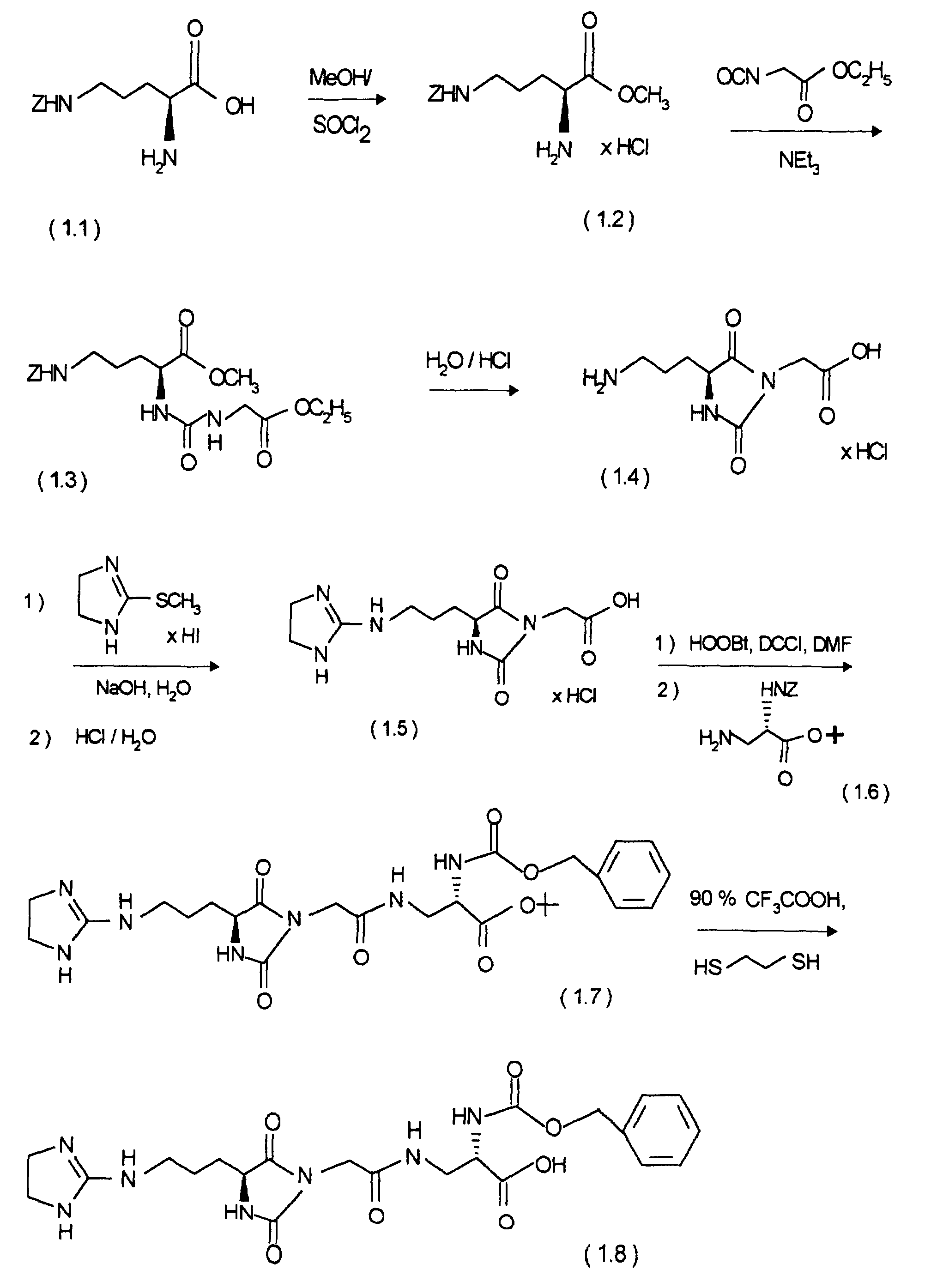

### 1a) (2S)-2-Amino-5-benzyloxycarbonylamino-pentansäure-methylester-hydrochlorid (1.2)

Man tropft unter Eiskühlung in einer Argonatmosphäre 240 ml Thionylchlorid zu 300 ml abs. Methanol und gibt anschließend 40 g (150 mMol) (2S)-2-Amino-5-benzyloxycarbonylamino-pentansäure (1.1) zu und läßt 3 h bei Raumtemperatur und über Nacht bei 4°C reagieren. Die Lösung wird in Methyl-tert.-butylether gegossen, das Lösungsmittel wird abdekantiert und der Rückstand mit Diethylether verrieben. Nach Absaugen erhält man 29,14 g (61 %) an (1.2) als farblosen Feststoff.

### 1b) 5-Benzyloxycarbonylamino-(2S)-(3-ethoxycarbonylmethylharnstoff)pentansäure-methylester (1.3)

Man tropft bei 0°C zu einer Lösung von 9,41 g (29,7 mMol) der Verbindung 11.2) in 150 ml Dichlormethan/Tetrahydrofuran (2:1) unter Rühren 3,83 g (29,7 mMol) Ethylisocyanatoacetat und anschließend 3 g (29,7 mMol) Triethylamin. Nach 30 min bei 0°C wird das Eisbad entfernt und das Reaktionsgemisch 1,5 h bei Raumtemperatur weitergerührt. Nach Entfernen des Lösungsmittels i.Vak. wird der Rückstand mit Essigester über Kieselgel chromatographiert. Die Produktfraktionen werden eingeengt und der Rückstand mit Ether verrieben und abgesaugt. Man erhält 11,02 g (83 %) an (1.3) als farblosen Feststoff.

### 1c) [(4S)-(3-Amino-propyl)-2,5-dioxo-imidazolidin-1-yl]-essigsäure-hydrochlorid (1.4)

10,4 g (42,9 mMol) an (1.3) werden mit 100 ml 6N Salzsäure 45 min. zum Rückfluß erhitzt. Die Lösung wird eingeengt, der Rückstand mit H₂O versetzt und gefriergetrocknet. Man erhält 7,1 g (66 %) an (1.4) als farblosen Feststoff.

### 1d) [(4S)-(3-(4,5-Dihydro-1H-imidazol-2-ylamino)-propyl)-2,5-dioxiimidazolidin-1-yl]-essigsäure-hydrochlorid (1.5)

400 mg (1,59 mMol) an (1.4) und 388 mg (1,59 mMol) 2-(Methylmercapto)-2-imidazolin-hydrojodid werden in 5 ml H₂O gelöst. Man stellt das Gemisch mit 1N NaOH auf pH 9 und erhitzt 2,5 h auf 60°C, wobei der pH-Wert der Lösung durch Zugabe von 1N NaOH auf 9 gehalten wird (Gesamtverbrauch an 1N NaOH: 3.4 ml). Man läßt das Reaktionsgemisch 3 Tage bei Raumtemperatur stehen, stellt mit 1N HCI pH 1 ein, entfernt das Lösungsmittel im Vakuum und chromatographiert den Rückstand mit MeOH/H₂O = 9/1 über Kieselgel. Die Produktfraktionen werden eingeengt und gefriergetrocknet. Man erhält 230 mg (45 %) an (1.5) als farbloses Pulver.

### 1e) (2S)-3-Amino-2-benzyloxycarbonylaminopropionsäure-tert.Butylester (1.6)

10 g (42 mMol) (2S)-3-Amino-2-benzyloxycarbonylamino-propionsäure werden in einem Gemisch aus 100 ml Dioxan, 100 ml Isobutylen und 8 ml konz. H₂SO₄ 3 Tage bei 20 atm. N₂-Druck im Autoklaven geschüttelt. Überschüssiges Isobutylen wird abgeblasen und zur verbleibenden Lösung werden 150 ml Diethylether und 150 ml gesättigte NaHCO₃-Lösung gegeben. Die Phasen werden getrennt und die wäßrige Phase wird 2 mal mit je 100 ml Diethylether extrahiert. Die vereinigten organischen Phasen werden mit 2x100 ml H₂O gewaschen und über Na₂SO₄ getrocknet. Nach Entfernen des Lösungsmittels i. Vak. erhält man 9,58 g (78 %) an (1.6) als blaßgelbes Öl.

### 1f) (2S)-Benzoyloxycabronylamino-3-[2-((4S)-(3-(4,5-dihydro-1H-imidazol-2-ylamino)-propyl)-2,5-dioxo-imidazolidin-1-yl)-acetylamino]-propionsäure-tert.-butylester (1.7)

200 mg (0,7 mMol) an (1.5) und 114 mg (0,7 mMol) HOOBt werden in 5 ml DMF suspendiert und bei 0°C mit 154 mg (0,7 mMol) DCCI versetzt. Man rührt 1h bei 0°C und 1h bei RT und gibt anschließend 206 mg (0,7 mMol) (1.6) zu, rührt 2h bei RT und läßt über Nacht bei RT stehen. Das Lösungsmittel wird i.Vak. entfernt und der Rückstand mit Dichlormethan/Methanol/Eisessig/Wasser = 8/2/0,2/0,2 über Kieselgel chromatographiert. Nach Einengen und Gefriertrocknen erhält man 105 mg (27 %) an (1.7) als farblosen Feststoff.

### 1g) (2S)-Benzyloxycarbonylamin-3-[2-((4S)-(3-(4,5-dihydro-1H-imidazol-2-ylamino)-propyl)-2,5-dioxo-im dazolidin-1-yl)-acetylamino]-propionsäure (1.8)

105 mg (0,188 mMol) an (1.7) werden in einer homogenen Lösung aus 2 ml 90 %ige Trifluoressigsäure und 0,2 ml 1,2-Dimercaptoethan gelöst und 1h bei RT stehen gelassen. Nach Einengen i.Vak. wird der Rückstand zwischen Diethylether/Wasser verteilt und die wäßrige Phase gefriergetrocknet. Nach Chromatographie über ® Sephadex LH 20 mit H₂O/n-Butanol/HOAc = 43/4,3/3,5 und anschließender Gefriertrocknung erhält man 45 mg (48 %) an (1.8) als farblosen Feststoff.

### Beispiel 2

### 3-[2-((4S)-(3-(5-Amino-2H-[1,2,4]triazol-3-ylamino)-propyl)-2,5-dioxo-imidazolidin-1-yl)-acetylamino]-(2S)-benzyloxycarbonylamino-pripionsäure (2.5)

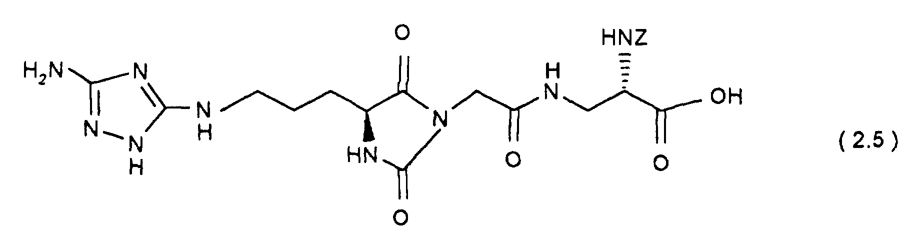

Die Synthese erfolgte nach folgender Reaktionssequenz:

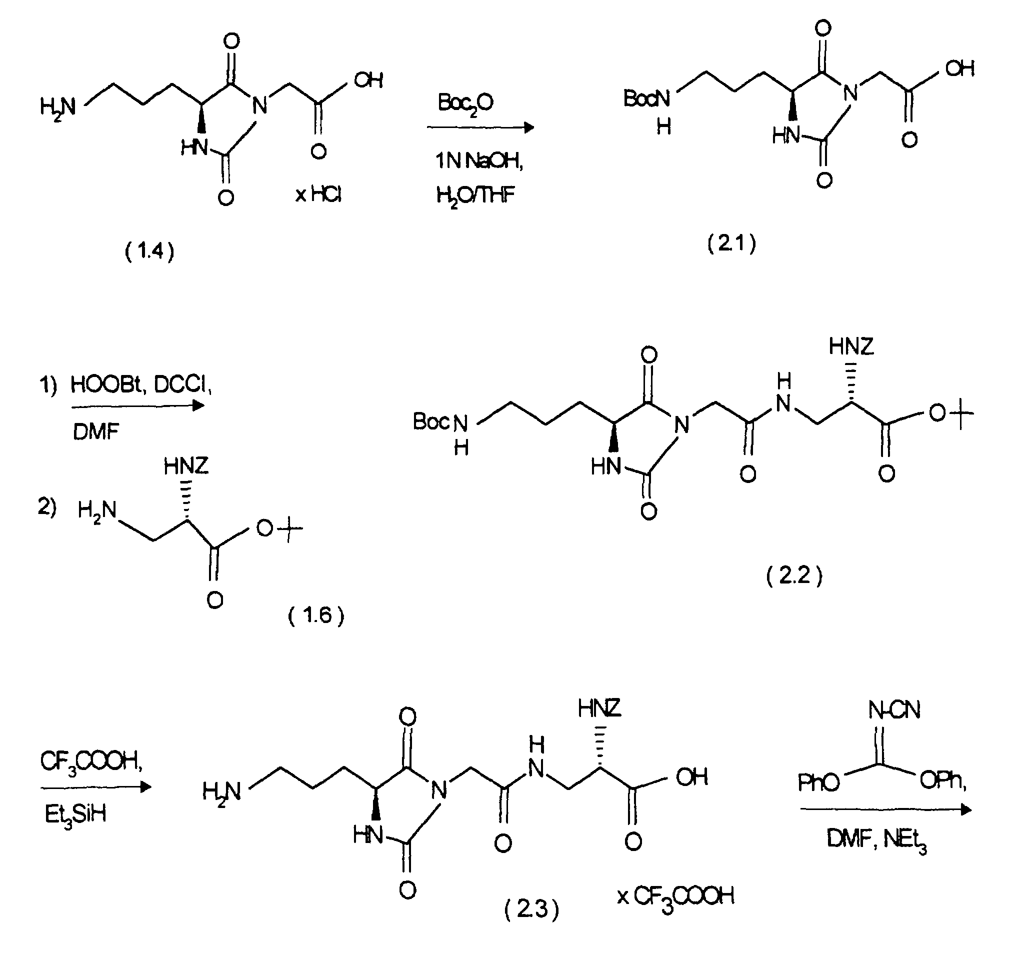

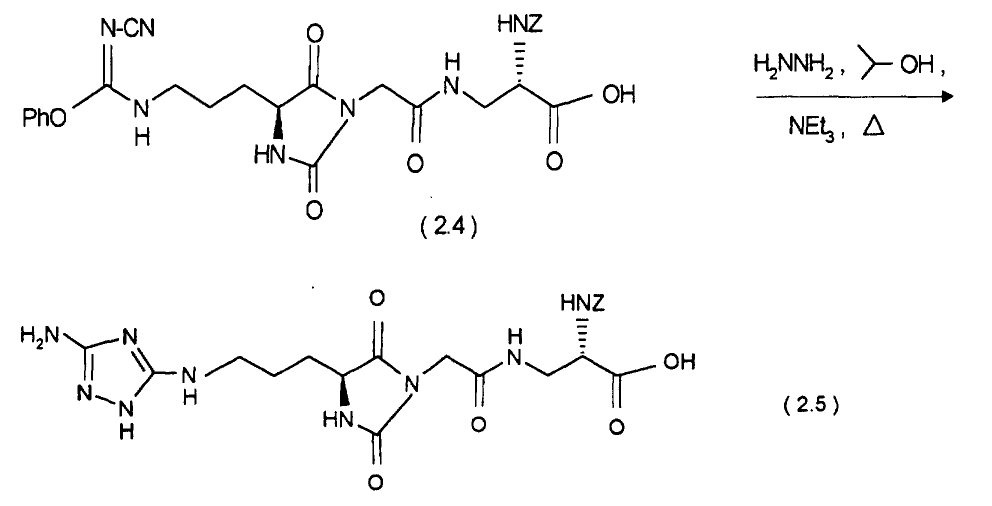

Zur Synthese von (1.4) und (1.6) siehe Beispiel 1.

### 2a) [(4S)-(3-tert.-Butoxycarbonylamino-propyl)-2,5-dioxo-imidazlidin-1-yl]-essigsäure (2.1)

6 g (23,84 mMol) an (1.4) werden in 350 ml THF/H₂O = 2/1 gelöst. Man stellt bei 0°C mit 1N NaOH pH 10,5 ein,gibt 6,24 g (28,61 mMol) Di-tert.-butyldicarbonat zu und hält den pH-Wert der Lösung durch Zugabe von 1N NaOH zwischen 9-10,5. Man rührt 1h bei 0°C und läßt über Nacht bei 4°C stehen. Man stellt mit Phosphatpuffer pH 4 ein und entfernt das Lösungsmittel i.Vak.. Der Rückstand wird in Methanol verrieben, filtriert und das Filtrat eingeengt. Nach Chromatographie über Kieselgel mit Dichlormethan/MeOH/ Eisessig/Wasser = 7/3/0,3/0,3 erhält man 5,65 g (75 %) eines zähen Sirups an (2.1).

### 2b) (2S)-Benzyloxycarbonalamino-3-[2-((4S)-(3-tert.-butoxycarbonyl-aminopropyl)-2,5-dioxo-imidazolidin-1-yl)-acetylamino]-pripionsäure-tert.-butylester (2.2)

2,8 g (8,9 mMol) an (2.1) und 1,45g (8,9 mMol) HOOBt werden in 50 ml DMF gelöst und bei 0°C mit 1,95 g (8,9 mMol) DCCI versetzt. Nach 1h bei 0°C und 1h bei RT werden 2,6 g (8,9 mMol) an (1.6) zugegeben, 2h bei RT gerührt und das Reaktionsgemisch über Nacht bei RT stehen gelassen. Nach Filtration wird das Filtrat eingeengt, der Rückstand zwischen H₂O und Essigester verteilt, die organische Phase über Na₂SO₄ getrocknet, das Lösungsmittel i.Vak. entfernt und der Rückstand über Kieselgel mit Essigester/Heptan 9/1 bis 6/4 chromatographiert. Man erhält 2,98 g (57 %) an (2.2).

### 2c) 3-[2-((4S)-(3-Aminopropyl)-2,5-dioxo-imidazolidin-1-yl)-acetylamino]-(2S)-benzyloxycarbonylamino-propionsäure (2.3)

2,9 g (4,9 mMol) an (2.2) werden in einem Gemisch aus 20 ml Dichlormethan, 9,8 ml Trifluoressigsäure und 2,35 ml Triethylsilan gelöst. Nach 3,5 h bei RT wird das Gemisch eingeengt und anschließend gefriergetrocknet. Der Rückstand wird in Diethylether verrieben, getrocknet, in wenig Methanol kristallisiert und mit Ether verrieben. Man erhält 1,34 g (50 %) an (2.3) als farblosen Feststoff.

### 2d) 3-[2-((4S)-4-(3-(Phenoxy-N-Cyano-iminocarbonylamino)propyl)-2,5-dioxo-imidazolidin-1-yl)acetylamino]-(2S)-benzyloxycarbonylamino-propionsäure (2.4)

400 mg (0,73 mMol) an (2.3) werden in 10 ml DMF gelöst. Man gibt 0,14 ml Triethylamin und anschließend 190,7 mg (0,8 mMol) Cyancarbamidsäurediphenylester in2 ml DMF zu. Nach 2h Rühren bei RT wird das Lösungsmittel i.Vak. entfernt, der Rückstand in 50 ml 5 %iger Essigsäurelösung gelöst und gefriergetrocknet. Nach Chromatographie über Kieselgel mit Methanol erhält man 260 mg (61 %) an (2.4).

### 2e) 3-[2-((4S)-(3-(5-Amino-2H-[1,2,4]triazol-3-ylamino)-propyl)-2,5-dioxo-imidazolidin-1-yl)-acetylamino]-(2S)-benzyloxycarbonylamino-propionsäure (2.5)

260 mg (0,45 mMol) an (2.4) werden in 10 ml Isopropanol suspendiert und mit 62,4 µl (0,45 mMol) Triethylamin versetzt. Mangibt 28,5 µl (0,585 mMol) Hydrazin zu und erhitzt 10h zum Rückfluß. Man läßt über Nacht bei RT stehen, saugt den Niederschlag ab und wäscht mit Butanol, THF und Ether. Nach Chromatographie über ® Sephadex LH 20 mit H₂O/n-Butanol/HOAc = 43/4,3/3,5 und anschließender Gefriertrocknung erhält man 80 mg (34 %) an (2.5) als farblosen Feststoff.

### Beispiel 3

### (2S)-Benzyloxycarbonylamino-3-[((4S)-(Guanidinoacylamino-methyl)-2,5-dioxo-imidazolidin-1-yl)-acetylaminol-propionsäure (3.11)

Die Synthese erfolgte nach folgender Reaktionssequenz:

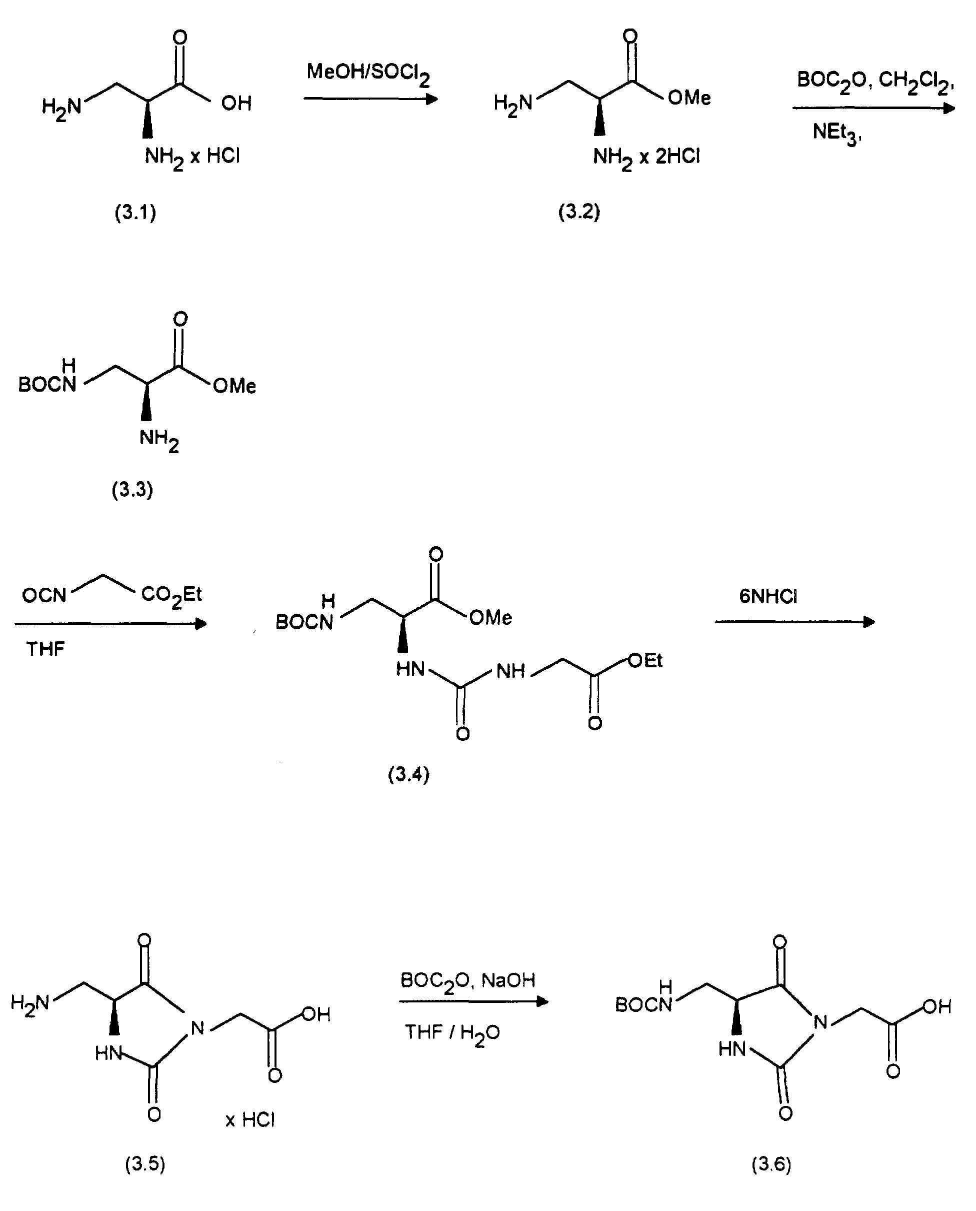

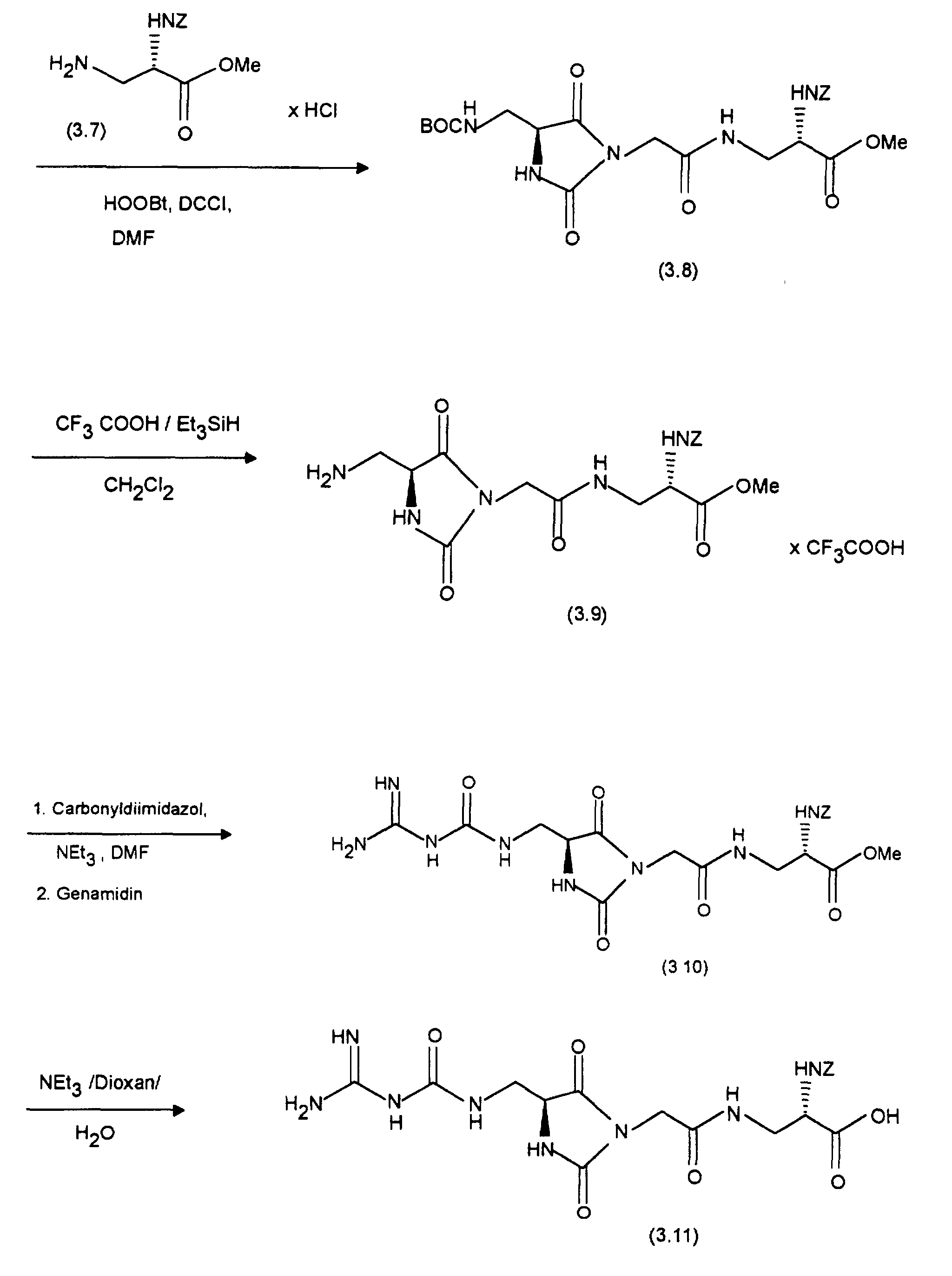

### 3a) (2S),3-Diamino-propionsäuremethylester-dihydrochlorid (3.2)

Man tropft bei -15°C in einer Argonatmosphäre 35 ml Thionylchlorid zu 60 ml abs. Methanol und gibt anschließend 25 g (240 mMol) (2S),3-Diaminopropionsäure-hydrochlorid (3.1) und weitere 100 ml Methanol zu. Nach 16 h Rühren bei Raumtemperatur wird das Gemisch 4h unter Rückfluß erhitzt.Die Lösung wird in Diisopropylether gegossen, das Lösungsmittel wird abdekantiert, und der Rückstand mit Methanol über Kieselgel chromatographiert. Die Produktfraktionen werden eingeengt und der Rückstand mit Diisopropylether verrieben.Nach Absaugen des Niederschlags und Trocknen über Paraffin erhält man 26,3 g (57 %) an (3.2) als blaßgelben Feststoff.

### 3 b) (2S)-Amino-3-tert.-butoxycarbonylamino-propionsäuremethylesterhydrochlorid (3.3)

Zu einer Suspension von 26 g (220 mMol) an (3.2) in 4,4 I Dichlormethan gibt man bei -78°C 75,8 ml Triethylamin und anschließend tropfenweise eine Lösung von 26,78 g (123 mMol) Di-tert.butyl-dicarbonat in 220 ml Dichlormethan, läßt auf 0°C erwärmen und 1,5 h bei dieser Temperatur rühren. Das Reaktionsgemisch wird eingeengt und mit Essigester/Methanol = 20/1 über Kieselgel chromatographiert. Man erhält 15,67 g (58%) an (3.3) als blaßgelben Sirup.

### 3c) (2S)-Ethyloxycarbonylmethyl-aminocarbonylamino-3-tert.butoxycarbonylamino-propionsäure-methylester (3.4)

Man tropft zu einer Suspension von 10 g (45,8 mMol) der Verbindung (3.3) in 100 ml Tetrahydrofuran 5,13 ml (45,8 mMol) Ethylisocyanatoacetat , läßt 3 h bei Raumtemperatur rühren, entfernt das Lösungsmittel im Vakuum und chromatographiert den Rückstand mit Essigester/Methanol = 20/1 über Kieselgel. Man erhält 13,44 g (85%) an (3.4) als blaßgelben Öl.

### 3d) 2-[(4S)-(Aminomethyl)-2,5-dioxoimidazolidin]-essigsäure-hydrochlorid (3.5)

13,2 g (34 mMol) an (3.4) werden mit 150 ml 6N Salzsäure 45 min. zum Rückfluß erhitzt. Die Lösung wird eingeengt und der Rüchstand gefriergetrocknet. Nach Chromatographie über Kieselgel mit Dichlormethan/Methanol = 7/3 und Gefriertrocknen der Produktfraktionen erhält man 6.4 g (84 %) an (3.5) als farblosen Feststoff.

### 3e) 2-[(4S)-(tert.Butoxycarbonylamino-methyl)-2,5-dioxoimidazolidin]-essigsäure (3.6)

Man gibt zu einer Lösung von 4,4 g (19,7 mMol) an (3.5) in 300 ml Terahydrofuran/Wasser = 2/1 bei 0°C 1N NaOH bis pH 10,5 erreicht ist (Verbrauch: 26 ml), gibt 5,16 g (23,6 mMol) Di-tert.-butyldicarbonat zu und hält durch Zugabe von 1N NaOH den pH zwischen 9,5 - 10,5. Nach 1h bei 0°C und 2h bei Raumtemperatur läßt man das Gemisch über Nacht bei 4°C stehen, stellt mit 1N HCI pH 7 und anschließend mit Phosphatpuffer pH 4.1 ein. Das Lösungsmittel wird im Vakuum entfernt, der Rückstand mit Methanol verrieben, filtriert, das Filtrat eingeengt und der Rückstand mit Dichlormethan/Methanol/Essigsäure/Wasser = 8/2/0.2/0.2 über Kieselgel chromatographiert. Die Produktfraktionen werden eingeengt und gefriergetrocknet. Ausbeute an (3.6): 3,6 g (64%), farbloser Feststoff.

### 3f) 3-Amino-(2S)-benzyloxycarbonylamino-propionsäure-methylester-hydrochlorid (3.7)

Man tropft zu 50 ml absolutem Methanol bei -15°C 7,4 ml (100,8 mMol) Thionylchlorid. Man gibt 12 g (50,4 mMol) 3-Amino-(2S)-benzyloxycarbonylamino-propionsäure-hydrochlorid und anschließend 40 ml absolutes Methanol zu. Nach 45 min. Rühren bei -15°C und 20 h bei Raumtemperatur wird das Reaktionsgemisch in Diisopropylether gegossen, der Niederschlag abgesaugt und am Hochvakuum getrocknet. Man erhält 14,18 g (98 %) an (3.7) als farblosen Feststoff.

### 3g) (2S)-Benzyloxycarbonylamino-3-[((4S)-(tert.butoxycarbonylamino-methyl)-2,5-dioxo-imidazolidin-1-yl)-acetylamino]-propionsäure-methylester (3.8)

Man gibt zu einer Lösung von 1,5 g (5,2 mMol) an (3.6) in 20 ml abs. DMF bei 0°C 848 mg (5,2 mMol) HOOBt und anschließend 1,144 g (5,2 mMol) DCCI. Nach 1h Rühren bei 0°C und 1h bei Raumtemperatur gibt man 1,5 g (5,2 mMol) an (3.7) und 0,67 ml N-Ethylmorpholin zu und rührt 3h bei Raumtemperatur. Der Niederschlag wird abfiltriert, das Filtrat eingeengt, der Rückstand in Essigester aufgenommen und nacheinander mit gesättigter NaHCO₃-Lösung, KHSO₄/ K₂SO₄-Lösung und Wasser gewaschen und die organische Phase über Natriumsulfat getrocknet. Nach Filtration und Entfernen des Lösungsmittel im Vakuum wird der Rückstand mit Essigester/Heptan = 6/4 bis Essigester über Kieselgel chromatographiert und man erhält 1,75 g (65%) an (3.8).

### 3h) 3-[((4S)-(Amino-methyl)-2,5-dioxo-imidazolidin-1-yl)-acetylamino]-(2S)-benzyloxycarbonylamino-propionsäure-methylester-trifluoressigsäuresalz (3.9)

1,7 g (3,26 mMol) an (3.8) in 6,7 ml Dichlormethan, 3,3 ml Trifluoressigsäure und 0,78 ml Triethylsilan werden 4h bei Raumtemperatur gerührt.Die Lösung wird in Diethylether gegossen und der Niederschlag abfiltriert. Man erhält 1,54 g (88%) an (3.9) als farblosen Feststoff.

### 3g) (2S)-Benzyloxycarbonylamino-3-[((4S)-(guanidinoacylamino-methyl)-2,5-dioxo-imidazolidin-1-yl)-acetylamino]-propionsäure-methylester (3.10)

Man gibt zu einer Lösung von 500 mg (0.93 mMol) an (3.9) in 10 ml absolutem DMF 0,129 ml Triethylamin und anschließend bei 0°C eine Lösung von 152 mg (0.93 mMol) Carbonyldiimidazol in 10 ml absolutem DMF. Nach 4 h Rühren bei Raumtemperatur gibt man 89 mg (1,86 mMol) Guanidin zu, läßt 2 h bei Raumtemperatur rühren, entfernt das Lösungsmittel im Vakuum und chromatographiert den Rückstand mit Dichlormethan/Methanol/Essigsäure/Wasser = 8,5/1,5/0,15/0,15 über Kieselgel. Nach Einengen der Produktfraktionen und Gefriertrocknen erhält man 300 mg (64 %) an (3.10) als farblosen Feststoff.

### 3h) (2S)-Benzyloxycarbonylamino-3-[((4S)-(guanidinoacylamino-methyl)-2,5-dioxo-imidazolidin-1-yl)-acetylamino]-propionsäure (3.11)

180 mg (0.32 mMol) an (3.10) werden in einem Gemisch aus Dioxan/Wasser/Triethylamin = 1/1/1 gelöst. Nach 16 h bei Raumtemperatur wird das Gemisch einrotiert, mit Wasser versetzt und gefriergetrocknet. Der Rückstand wird mit Dichlormethan/Methanol/Essigsäure/Wasser = 8/2/0,2/0,2 über Kieselgel chromatographiert. Die Produktfraktionen werden einrotiert, mit Wasser versetzt, gefriergetrocknet und der Rückstand anschließend mit Essigester und Diethylether verrieben. Nach Absaugen erhält man 62 mg (39%) an (3.11) als farblosen Feststoff.

ES(+)-MS: 493 (M + H)⁺

### Beispiel 4

### (2S)-Benzyloxycarbonylamino-3-[((4S)-(3-(2-pyrimidylamino)-propyl)-2,5-dioxo-imidazolidin-1-yl)-acetylamino]-propionsäure (4.2)

Die Synthese erfolgte nach folgender Reaktionssequenz:

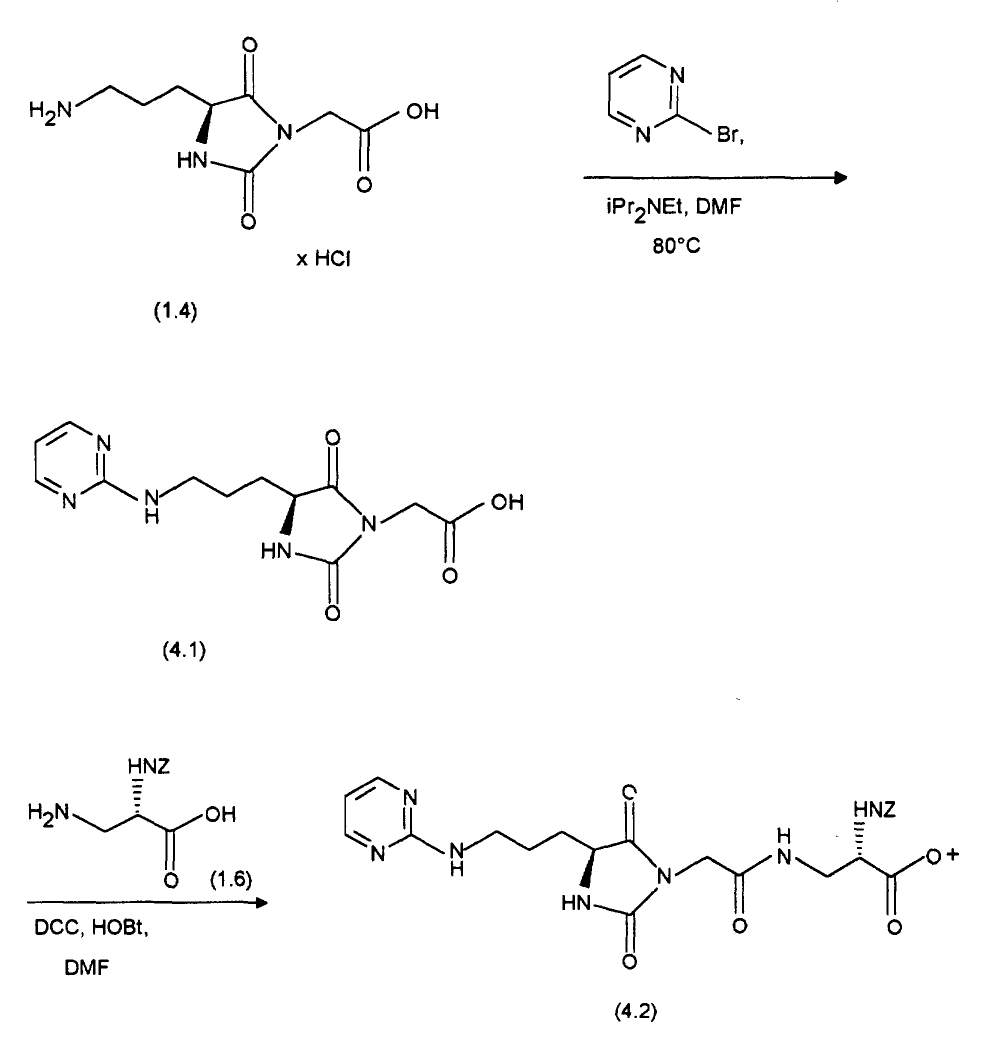

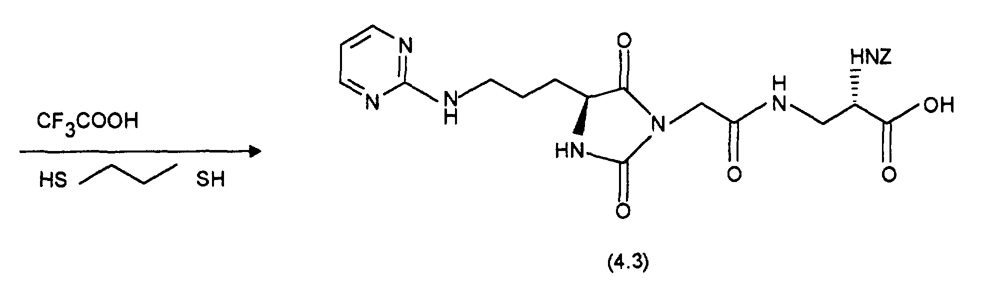

### 4a) 2-[(4S)-(3-(2-Pyrimidylamino)-propyl)-2,5-dioxoimidazolidin]-essigsäure (4.1)

Ein Gemisch aus 1 g (4 mMol) an (1.4), 632 mg (4 mMol) an 2-Brompyrimidin und 2,04 ml (12 mMol) Diisopropylethylamin (DIPEA) in 9 ml DMF werden 26 h auf 80°C erhitzt. Das Lösungsmittel wird im Vakuum entfernt und der Rückstand mit Dichlormethan/Methanol/Essigsäure/Wasser = 8/2/0,2/0,2 über Kieselgel chromatographiert. Die Produktfraktionen werden eingeengt und man erhält 144 mg (12 %) an (4.1) als farblosen Feststoff.

### 4b) (2S)-Benzyloxycarbonylamino-3-[((4S)-(3-(2-pyrimidylamino)-propyl)-2,5-dioxo-imidazolidin-1-yl)-acetylamino]-propionsäure-tert.butylester (4.2)

Man gibt zu einer Lösung von 128 mg (0,44 mMol) an (4.1), 128 mg (0,44 mMol) an (1.6), 60 mg (0,44 mMol) HOBt und 0.035 ml N-Ethyl-morpholin in 5 ml DMF 97 mg (0,44 mMol) DCCI. Nach 1 h bei 0°C und 16 bei Raumtemperatur wird das Lösungsmittel im Vakuum entfernt und der Rückstand mit Dichlormethan/Methanol/Essigsäure/Wasser = 9,5/0,5/0,05/0,05 über Kieselgel chromatographiert. Man erhält 180 mg (72 %) an (4.2).

### 4c) (2S)-Benzyloxycarbonylamino-3-[((4S)-(3-(2-pyrimidylamino)-propyl)-2,5-dioxo-imidazolidin-1-yl)-acetylamino]-propionsäure (4.3)

170 mg (0,3 mMol) an (4.2) werden in einem Gemisch aus 2 ml 90%-ige Trifluoressigsäure und 0,2 ml 1,2-Dimercaptoethan gelöst. Nach 1 h bei Raumtemperatur wird das Gemisch in Diethylether gegeben, der Niederschlag abzentrifugiert, nochmals mit Diethylether aufgeschlämmt und erneut zentrifugiert. Nach Lösen in Wasser und Gefriertrocknen wird der Rückstand mit Wasser/Butanol/Essigsäure = 43/4,3/3,5 über Sephadex LH 20 chromatographiert. Nach Gefriertrocknen erhält 76 mg (49%) an (4.3) als farblosen Feststoff.

ES(+)-MS: 514 (M + H)⁺

### Beispiel 5

### (2S)-Benzyloxycarbonylamino-3-[((4S)-(3-(benzimidazolyl-2-amino)-propyl)-2,5-dioxo-imidazolidin-1-yl)-acetylamino]-propionsäure (5.5)

Die Synthese erfolgte nach folgender Reaktionssequenz:

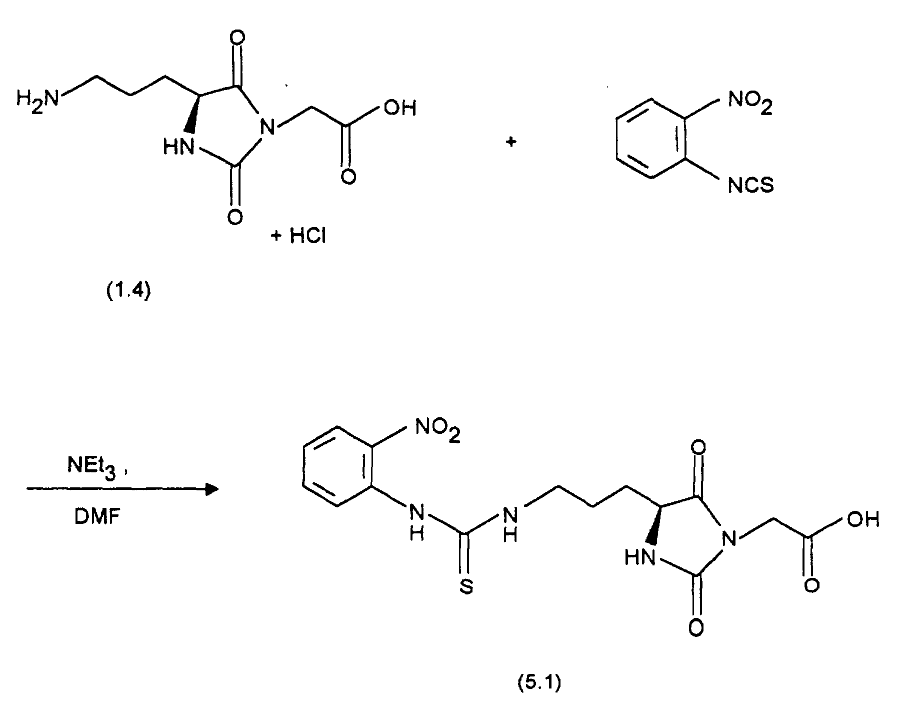

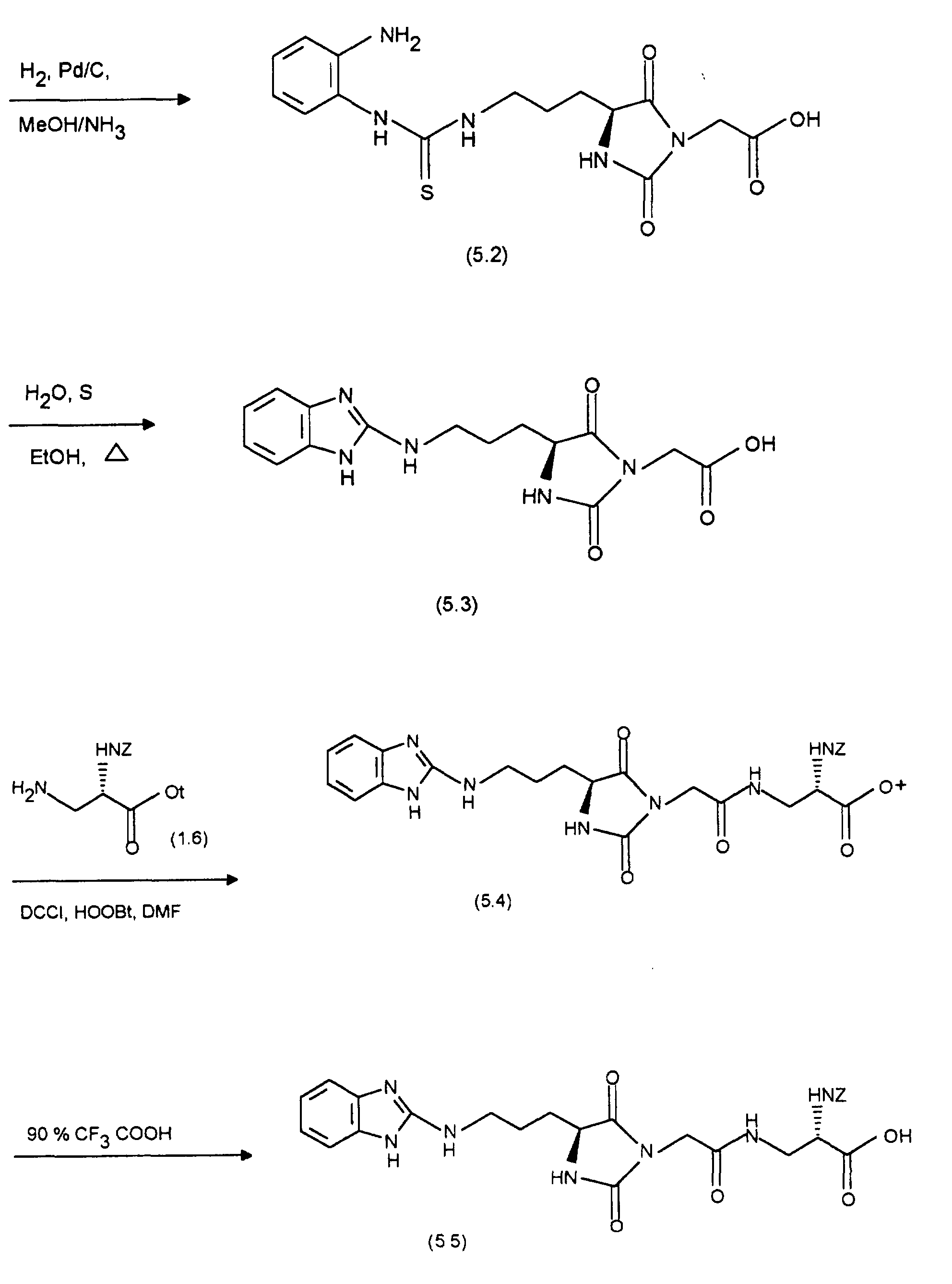

### 5a) 2-[(4S)-(3-(3-(2-Nitrophenyl-)-thioharnstoff)-propyl)-2,5-dioxo-imidazolidin-1-yl]-essigsäure (5.1)

Man gibt zu einer Lösung von 4,5 g (17,88 mMol) an (1.4) in 100 ml DMF bei 0°C 4,96 ml (35,76 mMol) Triethylamin und anschließend 3,22 g (17,88 mMol) 2-Nitrophenylisothiocyanat. Nach 4h Rühren bei Raumtemperatur wird das Lösungsmittel im Vakuum entfernt und der Rückstand zwischen Dichlormethan und 10%-iger Essigsäure verteilt. Die wäßrige Phase wird 2 mal mit Dichlormethan extrahiert und die organische Phase über Natriumsulfat getrocknet. Nach Filtration wird das Lösungsmittel im Vakuum entfernt und der Rückstand über Kieselgel chromatographiert. Die Produktfraktionen werden eingeengt und man erhält 3,16 g (45 %) an (5.1).

### 5b) 2-[(4S)-(3-(3-(2-Aminophenyl)-thioharnstoff)-propyl)-2,5-dioxo-imidazolidin-1-yl]-essigsäure (5.2)

4,6 g (11,6 mMol) an (5.1) in 40 ml absolutem Ethanol werden mit 3,8 g 10% Pd/C versetzt. Man gibt 200 ml mit Ammoniak gesättigtes Ethanol zu und hydriert 6 h bei Raumtemperatur. Der Katalysator wird abfiltriert, das Filtrat eingeengt und der Rückstand (1,85 g) direkt zur Synthese von (5.4) eingesetzt.

### 5c) 2-[(4S)-(3-(benzimidazolyl-2-amino)-propyl)-2,5-dioxo-imidazolidin-1-yl]-essigsäure (5.3)

1,85 g an (5.3) in 35 ml Ethanol werden mit 3 g Quecksilberoxid und 730 mg Schwefel 16 h unter Rückfluß erhitzt. Es wird filtriert und der Rückstand 5 mal mit Wasser ausgekocht. Die vereinigten Wasserphasen werden gefriergetrocknet. Man erhält 814 mg (21 % bezogen auf (5.1)) an (5.3) als farblosen Feststoff.

### 5d) (2S)-Benzyloxycarbonylamino-3-[((4S)-(3-(benzimidazolyl-2-amino)-propyl)-2,5-dioxo-imidazolidin-1-yl)- acetylamino]-propionsäure-tert.butylester (5.4)

Die Synthese von (5.4) erfolgt durch Reaktion von (5.3) mit (1.6) wie in Beispiel 4 bei der Herstellung von (4.2) aus (4.1) und (1.6) beschrieben. Aus 205 mg (0,56 mMol) an (5.3) erhält man nach Chromatographie des Rohproduktes über Kieselgel mit Dichlormethan/Methanol/Essigsäure/Wasser = 9/1/0,1/0,1 und 8/2/0,2/0,2 und anschließender Gefriertrocknung der Produktfraktionen 255 mg (75 %) an (5.4) als farblosen Feststoff.

### 5e) (2S)-Benzyloxycarbonylamino-3-[((4S)-(3-(benzimidazolyl-2-amino)-propyl)-2,5-dioxo-imidazolidin-1-yl)- acetylamino]-propionsäure (5.5)

250 mg (0,41 mMol) der Verbindung (5.4) werden in 3 ml 90%-iger Trifluoressigsäure 1 h bei Raumtemperatur gerührt. Die Trifluoressigsäure wird im Vakuum entfernt, der Rückstand mit Wasser versetzt und gefriergetrocknet. Der Rückstand wird mit Wasser/Butanol/Essigsäure = 43/4,3/3,5 über Sephadex LH 20 chromatographiert. Nach Gefriertrocknen erhält 155 mg (66 %) an (5.5) als farblosen Feststoff.

ES(+)-MS: 552 (M + H)⁺

### Beispiel 6:

### (2S)-Benzyloxycarbonylamino-3-[((4S)-(3-(cis-3a,4,5,6,7,7a-Hexahydro-1H-benzimidazol-2-ylamino)-propyl)-2,5-dioxo-imidazolidin-1-yl)-acetylamino]-propionsäure (6.4)

Die Synthese erfolgte nach folgender Reaktionssequenz:

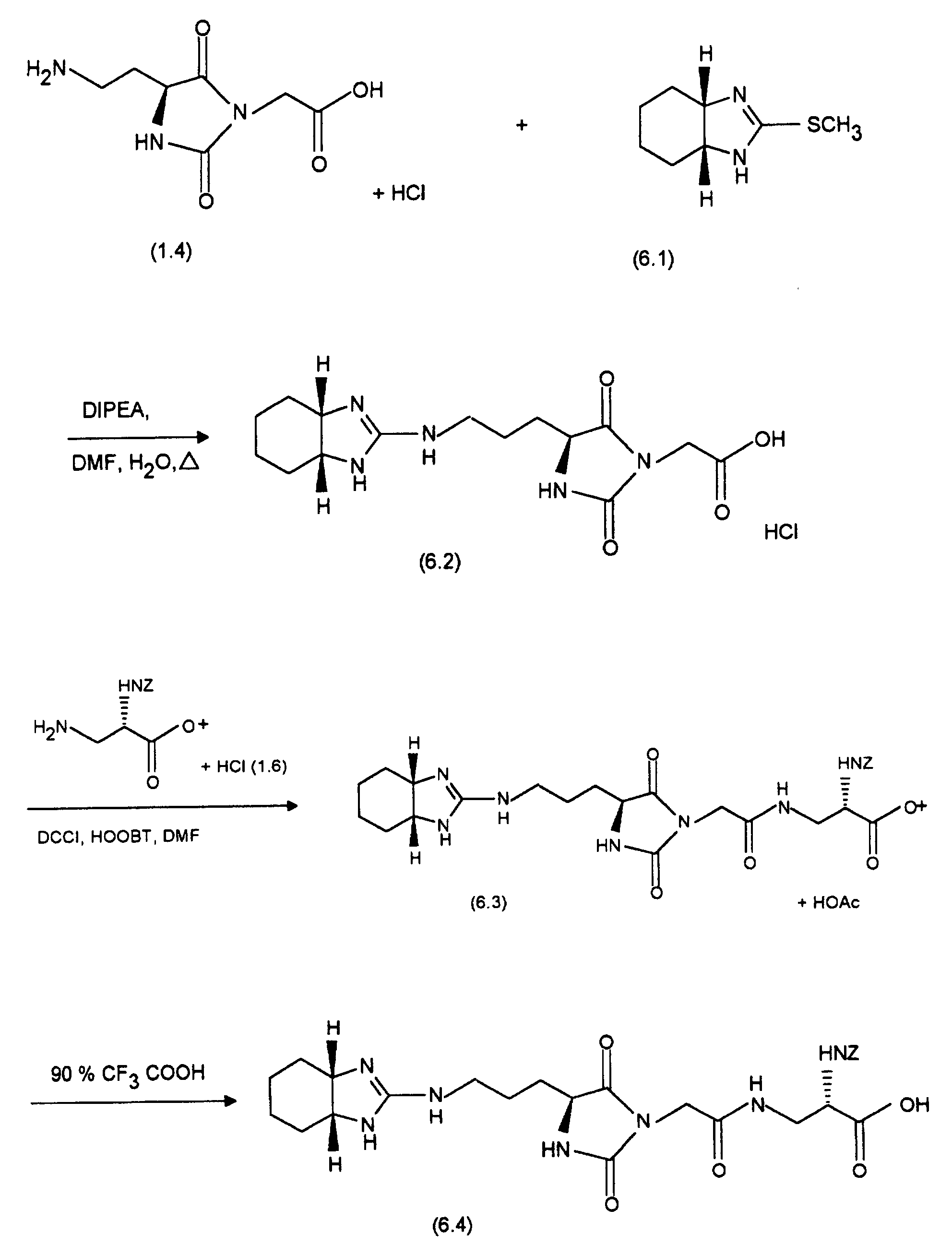

### 6a) [(4S)-(3-(cis-3a,4,5,6,7,7a-Hexahydro-1H-benzimidazol-2-ylamino)-propyl)-2,5-dioxo-imidazolidin-1-yl]-essigsäure-hydrochlorid (6.2)

Eine Lösung von 298 mg (1 mMol) an (6.1) (hergestellt nach G.D.Hartmann et al., WO 95/32710, S. 115) und 251 mg (1mMol) an (1.4) in 6 ml DMF wird mit 0.51 ml DIPEA und 6 Tropfen Wasser versetzt und 24 h bei 100°C gerührt. Das Lösungsmittel wird im Vakuum entfernt und der Rückstand mit Dichlormethan/Methanol/Essigsäure/Wasser = 9/1/0,1/0,1 über Kieselgel und anschließend die vereinigten Produktfraktionen mit Wasser/Butanol/Essigsäure = 43/4,3/3,5 über Sephadex LH 20 chromatographiert. Die Produktfraktionen werden vereinigt und gefriergetrocknet. Nach Überführen ins Hydrochlorid erhält man 130 mg (35%) an (6.2) als farblosen Feststoff.

### 6b) (2S)-Benzyloxycarbonylamino-3-[((4S)-(3-(cis-3a,4,5,6,7,7a-Hexahydro-1H-benzimidazol-2-ylamino)-propyl)-2,5-dioxo-imidazolidin-1-yl)-acetylamino]-propionsäure-tert.butylester-essigsäuresalz (6.3)

Die Synthese von (6.3) erfolgt durch Reaktion von (6.2) mit (1.6) wie in Beispiel 4 bei der Herstellung von (4.2) aus (4.1) und (1.6) beschrieben. Aus 130 mg (0,35 mMol) an (6.2) erhält man nach Chromatographie des Rohproduktes über Kieselgel mit Dichlormethan/Methanol/Essigsäure/Wasser = 8/2/0,2/0,2 und anschließend 9/1/0,1/0,1 und anschließender Gefriertrocknung der Produktfraktionen 45 mg (19 %) an (6.3) als farblosen Feststoff.

### 6c) (2S)-Benzyloxycarbonylamino-3-[((4S)-(3-(cis-3a,4,5,6,7,7a-Hexahydro-1H-benzimidazol-2-ylamino)-propyl)-2,5-dioxo-imidazolidin-1-yl)-acetylamino]-propionsäure (6.4)

40 mg ( 0.168 mMol) an (6.4) in 1 ml 90%-iger Trifluoressigsäure werden 1 h bei Raumtemperatur gerührt. Nach Entfernen der Trifluoressigsäure im Vakuum und Reinigung des Rohproduktes mittels präperativer HPLC über RP18 und anschließender Gefriertrocknung der Produktfraktionen erhält man 13 mg (14%) an (6.4) als farblosen Feststoff.

ES(+)-MS: 558 (M+H)⁺

### Beispiel 7

### (2S)-Benzyloxycarbonylamino-3-[2-((4S)-(N'-(4,5-dihydro-1H-imidazol-2-yl)-hydrazinocarbonylmethyl]-2,5-dioxo-imidazolidin-1-yl)-acetylamino]-propionsäure (7.8)

Die Synthese erfolgte nach folgender Reaktionssequenz:

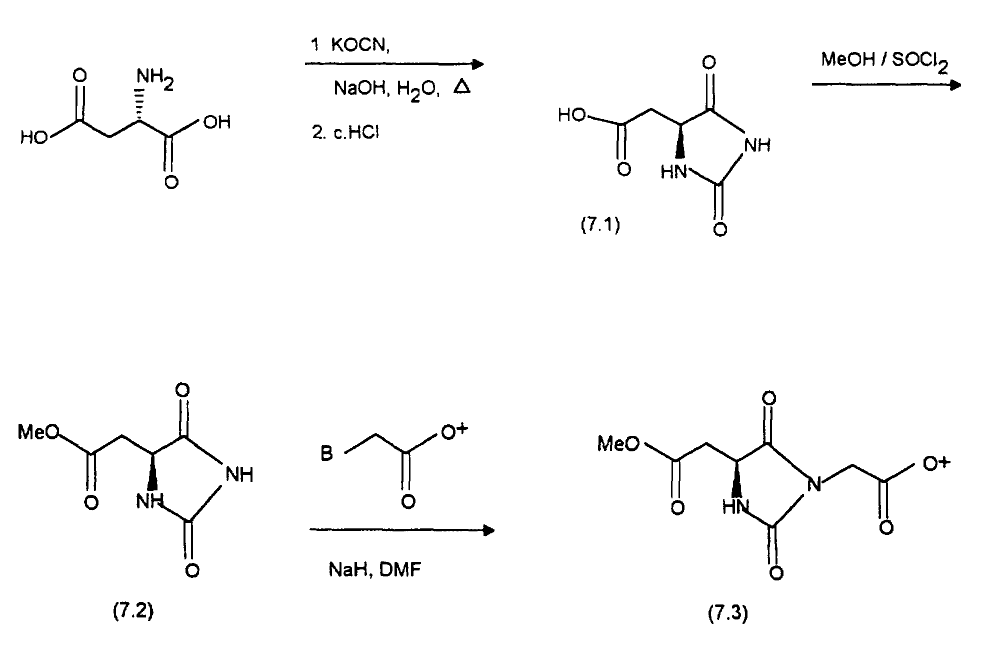

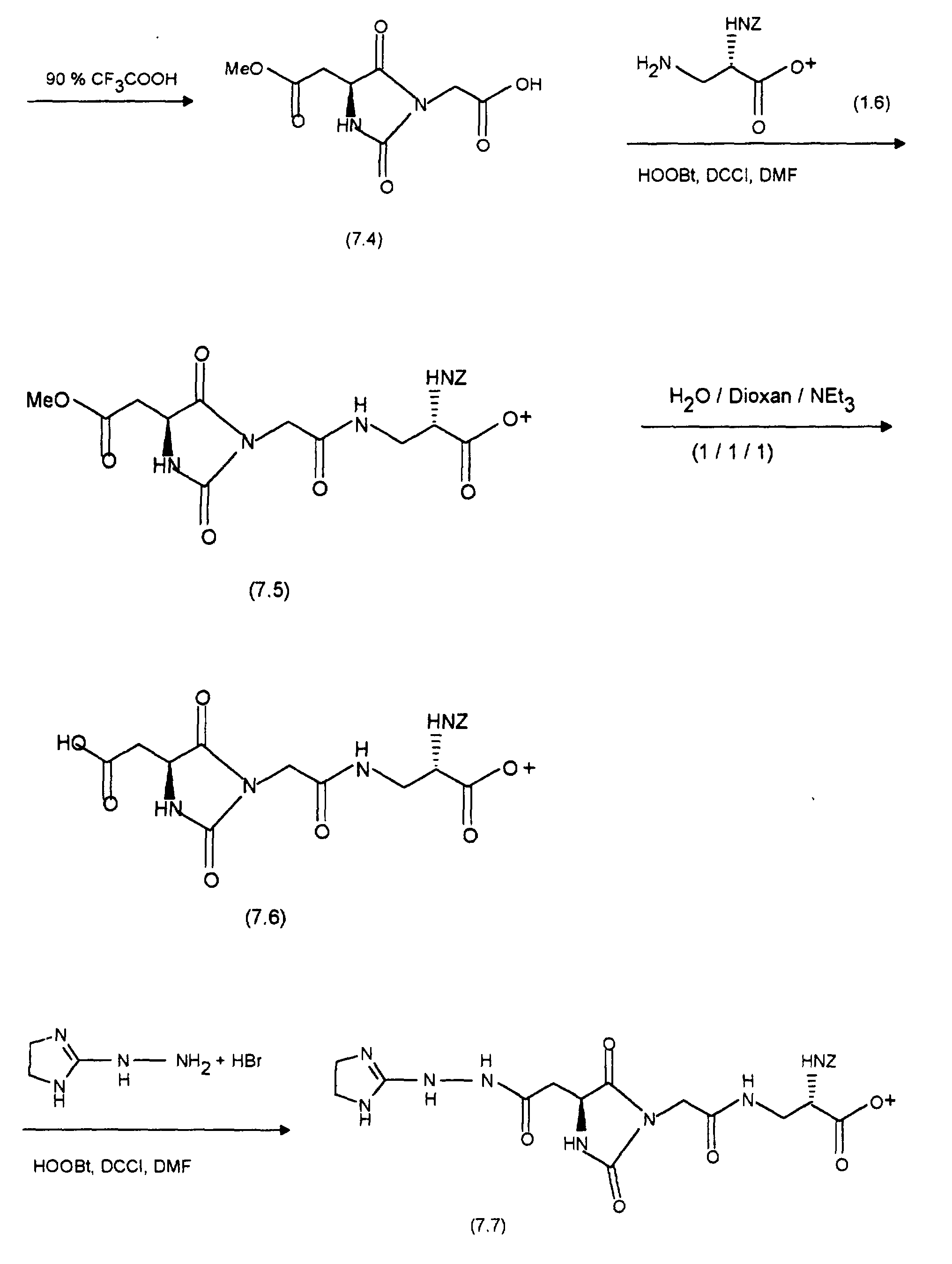

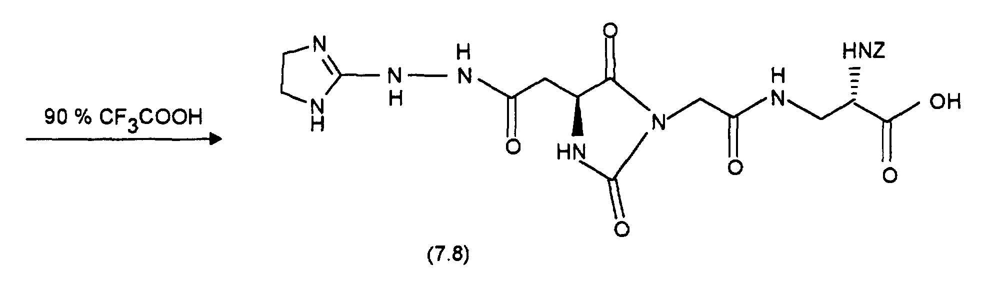

### 7a) (4S)-Carboxy-methyl-2,5-dioxo-imidazolidin (7.1)

Man gibt zu einer Suspension von 10 g (75 mMol) L-Asparaginsäure in Wasser bei 80 °C unter Rühren 5,5 ml 11,7 N Natronlauge und 7,5 g (92,5 mMol) Kaliumcyanat. Man stellt im Verlauf 1 h durch portionsweise Zugabe von conz. HCI bei 85°C jeweils pH 7 ein (Verbrauch: 1,5 ml). Anschließend wird mit 5,5 ml conz. HCI das Reaktionsgemisch auf pH 3,5 eingestellt. Man gibt weitere 9,5 ml conz. HCI zu, rührt 2 h bei 85°C und läßt die Reaktionslösung 18 h bei Raumtemperatur stehen. Der Niederschlag wird abgesaugt, mit wenig eiskaltem Wasser gewaschen und über P₂O₅ getrocknet. Man erhält 8,49 g (72 %) an (7.1) als farblosen Feststoff.

### 7b) (4S)-Methoxycarbonyl-methyl-2,5-dioxo-imidazolidin (7.2)

Man gibt bei -15°C zu 70 ml Methanol 7,9 ml (107 mMol) Thionylchlorid, 8,47 g (53,5 mMol) an (7.1) und rührt das Reaktionsgemisch 24 h bei Raumtemperatur. Anschließend wird die Lösung in Diethylether gegossen, der Niederschlag abgesaugt und mit Diethylether gewaschen. Nach Trocknen im Hochvakuum erhält man 5,12 g (56 %) an (7.2) als farblosen Feststoff. Einengen der Etherphase und Verreiben des Rückstandes mit Diethylether liefert nach Trocknen im Hochvakuum weitere 2,79 g (30 %) an (7.2).

### 7c) [(4S)- Methoxycarbonyl-methyl-2,5-dioxo-imidazolidin]-essigsäure-tert.butylester (7.3)

Man gibt unter Argonatmosphäre zu einer Suspension von 1.02 g (23,2 mMol) Natriumhydrid (55%-ig in Öl) in 20 ml absolutem DMF unter Eiskühlung 4 g (23,2 mMol) an (7.2). Nachdem die Wasserstoffentwicklung beendet ist werden 4,53 g (23,2 mMol) Bromessigsäure-tert-butylester zugegeben. Nach 1 h Rühren bei 0°C, 2 h bei Raumtemperatur und Stehen über Nacht wird das Lösungsmittel entfernt und der Rückstand mit Dichlormethan/Methanol = 40/1 über Kieselgel chromatographiert. Die Produktfraktionen werden eingeengt und der Rückstand mit Diethylether verrieben. Man erhält 4,21 g (63 %) an (7.3).

### 7d) [(4S)- Methoxycarbonyl-methyl-2,5-dioxo-imidazolidin]-essigsäure (7.4)

4,2 g (14,67 mMol) an (7.3) werden in 50 ml 90%-iger Trifluoressigsäure gelöst. Nach 1 h Rühren bei Raumtemperatur wird die Trifluoressigsäure im Vakuum entfernt und der Rückstand gefriergetrocknet Man erhält 3,17g (94 %) an (7.4).

### 7e) (2S)-Benzyloxycarbonylamino-3-[((4S)-methoxycarbonyl-methyl-2,5-dioxo-imidazolidin-1-yl)-acetylamino]-propionsäure-tert.butylester (7.5)

Die Synthese von (7.5) erfolgt durch Reaktion von (7.4) mit (1.6) wie in Beispiel 4 bei der Herstellung von (4.2) aus (4.1) und (1.6) beschrieben. Aus 1g (4,3 mMol) an (7.4) erhält man nach Chromatographie des Rohproduktes über Kieselgel mit Dichlormethan/Methanol = 20/1 und anschließend 40/1 nach Einengen der Produktfraktionen 1,72 g (79 %) an (7.5).

### 7f) (2S)-Benzyloxycarbonyl-amino-3-[((4S)-carboxy-methyl-2,5-dioxo-imidazolidin-1-yl)-acetylamino]-propionsäure-tert.butylester (7.6)

Eine Lösung von 700 mg (1,38 mMol) an (7.5) in Wasser/Dioxan/Triethylamin = 1/1/1 wird 6 h bei 50 °C gerührt. Nach Stehen über Nacht bei Raumtemperatur wird das Reaktionsgemisch eingeengt und der Rückstand mit Dichlormethan/Methanol/Essigsäure/Wasser = 9/1/0,1/0,1 über Kieselgel chromatographiert. Nach Einengen der Produktfraktionen erhält man 364 mg (54 %) an (7.6).

### 7g) (2S)-Benzyloxycarbonylamino-3-[2-((4S)-(N'-(4,5-dihydro-1H-imidazol-2-yl)-hydrazinocarbonylmethyl]-2,5-dioxo-imidazolidin-1-yl)-acetylamino]-propionsäure-tert.-butylester (7.7)

Man gibt zu einer Lösung von 100 mg (0,2 mMol) an (7.6) und 33,1 mg (0,2 mMol) HOOBt in 5 ml DMF
bei 0 °C 44,7 mg DCCI. Nach 1 h bei 0 °C und 1 h bei Raumtemperatur gibt man 36,8 mg (0,2 mMol) 2-Hydrazino-2-imidazol-hydrobromid zu und läßt 3 Tage bei Raumtemperatur rühren. Das Lösungsmittel wird im Vakuum entfernt und der Rückstand mit Dichlormethan/Methanol/Essigsäure/Wasser = 8/2/0,2/0,2 über Kieselgel chromatographiert. Die Produktfraktionen werden eingeengt und gefriergetrocknet. Man erhält 60 mg (52 %) an (7.7).

### 7h) (2S)-Benzyloxycarbonylamino-3-[2-((4S)-(N'-(4,5-dihydro-1 H-imidazol-2-yl)-hydrazinocarbonylmethyl]-2,5-dioxo-imidazolidin-1-yl)-acetylamino]-propionsäure (7.8)

55 mg (0.096 mMol) an (7.7) in 1 ml 90%-iger Trifluoressigsäure werden 1 h bei Raumtemperatur gerührt. Nach Entfernen der Trifluoressigsäure im Vakuum und Reinigung des Rohproduktes mittels präperativer HPLC über RP18 und anschließender Gefriertrocknung der Produktfraktionen erhält man 10,6 mg (21%) an (7.8) als farblosen Feststoff.

ES(+)-MS: 519 (M+H)⁺

### Beispiel 8

### (2S)-Benzyloxycarbonylamino-3-[2-(2,5-dioxo-(4S)-(N'-(1,4,5,6-tetrahydro-pyrimidin-2-yl)-hydrazinocarbonylmethyl)-imidazolidin-1-yl)-acetylamino]-propionsäure (8.2)

Die Synthese erfolgte nach folgender Reaktionssequenz:

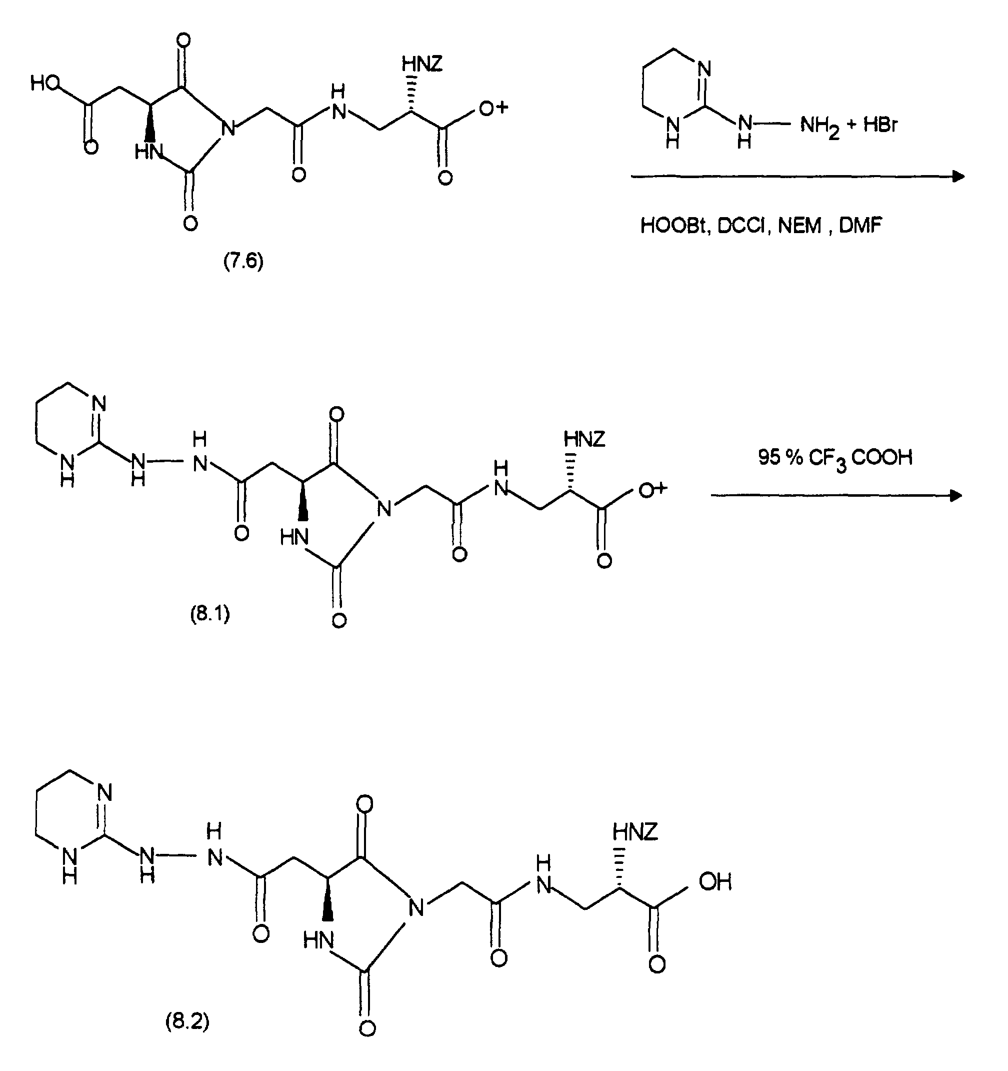

### 8a) (2S)-Benzyloxycarbonylamino-3-[2-(2,5-dioxo-(4S)-(N'-(1,4,5,6-tetrahydro-pyrimidin-2-yl)-hydrazinocarbonylmethyl)-imidazolidin-1-yl)-acetylamino]-propionsäure-tert.-butylester (8.1)

Man gibt zu einer Lösung von 130 mg (0,26 mMol) an (7.6) und 43 mg (0,26 mMol) HOOBt in 5 ml DMF bei 0 °C 58 mg DCCI, läßt 1 h bei 0 °C und 1 h bei Raumtemperatur rühren und gibt anschließend 0.034 ml N-Ethylmorpholin (NEM) und 52 mg (0,26 mMol) 2-Hydrazino-1,4,5,6-tetrahydropyrimidin-hydrobromid zu. Nach 28 h Rühren bei Raumtemperatur wird das Lösungsmittel im Vakuum entfernt und der Rückstand mit Dichlormethan/Methanol/Essigsäure/Wasser = 8,5/1,5/0,15/0,15 über Kieselgel chromatographiert. Die Produktfraktionen werden eingeengt und gefriergetrocknet. Man erhält 80 mg (52 %) an (8.1).

### 8b) (2S)-Benzyloxycarbonylamino-3-[2-(2,5-dioxo-(4S)-(N'-(1,4,5,6-tetrahydropyrimidin-2-yl)-hydrazinocarbonylmethyl)-imidazolidin-1-yl)-acetylamino]-propionsäure (8.2)

Eine Lösung von 80 mg (0,136 mMol) an (8.1) in 4 ml 90%-iger Trifluoressigsäure wird 1 h bei Raumtemperatur gerührt, im Vakuum eingeengt und der Rückstand mit Wasser/Butanol/Essigsäure = 43/4,3/3,5 über Sephadex LH 20 chromatographiert Die Produktfraktionen werden eingeengt und durch präparative HPLC über RP 18 gereinigt. Nach Einengen der Produktfraktionen und Gefriertrocknen erhält man 41,9 mg (59 %) an (8.2) als farblosen Feststoff.

ES(+)-MS: 533 (M+H)⁺

### Beispiel 9

### 2-Benzyloxycarbonylamino-3-[2-((4S)-(2-(N'-(4,5-dihydro-1H-imidazol-2-yl)-hydrazinocarbonyl)-ethyl)-2,5-dioxo-imidazolidin-1-yl)-acetylamino]-propionsäure (9.1)

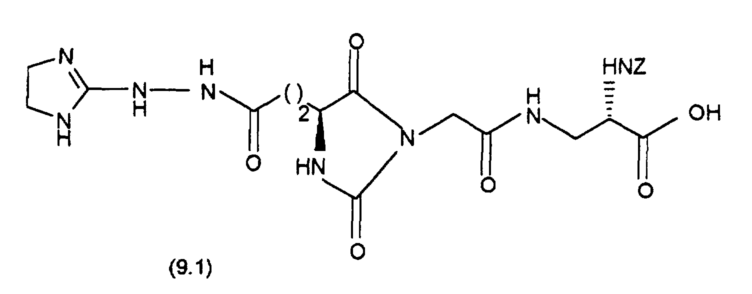

Die Synthese von (9.1) erfolgt ausgehend von L-Glutaminsäure wie in Beispiel 7 beschrieben.

ES(+)-MS: 533 (M+H)⁺

### Beispiel 10

### 3-[(2-((4S)-(8(1H-Benzimidazol-2-ylmethyl)-carbamoyl)-methyl)-2,5-dioxo-imidazolidin-1-yl)-acetylamino]-(2S)-benzyloxycarbonylamino-propionsäure (10.2)

Die Synthese erfolgte nach folgender Reaktionssequenz:

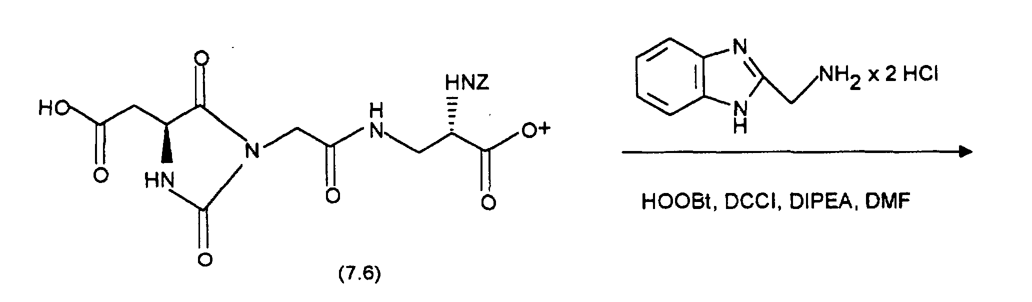

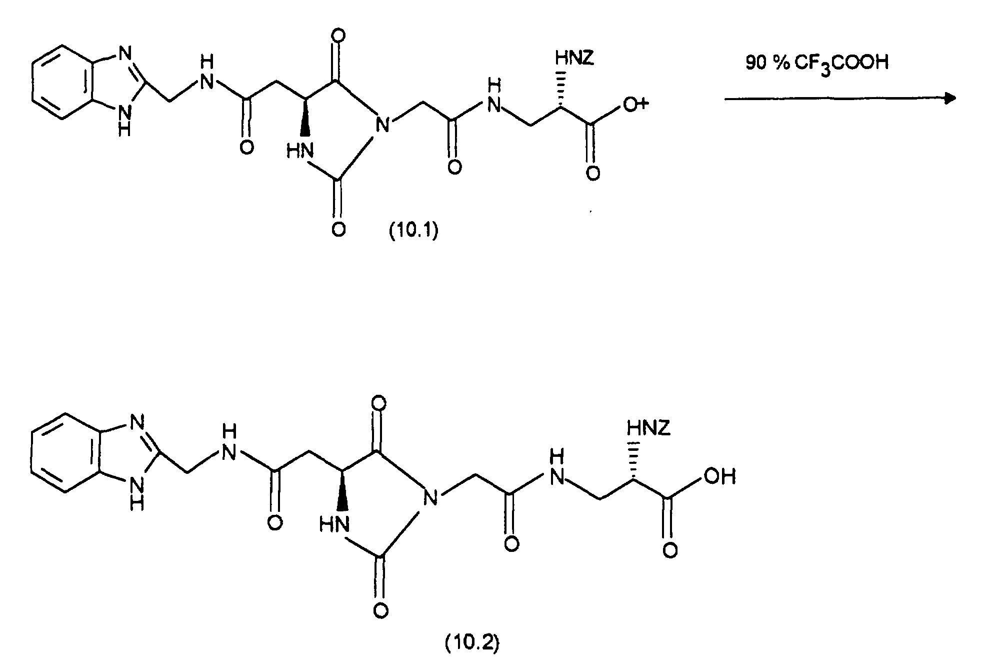

### 10a) 3-[(2-((4S)-(8(1H-Benzimidazol-2-ylmethyl)-carbamoyl)-methyl)-2,5-dioxo-imidazolidin-1-yl)-acetylamino]-(2S)-benzyloxycarbonylamino-propionsäure-tert.-butylester (10.1)

Man gibt zu einer Lösung von 430 mg (0,87 mMol) an (7.6) und 142,5 mg (0,87 mMol) HOOBt in 10 ml DMF bei 0 °C 192 mg DCCI. Nach 1h Rühren bei 0 °C und 1 h bei Raumtemperatur werden 231 mg (1,04 mMol) 2-(Aminomethyl)-benzimidazol und 0,5 ml Diisopropyl-ethylamin (DIPEA) zugegeben und das Gemisch 2 h bei Raumtemperatur weitergerührt. Nach Stehen über Nacht wird das Lösungsmittel im Vakuum entfernt und der Rückstand mit Dichlormethan/Methanol/Essigsäure/Wasser = 9,5/0,5/0,05/0,05 über Kieselgel chromatographiert.
Nach Einengen der Produktfraktionen erhält man 390 mg (72 %) an (10.1).

### 10b) 3-[(2-((4S)-(8(1H-Benzimidazol-2-ylmethyl)-carbamoyl)-methyl)-2,5-dioxo-imidazolidin-1-yl)-acetylamino]-(2S)-benzyloxycarbonylamino-propionsäure (10.2)

360 mg (0,579 mMol) an (10.1) werden in 10 ml 95%-iger Trifluoressigsäure gelöst. Nach 30 min bei Raumtemperatur wird die Lösung im Vakuum eingeengt, der Rückstand zwischen Essigester und Wasser verteilt, die Wasserphase mit Essigester extrahiert und gefriergetrocknet. Der Rückstand wird mit Wasser/Butanol/Essigsäure = 43/4,3/3,5 über Sephadex LH 20 chromatographiert Die Produktfraktionen werden eingeengt und gefriergetrocknet. Man erhält 135 mg (40 %) an (10.2) als farblosen Feststoff.

ES(+)-MS: 566 (M + H)⁺

### Beispiel 11

### (2S)-Benzyloxycarbonylamino-3-[2-((4S)-(3-(3H-imidazo[4,5-b]pyridin-2-ylamino)-propyl)-2,5-dioxo-imidazolidin-1-yl)-acetylamino]-propionsäure (11.7)

Die Synthese erfolgte nach folgender Reaktionssequenz:

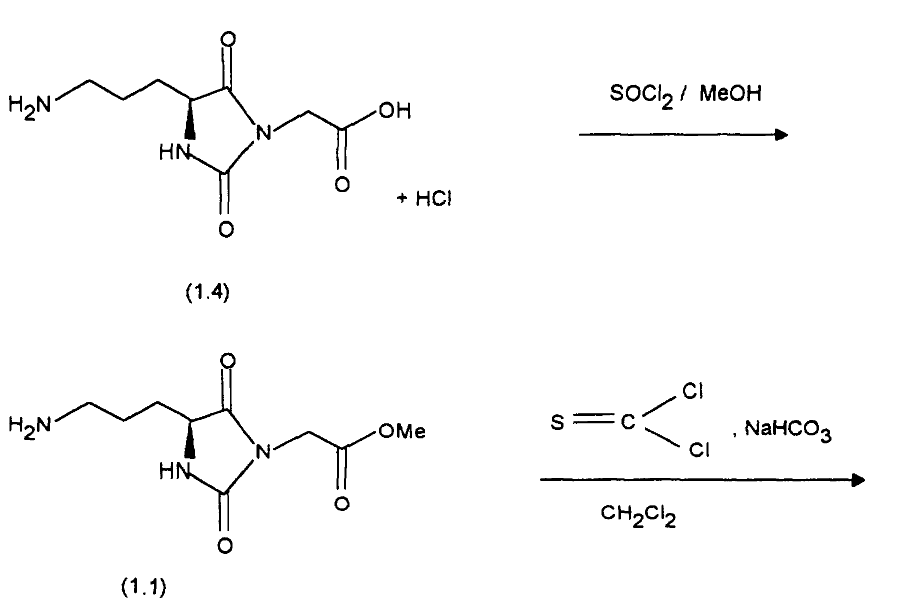

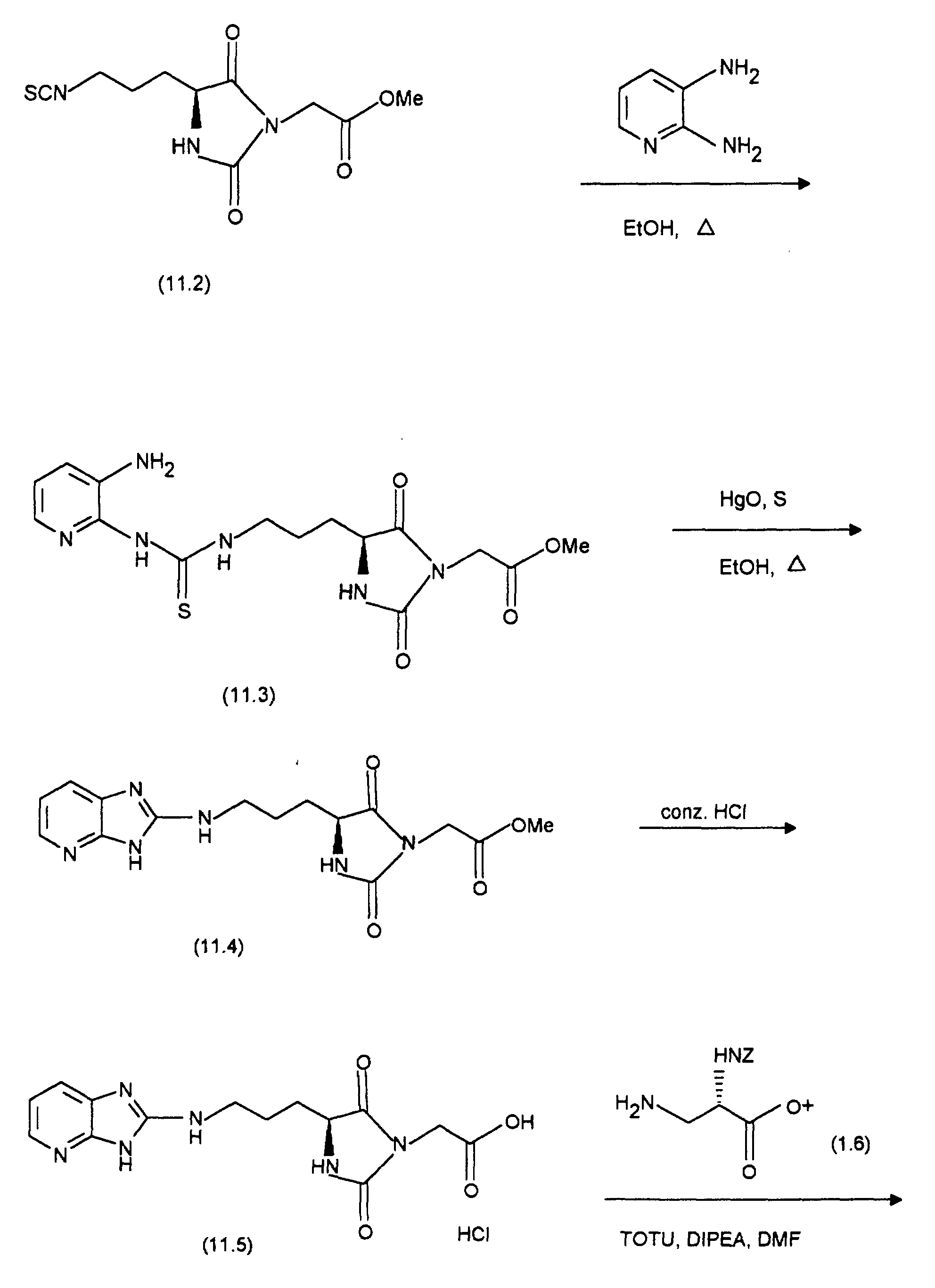

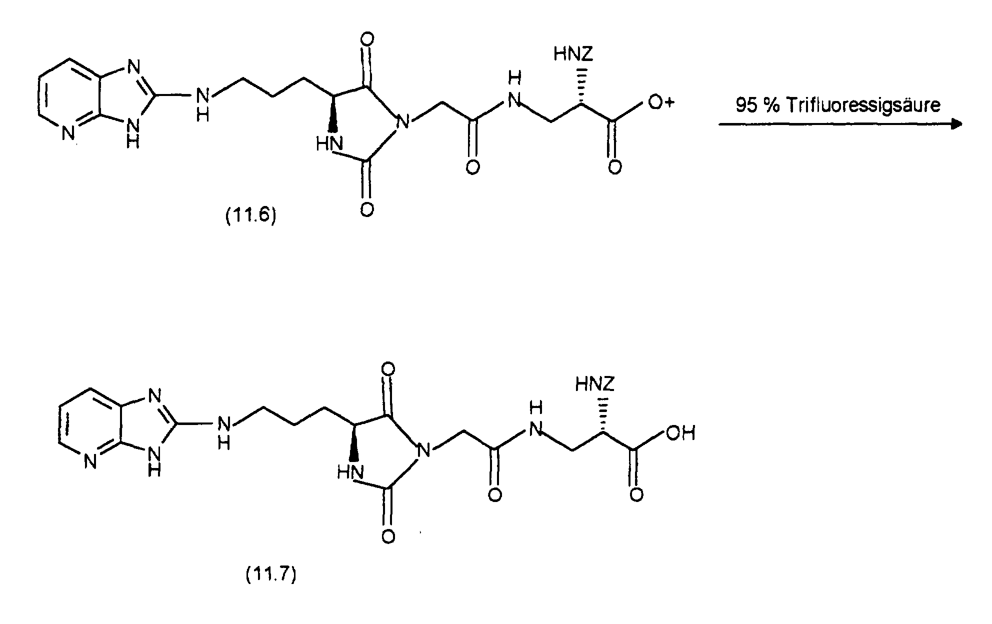

### 11a) [4-(3-Amino-propyl)-2,5-dioxo-imidazolidin-1-yl]-essigsäure-methylester-hydrochlorid (11.1)

Man gibt bei -15°C zu 100 ml Methanol 12,5 ml (107 mMol) Thionylchlorid, 21,5 g (85,4 mMol) an (1.4) und rührt das Reaktionsgemisch 24 h bei Raumtemperatur. Anschließend wird die Lösung in Diethylether gegossen, der Niederschlag abgesaugt und mit Diethylether gewaschen. Nach Trocknen im Hochvakuum erhält man 20,52 g (90 %) an (11.1) als farblosen Feststoff.

### 11b) [4-(3-Isothiocyanato-propyl)-2,5-dioxo-imidazolidin-1-yl]-essigsäure-methylester (11.2)

Man gibt zu einer Suspension von 2,65 g (10 mMol) an (11.1) in 30 ml Dichlormethan 100 ml einer gesättigten Natriumhydrogencarbonat-Lösung. Nach 10 min. Rühren unter Eiskühlung gibt man 1,53 ml (20 mMol) Thiophosgen zur Methylenchlorid-Phase. Man rührt 10 min. unter Eiskühlung, trennt die Phasen und extrahiert die Wasser-Phase 2 mal mit Dichlormethan. Die vereinigten organischen Phasen werden über Natriumsulfat getrocknet. Nach Filtration und Entfernen des Lösungsmittels im Vakuum erhält man 2,04 g (75 %) an (11.2).

### 11c) [(4S)-(3-(3-(3-Amino-pyridin-2-yl)-thioharnstoff)-propyl)-2,5-dioxo-imidazolidin-1-yl]-essigsäure-methyl ester (11.3)

Eine Lösung von 1,66 g (6,11 mMol) an (11.2) und 667 mg (6,11 mMol) 2,3-Diaminopyridin in 18 ml absolutem Ethanol werden 16 h bei Raumtemperatur und anschließend 2 h unter Rückfluß gerührt. Das Lösungsmittel wird im Vakuum entfernt und der Rückstand mit Dichlormethan/Methanol/Essigsäure/Wasser = 9/1/0,1/0,1 über Kieselgel chromatographiert. Nach Einengen der Produktfraktionen und Gefriertrocknen erhält man 1,67g (72 %) an (11.3).

### 11d) [(4S)-(3-(3H-lmidazo[4,5]pyridin-2-ylamino)-propyl)-2,5-dioxo-imidazolidin-1-yl]-essigsäure-methylester (11.4)

Man gibt zu einer Lösung von 1,43 g (3,75 mMol) an (11.3) in 80 ml Ethanol 1,62 g (7,51 mMol) Quecksilberoxid und 24 mg Schwefel und erhitzt das Gemisch 1h unter Rückfluß. Nach Filtration und Einengen des Filtrats im Vakuum erhält man 1,15 g (89 %) an (11.4).

### 11e) [(4S)-(3-(3H-lmidazo[4,5-b]pyridin-2-ylamino)-propyl)-2,5-dioxo-imidazolidin-1-yl]-essigsäure-hydrochlorid (11.5)

286 mg (0,83 mMol) an (11.4) in 10 ml conz. HCl werden 5 h bei 50 °C gerührt. Nach Filtration wird das Filtrat mit Wasser verdünnt und gefriergetrocknet. Man erhält 277 mg (91 %) an (11.5) als farblosen Feststoff.

### 11f) (2S)-Benzyloxycarbonylamino-3-[2-((4S)-(3-(3H-imidazo[4,5-b]pyridin-2-ylamino)-propyl)-2,5-dioxo-imidazolidin-1-yl)-acetylamino]-propionsäure-tert.-butylester (11.6)

Man gibt zu einer Lösung von 267 mg (0,72 mMol) an (11.5) und 213 mg (0,72 mMol) an (1.6) in 10 ml DMF 237 mg (0,72 mMol) O-[Cyan(ethoxycarbonyl)methylenamino]-1,1,3,3-tetramethyluroniumtetrafluoroborat (TOTU) und 0,245 ml Diisopropyl-ethylamin (DIPEA) und rührt 1 h bei Raumtemperatur. Das Lösungsmittel wird im Vakuum entfernt, der Rückstand in Essigester gelöst und die Essigester-Phase 2 mal mit gesättigter NaHCO₃-Lösung und 2 mal mit Wasser extrahiert. Die organische Phase wird über Natriumsulfat getrocknet. Nach Filtration und Eineengen des Filtrats im Vakuum erhält man 425 mg (97 %) an (11.6).

### 11g) (2S)-Benzyloxycarbonylamino-3-[2-((4S)-(3-(3H-imidazo[4,5-b]pyridin-2-ylamino)-propyl)-2,5-dioxo-imidazolidin-1-yl)-acetylamino]-propionsäure (11.7)

420 mg (0,69 mMol) an (11.6) werden in 10 ml 95%-iger Trifluoressigsäure gelöst. Nach 15 min bei Raumtemperatur wird die Lösung im Vakuum eingeengt und der Rückstand mit Wasser/Butanol/Essigsäure = 43/4,3/3,5 über Sephadex LH 20 chromatographiert Die Produktfraktionen werden eingeengt und gefriergetrocknet. Man erhält 234 mg (62 %) an (11.7) als farblosen Feststoff.

ES(+)-MS: 553 (M + H)⁺

### Beispiel 12

### (2S)-Benzyloxycarbonylamino-3-[2-(4S)-(3-(3H-imidazo[4,5-c]pyridin-2-ylamino)-propyl)-2,5-dioxo-imidazolidin-1-yl)-acetylamino]-propionsäure (12.3)

Die Synthese erfolgte nach folgender Reaktionssequenz:

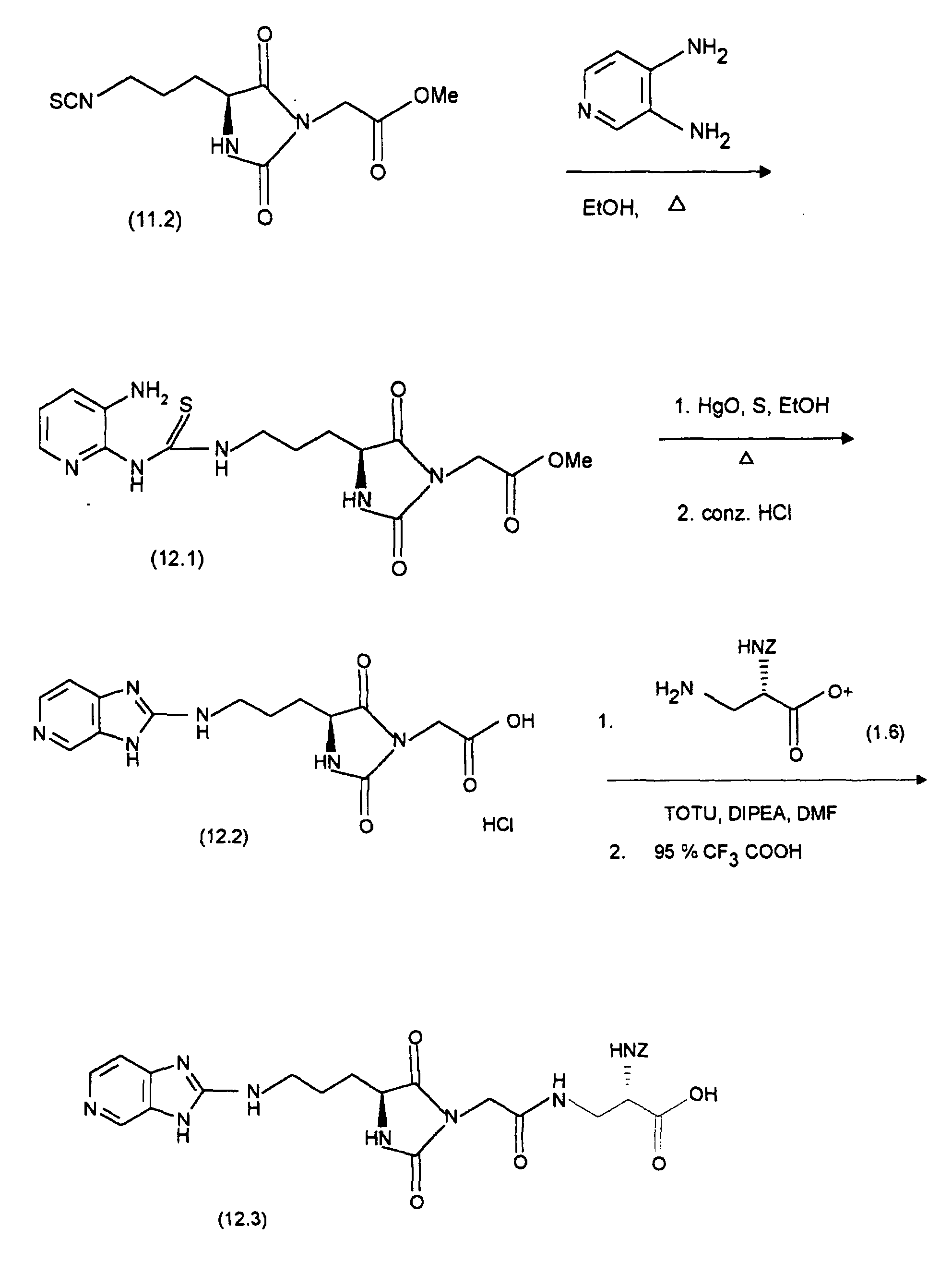

Ausgangspunkt der Synthese von (12.3) ist (11.2), das analog der Herstellung von (11.3) mit 3,4-Diaminopyridin zu (12.1) umgesetzt wird. Letzteres wird dann analog der Herstellung von (11.4) und (11.5) mit Quecksilberoxid zyclisiert und mit konz. HCI zu (12.2) umgesetzt. (12.2) wird mit (1.6), wie bei der Herstellung von (11.6) beschrieben, umgesetzt und das so erhaltene Kopplungsprodukt unter Spaltung des tert.-Butylesters, wie bei der Synthese von (11.7) aus (11.6) beschrieben, in (12.3) überführt.

ES(+)-MS: 553 (M + H)⁺

### Beispiel 13

### (2S)-(Adamantan-1-yl-methoxycarbonylamino)-3-[((4S)-(3-(benzimidazolyl-2-amino)-propyl)-5-oxo-2-thioxo-imidazolidin-1-yl)-acetylamino]-propionsäure (13.7)

Die Synthese erfolgte nach folgender Reaktionssequenz:

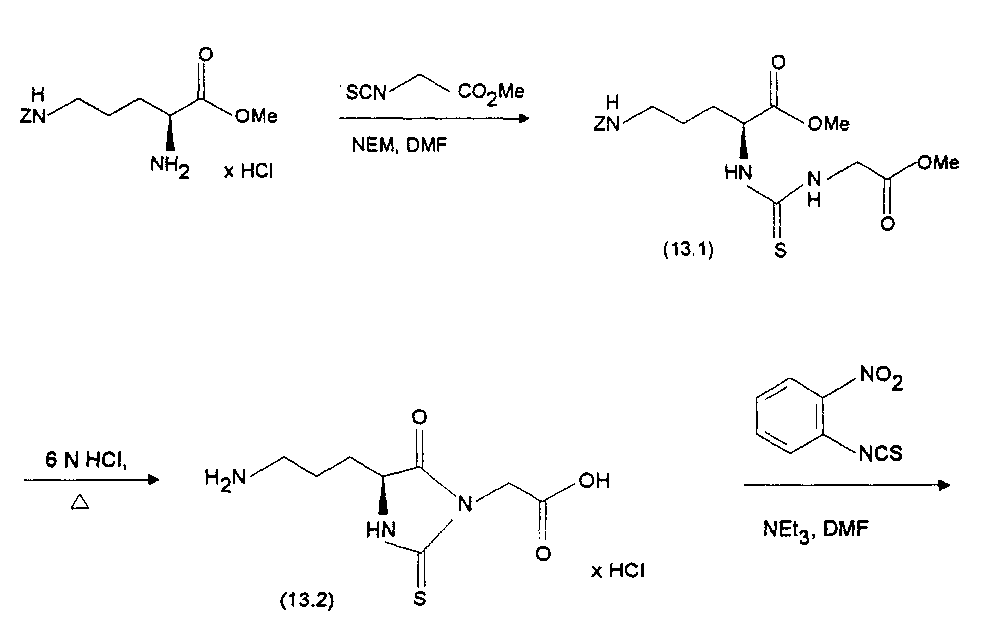

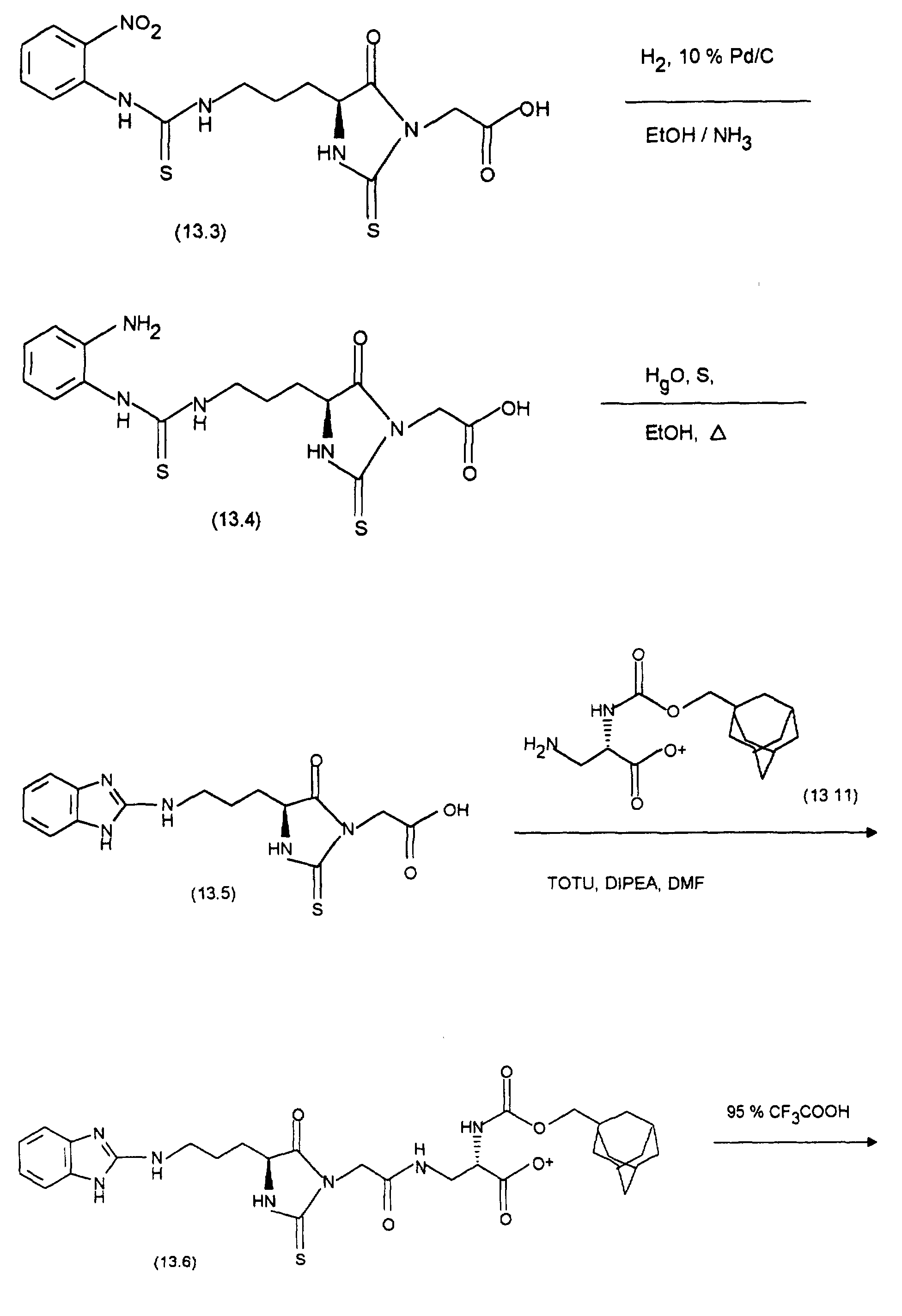

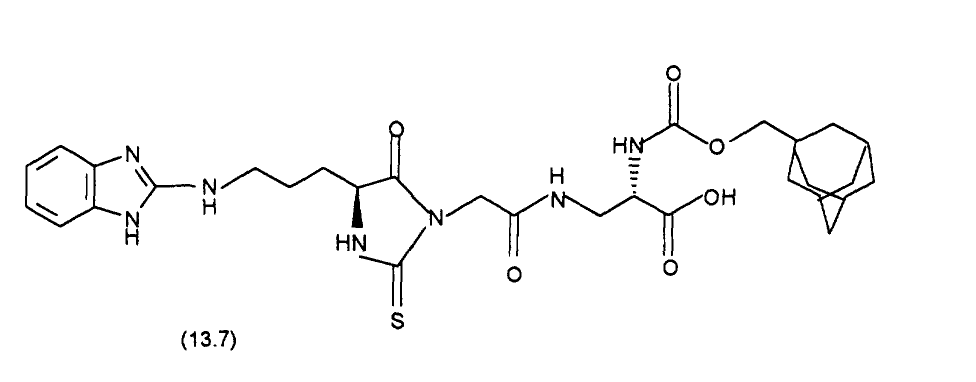

### 13a) 5-Benzyloxycarbonyl-amino-(2S)-methoxycarbonylmethylaminothiocarbonylamino -5-pentansäure-methylester (13.1)

Man gibt zu einer Lösung von 6,32 g (20 mMol) H-Orn(Z)-OMe x HCI (Bachem) in 40 ml DMF bei 0 °C 2,62 g (20 mMol) Isothiocyanato-essigsäuremethylester und 2,3 g (20 mMol) N-Ethylmorpholin (NEM). Nach 20 h Rühren bei Raumtemperatur wird das Lösungsmittel im Vakuum entfernt, der Rückstand in Dichlormethan aufgenommen und die Dichlormethanlösung 2 mal mit Wasser extrahiert.Nach Trocknen der organischen Phase über Natriumsulfat, Filtration und Entfernen des Lösungsmittels im Vakuum erhält man (13.1), das direkt zur Synthese von (13.2) eingesetzt wird.

### 13b) [((4S)-(3-Amino-propyl))-5-oxo-2-thioxo-imidazolidin-1-yl)-essigsäure-hydrochlorid (13.2)

Eine Suspension von (13.1) in 150 ml 6N HCI wird 5 h bei 50 °C gerührt. Die Lösung wird eingeengt, der Rückstand 2 mal mit Diethylether ausgerührt, und danach wird der Rückstand im Hochvakuum und anschließend über KOH getrocknet. Man erhält (13.2), das direkt zur Synthese von (13.3) eingesetzt wird.

### 13c) 2-[(4S)-(3-(3-(2-Nitrophenyl)-thioharnstoff)-propyl)-5-oxo-2-thioxo-imidazolidin-1-yl]-essigsäure (13.3)

Man tropft zu einer Lösung von (13.2) und 3,6 g (20 mMol) 2-Nitrophenylisothiocyanat in 50 ml DMF unter Eiskühlung 2,77 ml (20 mMol) Triethylamin. Man läßt die Lösung über Nacht bei Raumtemperatur rühren, gibt nochmals 360 mg (2 mMol) 2-Nitrophenyl-isothiocyanat und 0,8 ml Triethylamin zu und läßt witere 5 h bei Raumtemperatur rühren. Das Lösungsmittel wird im Vakuum entfernt, der Rückstand in Essigester gelöst und die Essigester-Phase 2 mal mit einer wäßrigen KHSO47K₂SO₄-Lösung gewaschen und über Natriumsulfat getrocknet. Nach Filtration wird der Essigester im Vakuum entfernt und der Rückstand mit Dichlormethan/Methanol/Essigsäure/Wasser = 9/1/0,1/0,1 über Kieselgel chromatographiert.
Nach Einengen der Produktfraktionen erhält man 4,06g (49 %, bezogen auf (13.1)) an (13.3).

### 13d) 2-[(4S)-(3-(3-(2-Aminophenyl)-thioharnstoff)-propyl)-5-oxo-2-thioxo-imidazolidin-1-yl]-essigsäure (13.4)

611 mg (1,48 mMol) an (13.4) in 50 ml gesättigter ethanolischer Ammoniaklösung werden über 600 mg 10% Pd/C 3 h bei Raumtemperatur hydriert. Nach Filtration und Einengen des Filtrats im Vakuum erhält man 488 mg (87 %) an (13.4).

### 13e) [(4S)-(3-(benzimidazolyl-2-amino)-propyl)-5-oxo-2-thioxo-imidazolidin-1-yl]-essigsäure (13.5)

Man gibt zu einer Lösung von 485 mg (1,27 mMol) an (13.4) in 50 ml Ethanol 550 mg (2,54 mMol) Quecksilberoxid und 8,1 mg Schwefel und erhitzt das Gemisch 3 h unter Rückfluß. Nach Filtration und Entfernen des Lösungsmittels im Vakuum wird der Rückstand in 10%-iger Essigsäure aufgenommen und gefriergetrocknet. Man erhält 307 mg (79 %) an (13.5) als farblosen Feststoff.

### 13f) (2S)-(Adamantan-1-yl-methoxycarbonylamino)-3-[((4S)-(3-(benzimidazolyl-2-amino)-propyl)-5-oxo-2-thioxo-imidazolidin-1-yl)-acetylamino]-propionsäure-tert.butylester (13.6)

Man gibt zu einer Lösung von 290 mg (0,834 mMol) an (13.5) und 297 mg (0,834 mMol) an (13.11) (Synthese siehe 13h-k) in absolutem DMF 273 mg (0,834 mMol) TOTU und 0,28 ml Diisopropyl-ethylamin (DIPEA) und läßt das Gemisch 2,5 h bei Raumtemperatur rühren. Das Lösungsmittel wird im Vakuum entfernt, der Rückstand in Essigester aufgenommen und die Essigester-Phase 2 mal mit gesättigter Natriumhydrogencarbonat-Lösung und Wasser extrahiert. Nach Trocknen über Natriumsulfat wird der Essigester im Vakuum entfernt und der Rückstand mit Dichlormethan/Methanol/Essigsäure/Wasser = 9,5/0,5/0,05/0,05 über Kieselgel chromatographiert.
Nach Einengen der Produktfraktionen erhält man 46,3mg (8 %) an (13.6).

### 13g) (2S)-(Adamantan-1-yl-methoxycarbonylamino)-3-[((4S)-(3-(benzimidazolyl-2-amino)-propyl)-5-oxo-2-thioxo-imidazolidin-1-yl)-acetylamino]-propionsäure (13.7)

38,1 mg (0,056 mMol) an (13.6) werden in 2 ml 95%-iger Trifluoressigsäure gelöst. Nach 10 min bei Raumtemperatur wird die Lösung im Vakuum eingeengt und der Rückstand über präperative HPLC (RP18) gereinigt. Nach Gefriertrocknen der Produktfraktionen erhält man 11 mg (32 %) an (13.7).

FAB-MS: 626 (M + H)⁺

### Synthese von 3-Amino-(2S)-(Adamantan-1-yl-methoxycarbonylamino)-propionsäure-tert.-butylester (13.11)

Die Synthese erfolgte nach folgender Reaktionssequenz:

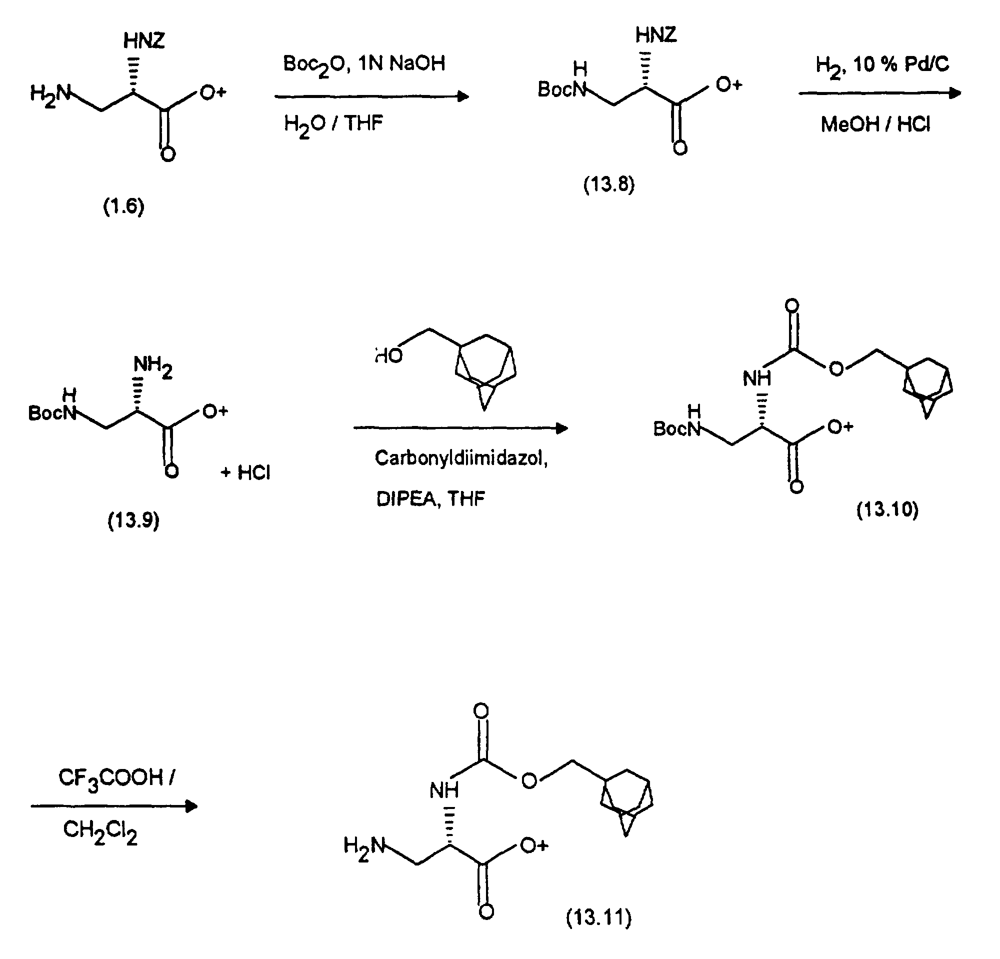

### 13h) (2S)-Benzyloxycarbonylamino-3-tert.-butoxycarbonylamino-propionsäure-tert.-butylester (13.8)

Man gibt zu einer Lösung von 10 g (34 mMol) an (1.6) in 600 ml Tetrahydrofuran/Wasser = 2/1 bei 0°C 8,9 g (40,8 mMol) Di-tert.butyl-dicarbonat und anschließend portionsweise 1N NaOH, so daß der pH der Lösung zwischen 9-10 liegt (Verbrauch an 1N NaOH: 32 ml).Nach 3 h Rühren bei Raumtemperatur gibt man 1 l Wasser zu und extrahiert 3 mal mit Diethylether. Nach Trocknen über Natriumsulfat, Filtration und Entfernen des Lösungsmittels im Vakuum wird der Rückstand mit Dichlormethan/Methanol = 20/1 über Kieselgel chromatographiert. Man erhält 13,19 g (98 %) an (13.8).

### 13i) (2S)-Amino-3-tert.-butoxycarbonylamino-propionsäure-tert.-butylester-hydrochlorid (13.9)

13,1 g (33,2 mMol) an (13.8) werden in Methanol/HCl über 10 % Pd/C hydriert. Nach 1,5 h wird filtriert, das Filtrat im Vakuum eingeengt und man erhält 9,77 g (99 %) an (13.9) als farblosen Feststoff.

### 13j) (2S)-(Adamantan-1-yl-methoxycarbonylamino)-3-tert.-butoxycarbonylamino-propionsäure-tert.-butylester (13.10)

Eine Lösung von 10,9 g (65,4 mMol) (1-Hydroxymethyl)-adamantan und 10,6 g (65,4 mMol) Carbonyldiimidazol in 60 ml THF wird 1,5 h bei 50 °C gerührt. Man gibt 9,7 g (32,7 mMol) an (13.9) in 25 ml THF und 5,6 ml (32,7 mMol) Diisopropyl-ethylamin (DIPEA) zu, rührt 4 h bei 60 °C und läßt über Nacht bei Raumtemperatur stehen. Das Lösungsmittel wird im Vakuum entfernt und der Rückstand mit Heptan/Essigester = 7/3 über Kieselgel chromatographiert. Man erhält 8,7 g (59 %) an (13.10) als farbloses Öl.

### 13k) (2S)-(Adamantan-1-yl-methoxycarbonylamino)-3-amino-propionsäure-tert.-butylester (13.11)

Eine Lösung von 8,7 g (19,22 mMol) an (13.10) in 180 ml Trifluoressigsäure/Dichlormethan = 1/1 wird nach 1 min. in 1,5 ml eiskalte NaHCO₃-Lösung gegeben. Die Lösung wird 3 mal mit Dichlormethan extrahiert und die Dichlormethan-Phase anschließend über Natriumsulfat getrocknet. Nach Filtration und Entfernen des Lösungsmittels im Vakuum erhält man 6,35 g (94 %) an (13.11) als farblosen Feststoff.

### Beispiel 14

### 3-[2-((4R,S)-(3-(1H-Benzimidazol-2-ylamino)-propyl)-4-methyl-2,5-dioxo-imidazolidin-1-yl)-acetylamino]-(2S)-benzyloxycarbonylamino-propionsäure (14.8)

Die Synthese erfolgte nach folgender Reaktionssequenz:

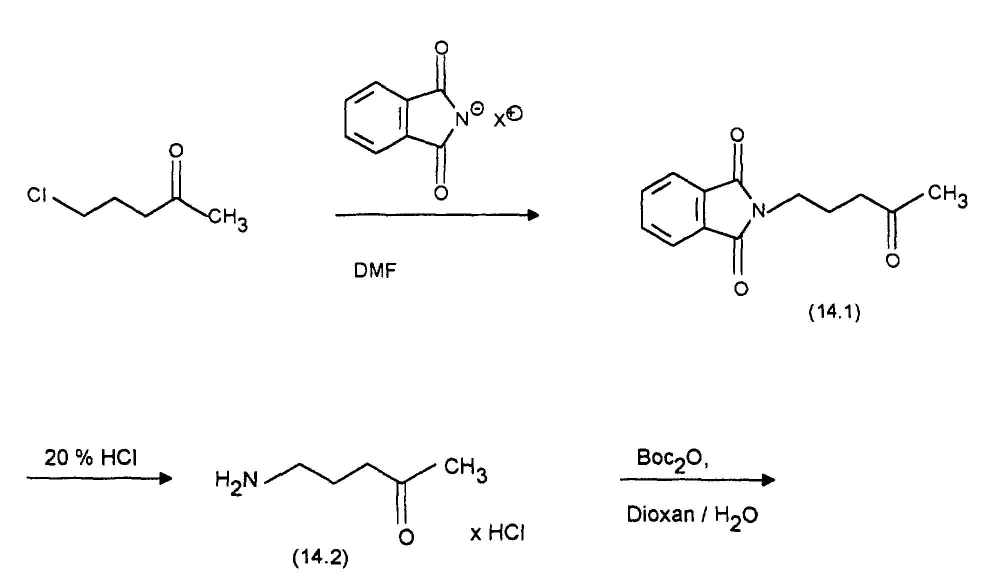

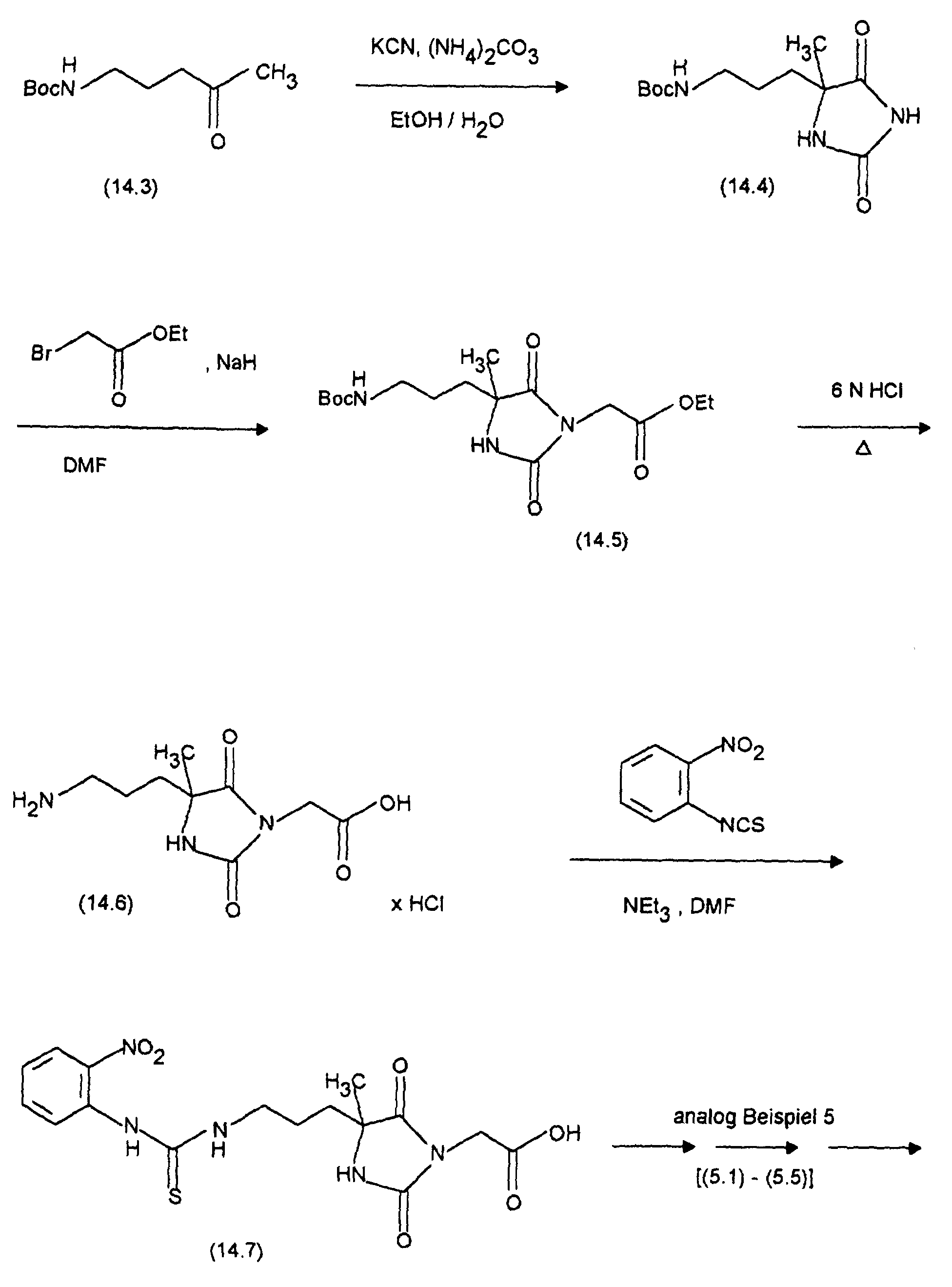

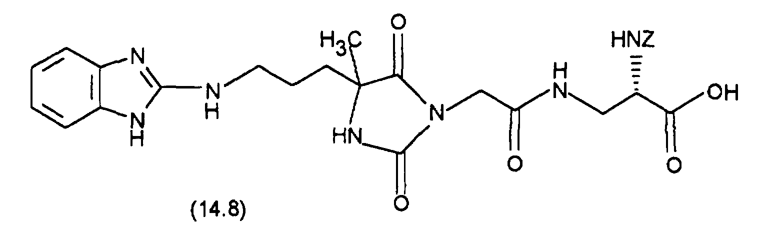

### 14a) 2-(4-Oxo-pentyl)-isoindol-1,3-dion (14.1)

Man gibt zu einer Lösung von 14,3 ml (124,4 mMol) 5-Chlor-2-pentanon in 100 ml DMF 24,59 g (132,8 mMol) Kalium-phthalimid, läßt 3 h bei Raumtemperatur und 30 h bei 60 °C rühren. Nach Filtration wird das Filtrat zwischen Wasser und Dichlormethan verteilt. Die Phasen werden getrennt und die organische Phase nacheinander mit Wasser, 2 mal mit 0,2 N NaOH-Lösung und Wasser gewaschen und über Natriumsulfat getrocknet. Nach Filtiation wird das Lösungsmittel im Vakuum entfernt und der Rückstand mit Heptan/Essigester = 6/4 über Kieselgel chromatographiert. Nach Einengen der Produktfraktionen erhält man 9,8 g (34 %) an (14.1).

### 14b) 5-Amino-2-oxo-pentan-hydrochlorid (14.2)

13 g (56,2 mMol) an (14.1) werden in 335 ml 20 %-iger Salzsäure gelöst und 6 h am Rückfluß erhitzt. Nach Stehen über Nacht bei Raumtemperatur wird filtriert und das Filtrat im Vakuum eingeengt. Man erhält ein blaßgelbes Öl an rohem (14.2), das direkt zur Synthese von (14.3) eingesetzt wird.

### 14c) 5-tert.-Butoxycarbonyl-amino-2-oxo-pentan (14.3)

(14.2) (aus 14b)) wird in 110 ml Dioxan und 55 ml Wasser gelöst und die Lösung durch Zugabe von 65 ml 1 N NaOH auf pH 8,5 gestellt. Bei 0 °C werden 13,25 g (60,8 mMol) Di-tert.-butyl-dicarbonat zugegeben und der pH-Wert der Lösung durch wiederhohlte Zugabe von 1N NaOH auf 8,5 eingestellt. Nach 4,5 h Rühren bei Raumtemperatur wird Dioxan im Vakuum entfernt, die verbleibende Lösung durch Zugabe von KHSO₄-Lösung auf pH 2-3 gestellt und 3 mal mit Essigester extrahiert. Die vereinigten organischen Phasen werden mit gesättigter Natriumhydrogencarbonat-Lösung gewaschen und über Natriumsulfat getrocknet. Nach Filtration und Entfernen des Lösungsmittels im Vakuum erhält man 11,1 g (99 % ausgehend von (14.1)) an (14.3).

### 14d) (4R,S)-(3-tert.-Butoxycarbonyl-amino)-propyl-4-methyl-2,5-dioxo-imidazolidin (14.4)

Man gibt zu einer Lösung von 10,06 g (50 mMol) an (14.3) in 130 ml Ethanol/Wasser = 1/1 41,96 g (439,2 mMol) Ammoniumcarbonat und 4,2 g (65,1 mMol) Kaliumcyanid und erhitzt das Gemisch auf 55-65 °C. Nach 5,5 h bei dieser Temperatur wird das Reaktionsgemisch mit 100 ml 6N HCI auf pH 6,3 gestellt und 2 h nachgerührt. Man läßt das Gemisch über Nacht bei Raumtemperatur stehen, entfernt das Lösungsmittel im Vakuum und verteilt den Rückstand zwischen Wasser und Essigester.Man trennt die Phasen und extrahiert die Wasserphase 2 mal mit Essigester. Die vereinigten organischen Phasen werden über Natriumsulfat getrocknet, das Trockenmittel abfiltriert und das Lösungsmittel im Vakuum entfernt. Man erhält 12,42 g (92 %) an (14.4) als farblose Kristalle.

### 14e) [(4R,S)-(3-tert.-Butoxycarbonylamino-propyl)-4-methyl-2,5-dioxo-imidazolidin-1-yll-essigsäure-ethylester (14.5)

Man gibt in einer Argonatmosphäre zu einer Lösung von 4 g (14,7 mMol) an (14.3) in 100 ml DMF unter Eiskühlung 388 mg (16,1 mMol) Natriumhydrid und rührt 45 min bei Raumtemperatur. Man gibt 1,63 ml (14,7 mMol) Bromessigsäure-ethylester zu, läßt 3 h bei Raumtemperatur rühren und entfernt das Lösungsmittel im Vakuum. Der Rückstand wird in Essigester gelöst und die Essigester-Phase 2 mal mit Wasser gewaschen. Nach Trocknen über Natriumsulfat und Entfernen des Lösungsmittels im Vakuum erhält man 4,98 g (95 %) an (14.5) als blaßgelbes Öl.

### 14f) [(4R,S)-(3-Amino-propyl)-4-methyl-2,5-dioxo-imidazolidin-1-yl]-essigsäure-hydrochlorid (14.6)

4,98 g (13,9 mMol) an (14.5) werden in 50 ml 6N HCI suspendiert und 1 h unter Rückfluß erhitzt. Das Reaktionsgemisch wird im Vakuum eingeengt, der Rückstand in Wasser gelöst und gefriergetrocknet. Man erhält (14.6) als Rohprodukt, das direkt zur Synthese von (14.7) eingesetzt wird.

Die Synthese von (14.7) ausgehend von (14.6) und (14.8) ausgehend von (14.7) erfolgt analog der Synthese von (5.5) ausgehend von (1.4) (siehe Beispiel 5).

(14.8): ES(+)-MS: 566 (M + H)⁺

### Beispiel 15

### (2S)-Benzyloxycarbonylamino-3-[((4S)-(3-(6-methoxy-1H-benzimidazol-2-ylamino)-propyl)-2,5-dioxo-imidazolidin-1-yl)- acetylaminol-propionsäure (15.3)

Die Synthese erfolgte nach folgender Reaktionssequenz:

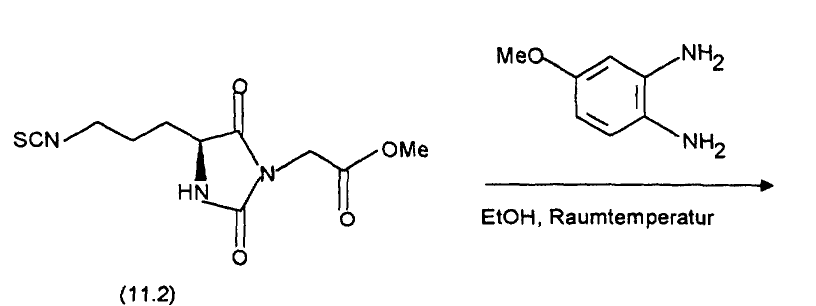

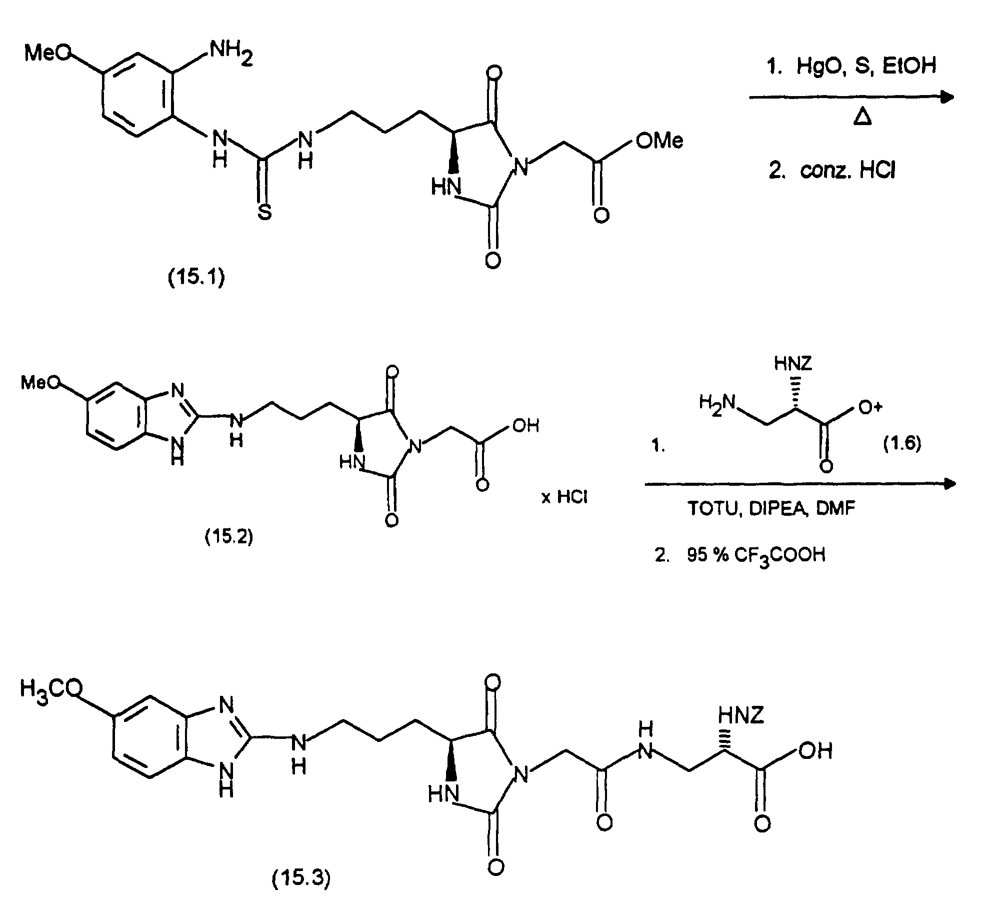

Ausgangspunkt der Synthese von (15.3) ist (11.2), das analog der Herstellung von (11.3) mit 4-Methoxy-ortho-phenylendiamin bei Raumtemperatur zu (15.1) umgesetzt wird. Letzteres wird dann analog der Herstellung von (11.4) und (11.5) mit Quecksilberoxid zyclisiert und mit konz. HCI zu (15.2) umgesetzt. (15.2) wird dann mit (1.6), wie bei der Herstellung von (11.6) beschrieben, umgesetzt und das so erhaltene Kopplungsprodukt unter Spaltung des tert.-Butylesters, wie bei der Synthese von (11.7) aus (11.6) beschrieben, in (15.3) überführt.

ES(+)-MS: 582 (M + H)⁺

### Beispiel 16

### (2S)-Benzyloxycarbonylamino-3-[((4S)-(3-(5,6-dimethoxy-1H-benzimidazol-2-ylamino)-propyl)-2,5-dioxo-imidazolidin-1-yl)- acetylamino]-propionsäure (16.1)

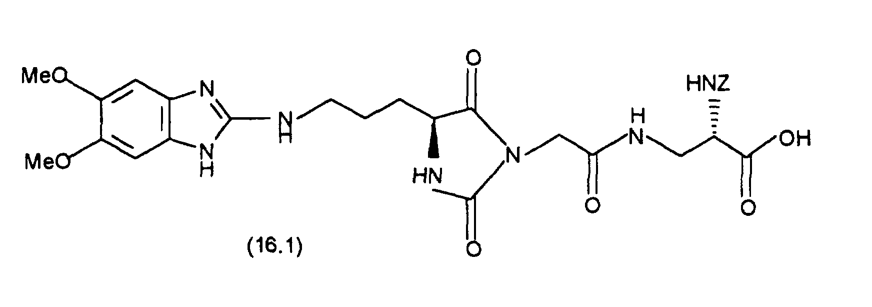

Die Synthese von (16.1) erfolgt wie bei Der Herstellung von (15.3) beschrieben, indem man anstelle von 1-Methoxy-3,4-diaminobenzol 1,2-Dimethoxy-4,5-diaminobenzol einsetzt, das aus 1,2-Dimethoxy-4,5-dinitrobenzol durch Hydrierung über 10 % Pd/C in Methanol hergestellt wird.

ES(+)-MS: 612 (M + H)⁺

### Beispiel 17

### (2S)-Benzyloxycarbonylamino-3-[((4S)-(3-(5,6-methylendioxy-benzimidazol-2-ylamino)-propyl)-2,5-dioxo-imidazolidin-1-yl)- acetylamino]-propionsäure (17.1)

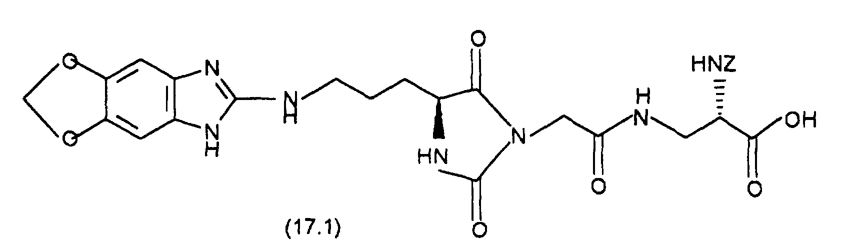

Die Synthese von (17.1) erfolgt wie bei Der Herstellung von (15.3) beschrieben, indem man anstelle von 1-Methoxy-3,4-diaminobenzol 1,2-Methylendioxy-4,5-diaminobenzol einsetzt, das aus 1,2-Methylendioxy-4,5-dinitrobenzol durch Hydrierung über 10 % Pd/C in Methanol hergestellt wird. Letzteres wird aus 1,2-Methylendioxy-4-nitrobenzol durch Nitrierung, wie in D.S.Wulfman et al., Synthesis 1978, 924 beschrieben, hergestellt.

ES(+)-MS: 624 (M + H)⁺

### Beispiel 18

### 3-[2-((4S)-(2-(1H-Benzimidazol-2-yl)-ethyl)-2,5-dioxo-imidazolidin-1-yl)-acetylamino]-(2S)-benzyloxycarbonylamino-propionsäure (18.5)

Die Synthese erfolgte nach folgende Reaktionssequenz:

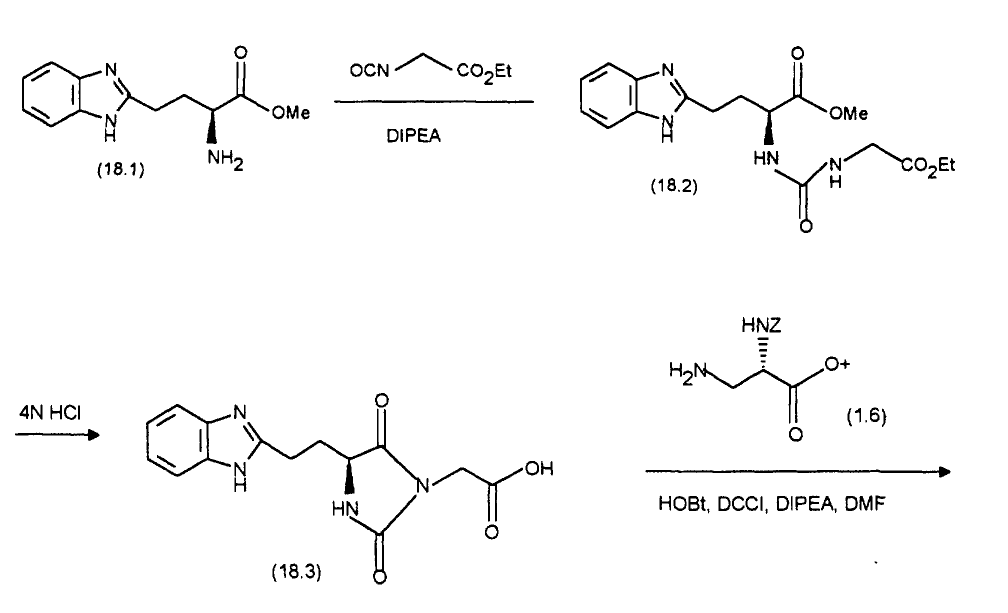

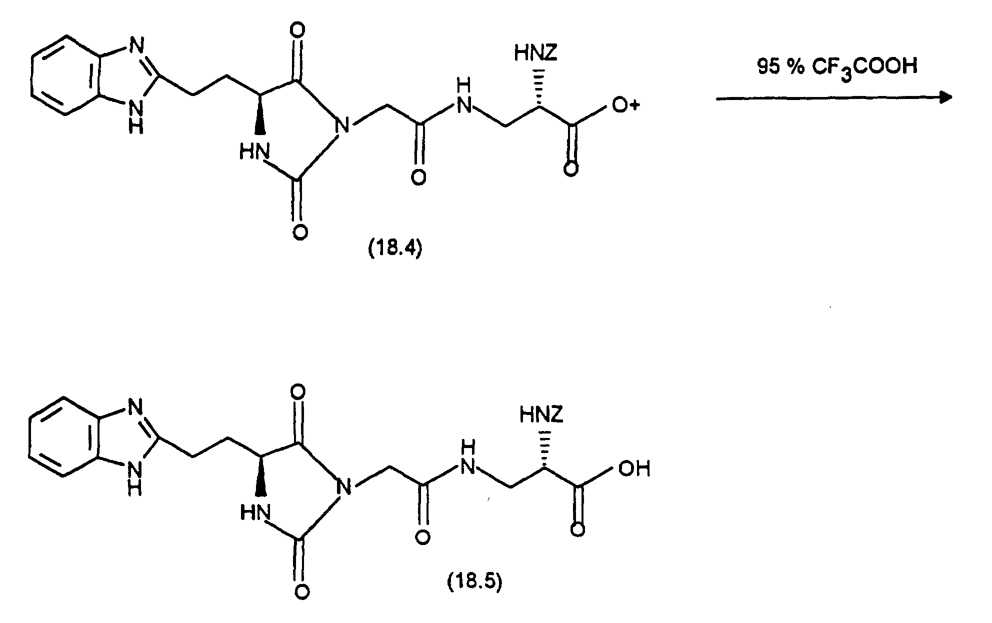

### 18 a) (2S)-Amino-4-(2-benzimidazolyl)-butansäure-methylester (18.1)

(18.1) wird wie bei Hans Lettre et al, Berichte 1951,84, 719 beschrieben, hergestellt.

### 18 b) (2S)-Ethyloxycarbonylmethyl-aminocarbonylamino-4-(2-(1H-benzimidazolyl)-butansäure- methylester (18.2)

Man gibt zu einer Lösung von 4,6 g (20 mMol) an in absolutem DMF, (18.1) bei 0 °C 3,4 ml (20 mMol) Diisopropyl-ethylamin (DIPEA) und 2,58 g (20 mMol) Isocyanatoessigsäureethylester.Nach 16 h Rühren bei Raumtemperatur wird das Lösungsmittel im Vakuum entfernt, der Rückstand in Essigester aufgenommen und die Essigesterphase 2 mal mit 10 %-iger Zitronensäure-Lösung gewaschen. Die wäßrige Phase wird mit 2N KOH-Lösung auf pH 10 gestellt und mehrmals mit Essigester extrahiert. Die vereinigten organischen Phasen werden über Natriumsulfat getrocknet, das Trockenmittel wird abfiltriert und das Filtrat im Vakuum eingeengt. Man erhält 4,4 g (61 %) an (18.2).

### 18c) [(4S)-(2-(1H-Benzimidazol-2-yl)-ethyl)-2,5-dioxo-imidazolidin-1-yl]-essigsäure (18.3)

Eine Lösung von 200 mg (0,55 mMol) an (18.2) in 4 ml 4N HCI wird 16 h bei Raumtemperatur gerührt. Nach Entfernen des Lösungsmittels im Vakuum wird der Rückstand in Wasser aufgenommen und gefriergetrocknet. Man erhält 160 mg (53 %) an (18.3) als farblosen Feststoff.

### 18d) 3-[2-((4S)-(2-(1H-Benzimidazol-2-yl)-ethyl)-2,5-dioxo-imidazolidin-1-yl)-acetylamino]-(2S)-benzyloxycarbonylamino-propionsäure-tert.-butylester (18.4)

Man gibt zu einer Lösung von 160 mg (0,53 mMol) an (18.3), 72 mg (0,53 mMol) HOBt und 156 mg (0,53 mMol) an (1.6) in 5 ml DMF bei 0 °C 0,09 ml (0,53 mMol) Diisopropyl-ethylamin (DIPEA) und 120 mg (0,58 mMol) DCCI . Man rührt 20 min. bei 0 °C und 16 h bei Raumtemperatur.Der Niederschlag wird abfiltriert, das Filtrat eingeengt, der Rückstand in Essigester aufgenommen und die Essigester-Phase mit 10 %-iger KHCO₃-Lösung und gesättigter NaCI-Lösung gewaschen und über Natriumsulfat getrocknet. Nach Filtration wird das Lösungsmittel im Vakuum entfernt und der Rückstand (18.4, Rohprodukt) direkt zu (18.5) umgesetzt.

### 18e) 3-[2-((4S)-(2-(1H-Benzimidazol-2-yl)-ethyl)-2,5-dioxo-imidazolidin-1-yl)-acetylamino]-(2S)-benzyloxycarbonylamino-propionsäure (18.5)

Eine Lösung von ((18.4), Rohprodukt) in 5 ml 95 %-iger Trifluoressigsäure wird 20 min bei Raumtemperatur gerührt. Das Lösungsmittel wird im Vakuum entfernt und der Rückstand in tert.-Butanol/Wasser = 1/1 gelöst und gefriergetrocknet. Man erhält 120 mg (44 %, ausgehend von (18.3)) an (18.5) als blaßgelben Feststoff.

ES(+)-MS: 492 (M + H)⁺

### Beispiel 19:

### (2S)-Benzyloxycarbonylamino-3-[2-((4S)-(3-guanidino-3-oxo-propyl)-2,5-dioxo-imidazolidin-1-yl)-acetylamino]-propionsäure (19.3)

Die Synthese erfolgt nach folgender Reaktionssequenz:

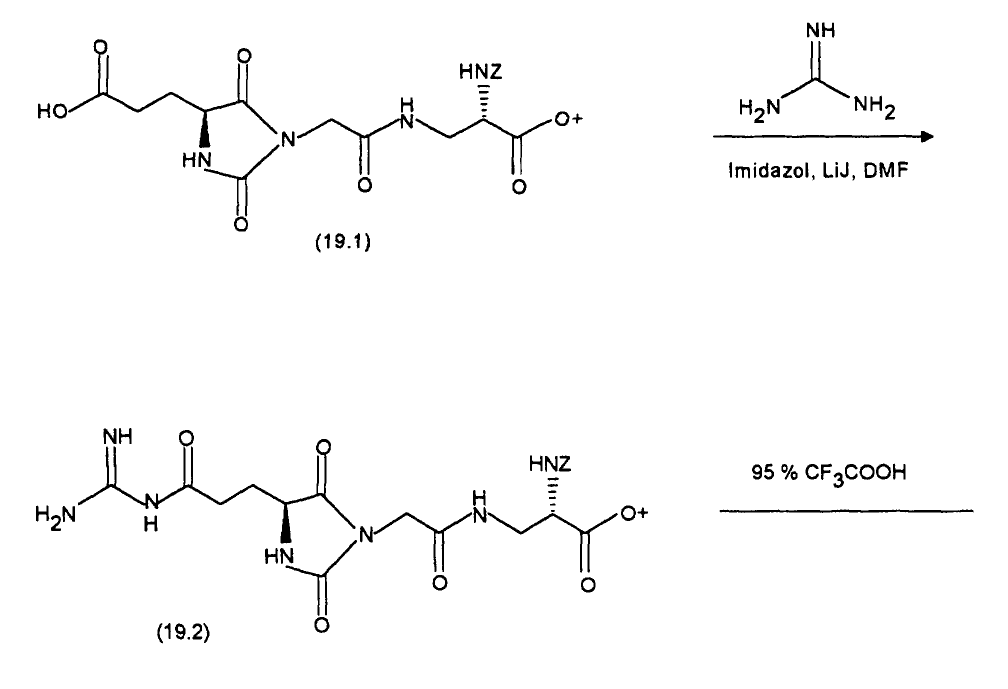

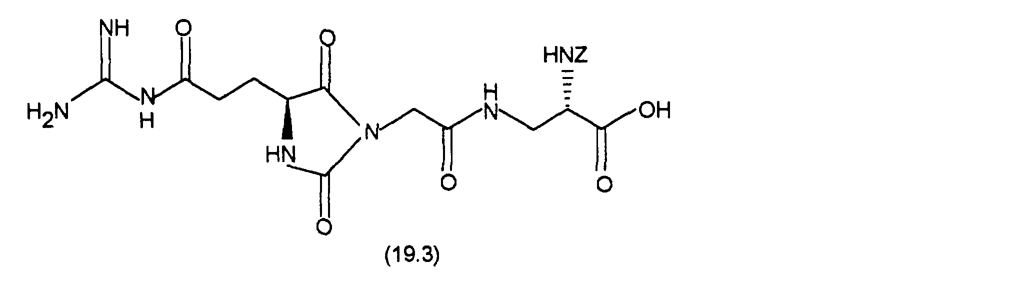

### 19a) (2S)-Benzyloxycarbonyl-amino-3-[((4S)-(2-carboxy-ethyl)-2,5-dioxo-imidazolidin-1-yl)-acetylamino]-propionsäure-tert.butylester (19.1)

Die Synthese von (19.1) erfolgt ausgehend von L-Glutaminsäure analog der Synthese von (7.6) ausgehend von L-/ sparaginsäure (siehe Beispiel 7).

### 19b) (2S)-Benzyloxycarbonylamino-3-[2-((4S)-(3-guanidino-3-oxo-propyl)-2,5-dioxo-imidazolidin-1-yl)-acetylamino]-propionsäure-tert.-butylester (19.2)

Man gibt zu einer Lösung von 170 mg (2,88 mMol) Guanidin und 6,5 mg (0,09 mMol) Imidazol in 2 ml DMF 12,8 mg (0,09 mMol) Lithiumiodid und eine Lösung von 500 mg (0,96 mMol) an (19.1) in 5 ml DMF und läßt das Gemisch über Nacht bei Raumtemperatur rühren. Das Lösungsmittel wird im Vakuum entfernt, der Rückstand mit Essigester behandelt, die Essigester-Phase mit KHCO₃-Lösung gewaschen und über Natriumsulfat getrocknet. Nach Filtration wird das Lösungsmittel im Vakuum entfernt und man erhält 30 mg an (19.2, Rohprodukt), die direkt zur Synthese von (19.3) eingesetzt werden.

### 19c) (2S)-Benzyloxycarbonylamino-3-[2-((4S)-(3-guanidino-3-oxo-propyl)-2,5-dioxo-imidazolidin-1-yl)-acetylamino]-propionsäure (19.3)

Eine Lösung von 30 mg an (19.2, Rohprodukt) in 1 ml 95 %-iger Trifluoressigsäure werden 10 min. bei Raumtemperatur gerührt. Das Lösungsmittel wird im Vakuum entfernt und der Rückstand mittels präperativer HPLC über RP 18 gereinigt. Nach Einengen der Produktfraktionen und Gefriertrocknen erhält man 9,5 mg (2 %, ausgehend von (19.1)) an (19.3) als farblosen Feststoff.

ES(+)-MS: 492 (M + H)⁺

### Beispiel 20

### (2S)-Benzyloxycarbonylamino-3-[2-(2,5-dioxo-(4S)-(2-(1,4,5,6-tetrahydro-pyrimidin-2-ylcarbamoyl)-ethyl)-imidazolidin-1-y')-acetylamino]-propionsäure (20.2)

Die Synthese erfolgte nach folgender Reaktionssequenz:

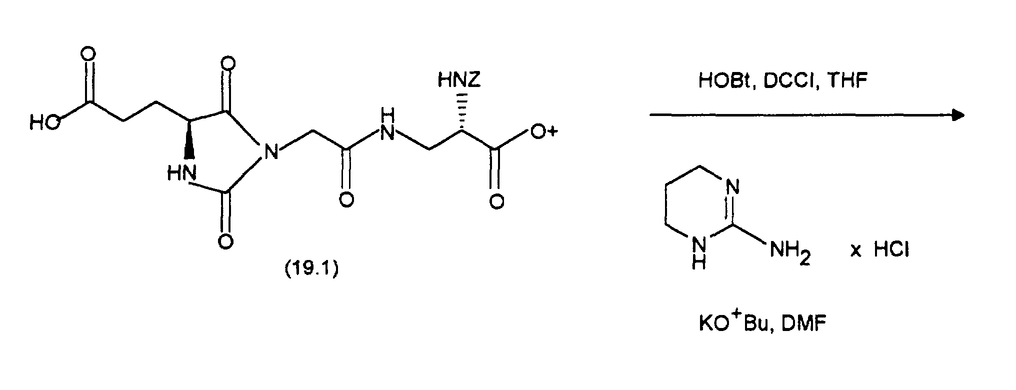

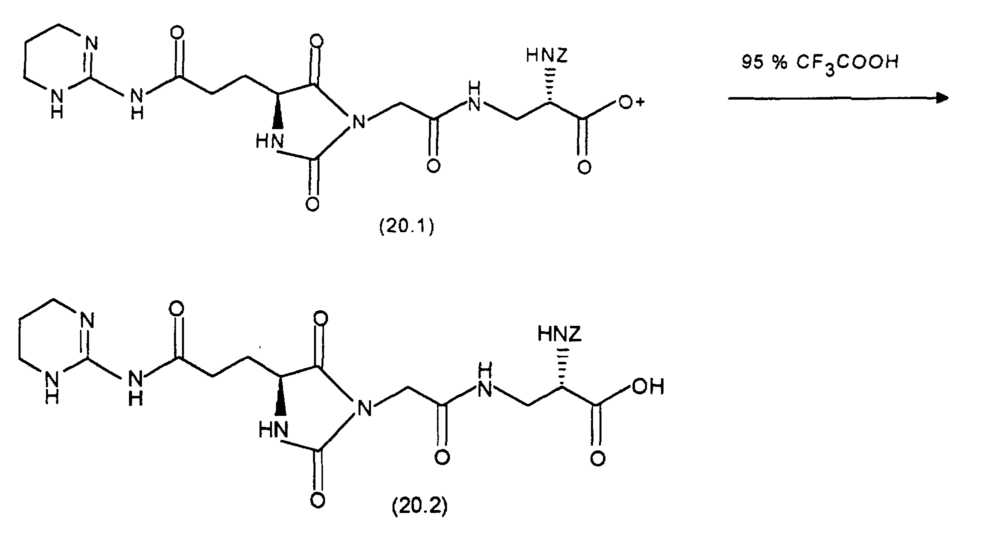

### 20a) (2S)-Benzyloxycarbonylamino-3-[2-(2,5-dioxo-(4S)-(2-(1,4,5,6-tetrahydro-pyrimidin-2-ylcarbamoyl)-ethyl)-imidazolidin-1-yl)-acetylamino]-propionsäure-tert.-butylester (20.1)

Man gibt zu einer Lösung von 506 mg (1mMol) an (19.1) in 4 ml absolutem Tetrahydrofuran unter Eiskühlung 135 mg (1 mMol) HOBt, 206 mg (1,1 mMol) DCCI und läßt das Gemisch 30 min. rühren. Diese Lösung gibt man anschließend zu einer Lösung von 136 mg (1 mMol) 1,4,5,6-Tetrahydro-pyrimidin-2-ylamin-hydrochlorid und 112 mg Kalium-tert.-butylat in DMF und läßt das Gemisch 1 h bei Raumtemperatur rühren. Das Lösungsmittel wird im Vakuum entfernt und der Rückstand mit Diethylether verrieben. Man erhält nach Chromatographie über Kieselgel mit Dichlormethan/Methanol/Essigsäure/Wasser = 9/1/0,1/0,1, Einengen der Produktfraktionen und Gefriertrocknen 90 mg (15 %) an (20.1).

### 20b) (2S)-Benzyloxycarbonylamino-3-[2-(2,5-dioxo-4-(2-(1,4,5,6-tetrahydro-pyrimidin-2-y)carbamoyl)-ethyl)-imidazolidin-1-yl)-acetylamino]-propionsäure (20.2)

Eine Lösung von 80 mg (0,136 mMol) an (20.1) in 10 ml 95 %-iger Trifluoressigsäure werden 20 min. bei Raumtemperatur gerührt. Das Lösungsmittel wird im Vakuum entfernt, der Rückstand in Wasser gelöst und gefriergetrocknet. Man erhält 70 mg (97 % ) an (20.2) als farblosen Feststoff.

ES(+)-MS: 532 (M + H)⁺

### Beispiel 21:

### 4-[(4R,S)-(4-((1H-Benzimidazol-2-ylamino)-methyl)-phenyl)-4-methyl-2,5-dioxo-imidazolidin-1-yl]-2-benzyloxycarbonylamino-butansäure (21.7)

Die Synthese erfolgte nach folgender Reaktionssequenz:

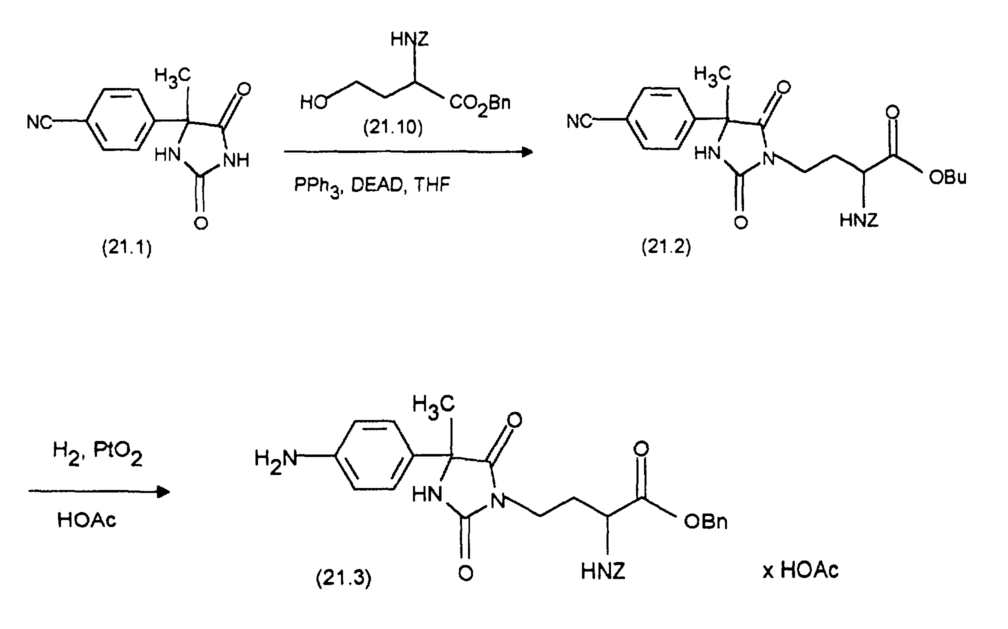

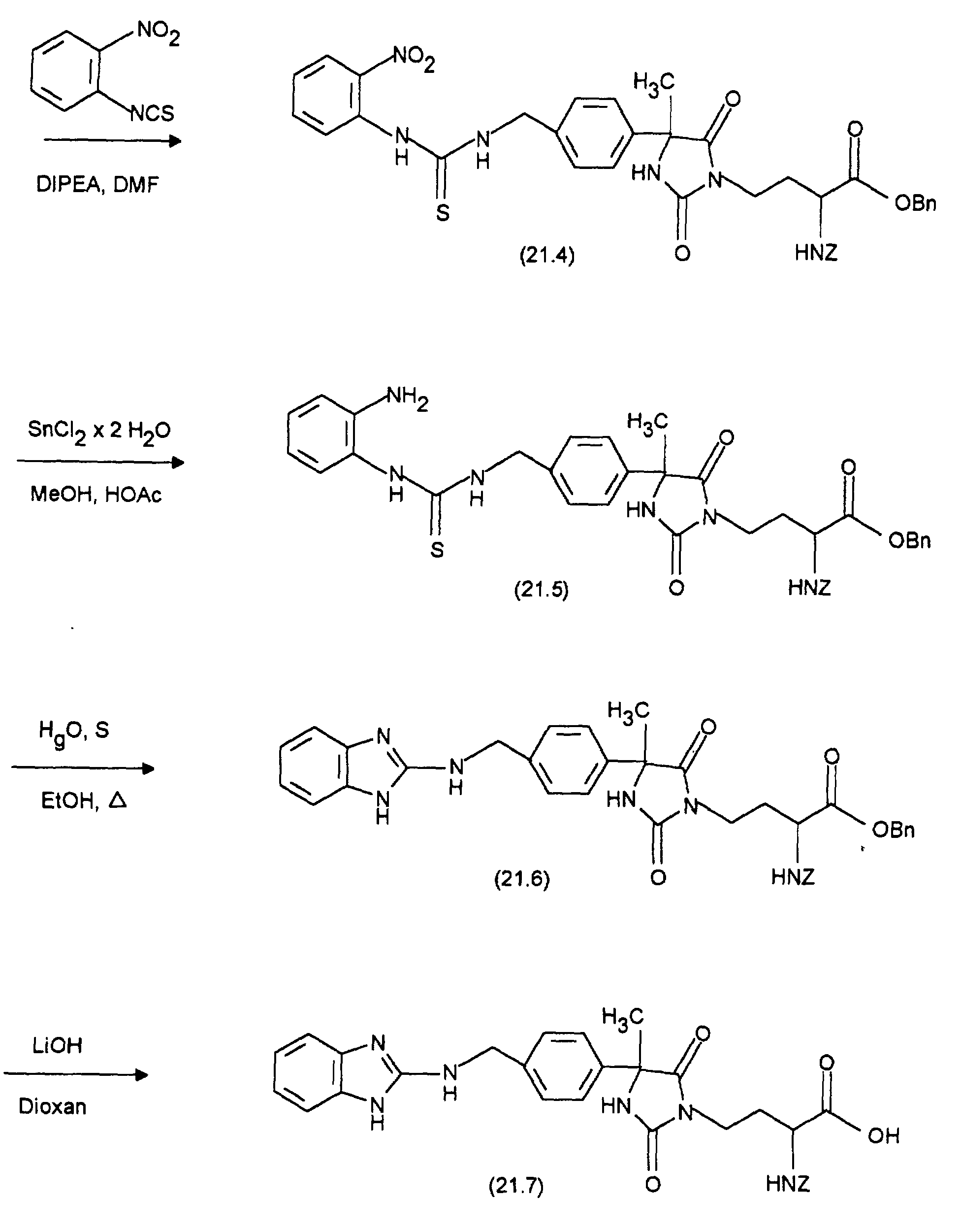

### 21a) 2-Benzyloxycarbonylamino-4-[(4R,S)-(4-cyano-phenyl)-4-methyl-2,5-dioxo-imidazolidin-1-yll-butansäure (21.2)

Man gibt zu einer Lösung von 1,07 g (5 mMol) an (21.1) (Synthese siehe WO 95/14008), 1,3 g (5 mMol) Triphenylphosphin und 1,7 g (5 mMol) an (21.10) in 20 ml absolutem Tetrahydrofuran eine Lösung von 870 mg (0,5 mMol) Diethyl-azo-dicarboxylat (DEAD) in absolutem Tetrahydrofuran. Nach 16 Rühren bei Raumtemperatur wird das Lösungsmittel im Vakuum entfernt, der Rückstand in Essigester aufgenommen, die Essigester-Phase mit 10 %-iger Zitronensäurelösung und gesättigter NaHCO₃-Lösung gewaschen und über Natriumsulfat getrocknet. Nach Filtration wird das Lösungsmittel im Vakuum entfernt und der Rückstand mit Dichlormethan/tert.-Butyl-methylether = 1/1 über Kieselgel chromatographiert. Nach Einengen der Produktfraktionen erhält man 1,2 g (44 %) an (21.2).

### 21b) 4-[(4R,S)-(4-Aminomethyl-phenyl)-4-methyl-2,5-dioxo-imidazolidin-1-yl]-2-benzyloxycarbonylamino-butansäure-benzylester-essigsäure-salz (21.3)

Eine Lösung von 1,2 g (2,22 mMol) an (21.2) in Essigsäure wird über 150 mg Platinoxid hydriert. Sobald die Wasserstoffaufnahme beendet ist (nach ca. 3 h) wird der Katalysator abfiltriert, das Filtrat eingeengt und der Rückstand mit Dichlormethan/Methanol/Essigsäure/Wasser = 6/1/0,1/0,1 über Kieselgel gereinigt. Nach Einengen der Produktfraktionen erhält man 450 mg (34 %) an (21.3).

### 21c) 2-Benzyloxycarbonylamino-4-[(4R,S)-methyl-4-(4-(3-(2-nitro-phenyl)-thioharnstoff-methyl)-phenyl)-2,5-dioxo-imidazolidin-1-yl]-butansäurebenzylester (21.4)

Man gibt zu einer Lösung von 410 mg (0,75 mMol) an (21.3) in 5 ml DMF 135 mg (0,75 mMol) 2-Nitrophenylisothiocyanat und 0,13 ml Diisopropyl-ethylamin (DIPEA). Nach 16 h Rühren bei Raumtemperatur wird das Lösungsmittel im Vakuum entfernt und der Rückstand mit tert.-Butyl-methylether über Kieselgel chromatographiert. Man erhält 380 mg (70 %) an (21.4).

### 21d) 4-[(4R,S)-(4-(3-(2-Amino-phenyl)-thioharnstoff-methyl)-phenyl)-4-methyl-2,5-dioxo-imidazolidin-1-yl]-2-benzyloxycarbonylamino-butansäure-benzylester (21.5)

Man gibt zu einer Lösung von 360 mg (0,49 mMol) an (21.4) in 5 ml Methanol 650 mg SnCl₂ x 2 H₂O (2,9 mMol) und 3 Tropfen Essigsäure und läßt das Gemisch 16 h bei Raumtemperatur rühren.

Nach Entfernen des Lösungsmittels im Vakuum wird der Rückstand mit Dichlormethan/Methanol = 20/1 über Kieselgel chromatographiert. Man erhält 280 mg (82 %) an (21.5).

### 21e) 4-[(4R,S)-(4-((1H-Benzimidazol-2-ylamino)-methyl)-phenyl)-4-methyl-2,5-dioxo-imidazolidin-1-yl]-2-benzyloxycarbonylamino-butansäure-benzylester (21.6)

Man erhitzt ein Gemisch aus 270 mg (0,38 mMol) an (21.5), 173 mg (0,8 mMol) Quecksilberoxid und 3 mg Schwefel in 10 ml Ethanol 12 h unter Rückfluß. Nach Filtration und Entfernen des Lösungsmittels im Vakuum erhält man 180 mg an (21.6, Rohprodukt) als rotbraunen Sirup, der direkt zu (21.7) umgesetzt wird.

### 21f) 4-[(4R,S)-(4-((1H-Benzimidazol-2-ylamino)-methyl)-phenyl)-4-methyl-2,5-dioxo-imidazolidin-1-yl]-2-benzyloxycarbonylamino-butansäure (21.7)

Man gibt zu einer Lösung von 180 mg (ca. 0,27 mMol) an (21.6, Rohprodukt) in 4 ml Dioxan 0,27 ml einer 1M LiOH-Lösung und läßt das Gemisch über Nacht bei Raumtemperatur rühren. Der Großteil des Dioxans wird im Vakuum entfernt, der wäßrige Rückstand mit Zitronensäure auf pH 5 gestellt und die Wasser-Phase mit Essigester extrahiert. Nach Trocknen über Natriumsulfat, Filtration und Einengen des Filtrats im Vakuum wird der Rückstand mit Dichlormethan/Methanol/Essigsäure/Wasser = 6/1/0,1/0,1 über Kieselgel chromatographiert. Nach Einengen der Produktfraktionen und Gefriertrocknen erhält man 107 mg (49 %, ausgehend von (21.5)) an (21.7) als farblosen Feststoff.

ES(+)-MS: 571 (M + H)⁺

### 21g) Synthese von 2-Benzyloxycarbonylamino-4-hydroxy-butansäurebenzylester (21.10)

Die Synthese erfolgte nach folgender Reaktionssequenz:

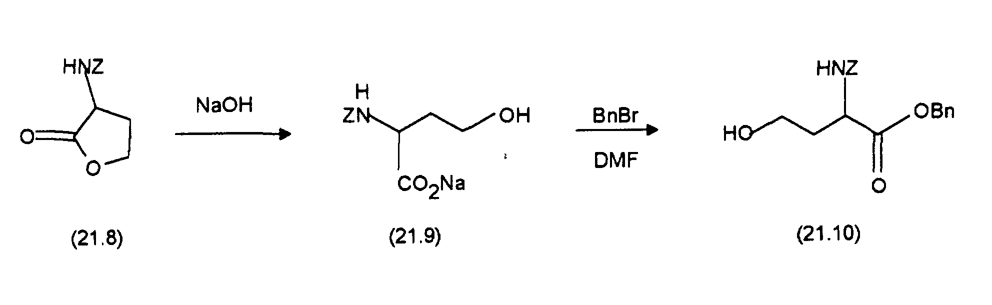

Man rührt eine Suspension von 10 g (42 mMol) an (21.8) in 420 ml 0,1 M NaOH in Ethanol 16 h bei Raumtemperatur. Die Reaktionslösung wird eingeengt, der Rückstand mit Toluol versetzt und das Lösungsmittel im Vakuum entfernt. Man gibt 130 ml DMF und 9,34 g (54,6 mMol) Benzylbromid zu und läßt 4 Tage bei Raumtemperatur rühren. Man gibt 2,1 Liter 1M NaHCO₃-Lösung zu und extrahiert 3 mal mit Essigester. Die vereinigten Essigester-Phasen werden mit 1 M NaHCO₃-Lösung und gesättigter NaCI-Lösung gewaschen und über Magnesiumsulfat getrocknet. Nach Filtration wird das Lösungsmittel im Vakuum entfernt und der Rückstand mit Dichlormethan/Acetonitril = 20/1 bis 20/6 über Kieselgel chromatographiert. Die Produktfraktionen werden eingeengt und der Rückstand mit Diethylether/Hexan kristallisiert. Man erhält 5,4 g (38 %) an (21.10) als farblose Kristalle.

### Beispiel 22:

### 3-[2-((4S)-(3-(1H-Benzimidazol-2-ylamino)-propyl)-2,5-dioxo-imidazolidin-1-yl)-acetylamino)-(2S)-(2-chloro-benzyloxycarbonylamino]-propionsäure (22.4)

Die Synthese erfolgte nach folgender Reaktionssequenz:

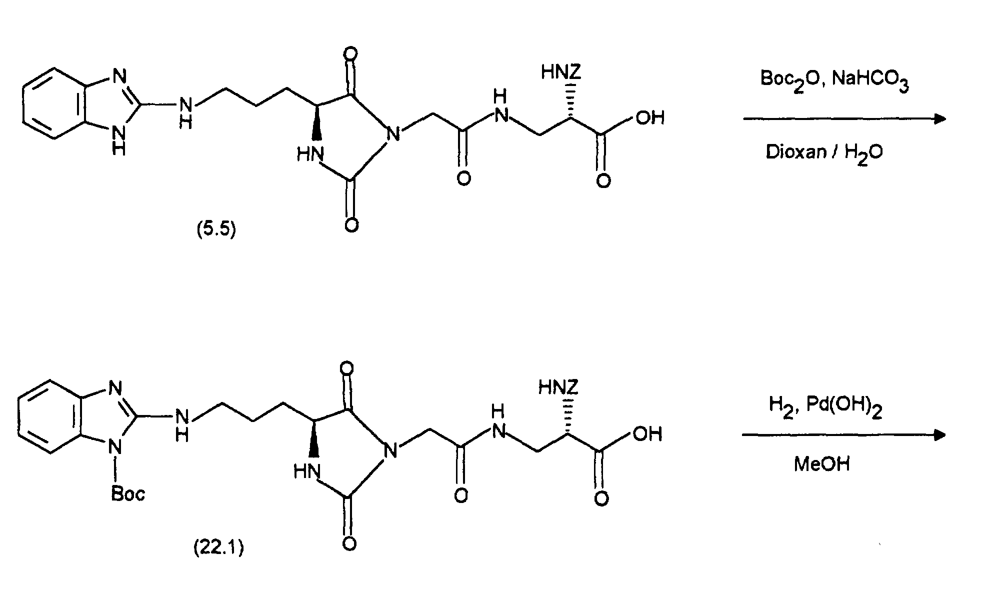

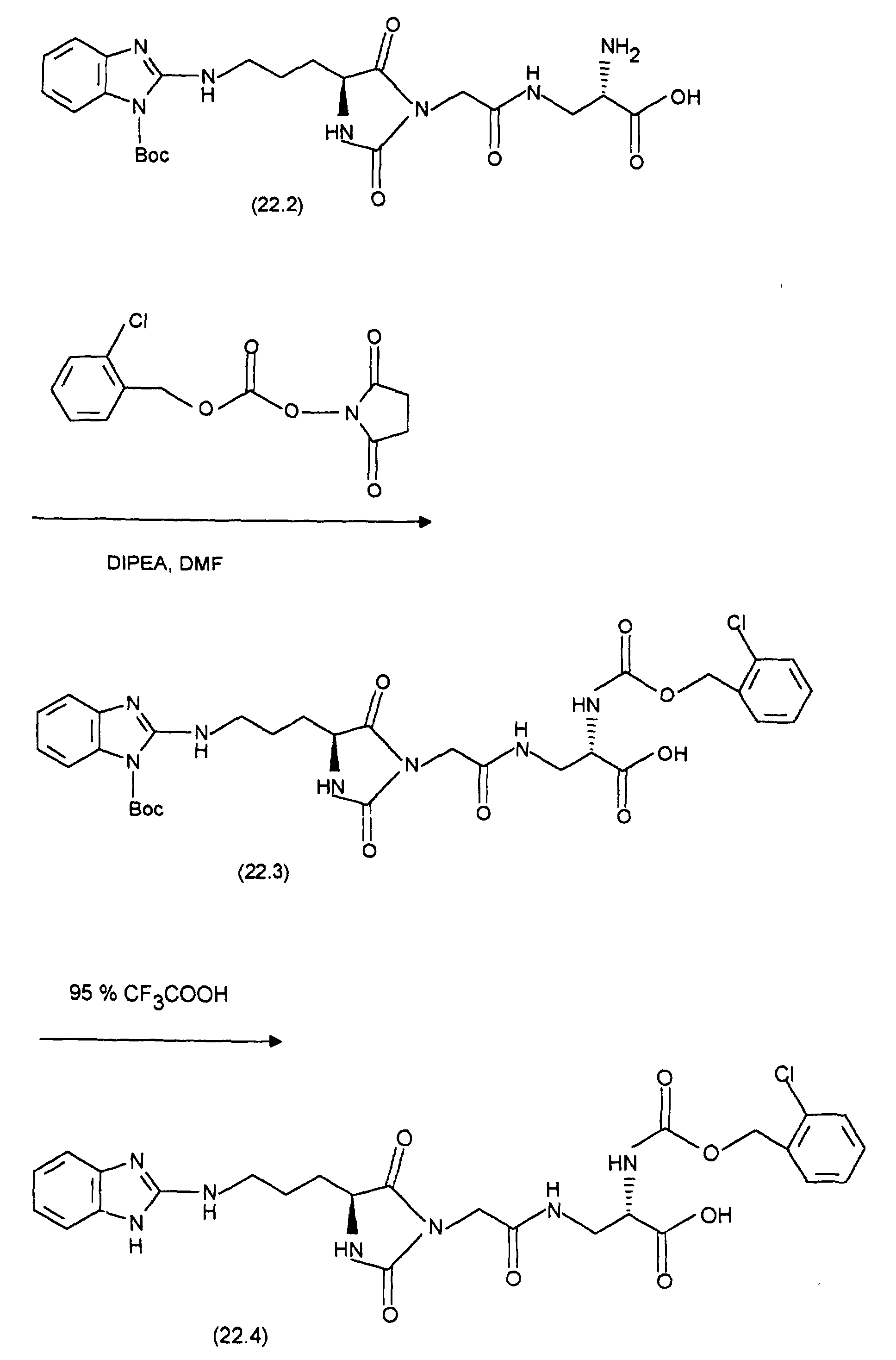

### 22a) 3-[2-((4S)-(3-(1-tert.-Butoxycarbonyl-benzimidazol-2-ylamino)-propyl)-2,5-dioxo-imidazolidin-1-yl)-acetylamino]-(2S)-benzyloxycarbonylamino-propionsäure (22.1)

Man gibt zu einer Lösung von 551 mg (1 mMol) an (5.5) in 4 ml Dioxan/Wasser = 1/1 168 mg (2 mMol) NaHCO₃ und 437 mg (2 mMol) Di-tert.butyldicarbonat und läßt das Gemisch 16 h bei Raumtemperatur rühren. Das Lösungsmittel wird im Vakuum entfernt, der Rückstand in Essigester aufgenommen, die EssigesterPhase mit 2 %-iger Zitronensäure-Lösung und gesättigter NaCl-Lösung gewaschen und über Magnesiumsulfat getrocknet. Nach Filtration wird das Lösungsmittel im Vakuum entfernt und man erhält 614 mg (94 %) an (22.1) als farblose Kristalle.

### 22b) (2S)-Amino-3-[2-((4S)-(3-(1-tert.-butoxycarbonyl-benzimidazol-2-ylamino)-propyl)-2,5-dioxo-imidazolidin-1-yl)-acetylamino]-propionsäure (22.2)

Eine Lösung von 600 mg (0,92 mMol) an (22.1) in Methanol wird über Palladiumhydroxid hydriert. Der Katalysator wird abfiltriert, das Filtrat eingeengt und der Rückstand 2 mal mit Toluol einrotiert und anschließend im Hochvakuum getrocknet. Man erhält 385 mg (81 %) an (22.2).

### 22c) 3-[2-((4S)-(3-(1-tert.-Butoxycarbonyl-benzimidazol-2-ylamino)-propyl)-2,5-dioxo-imidazolidin-1-yl)-acetylamino)-(2S)-(2-chloro-benzyloxycarbonylamino]-propionsäure (22.3)

Man gibt zu einer Lösung von 61,7 mg (0,12 mMol) an (22.2) in 2,5 ml DMF bei 0 °C 0,017 ml Diisopropyl-ethylamin und anschließend eine Lösung von 28,4 mg (0,1 mMol) N-(2-Chlorbenzyloxycarbonyloxy)-succinimid und läßt das Reaktionsgemisch 16 h bei Raumtemperatur rühren. Das Lösungsmittel wird im Vakuum entfernt und der Rückstand zwischen Essigester und 2 %-iger Zitronensäure verteilt. Die Phasen werden getrennt und die organische Phase über Magnesiumsulfat getrocknet. Nach Filtration, Einengen des Filtrats im Vakuum und Kristallisation des Rückstandes mit Diethylether erhält man 36,5 mg (45 %) an (22.3) als farblose Kristalle.

### 22d) 3-[2-((4S)-(3-(1H-Benzimidazol-2-ylamino)-propyl)-2,5-dioxo-imidazolidin-1-yl)-acetylamino)-(2S)-(2-chloro-benzyloxycarbonylamino]-propionsäure (22.4)

Eine Lösung von 36,5 mg an (22.3) in 3 ml 95 %-iger Trifluoressigsäure wird 1 h bei Raumtemperatur gerührt. Die Trifluoressigsäure wird im Vakuum entfernt und der Rückstand 2 mal mit Toluol einrotiert. Der Rückstand wird in Methanol gelöst und (22.4) mit Diethylether gefällt. Nach Zentrifugieren wird der Rüchstand in verdünnter Essigsäure aufgenommen und gefriergetrocknet. Man erhält 24 mg (77 %) an (22.4) als farblosen Feststoff.

ES(+)-MS: 586 (M + H)⁺

### Beispiele 23 - 33

Die Herstellung der Verbindungen (23) bis (33) erfolgt nach folgender Reaktionssequenz:

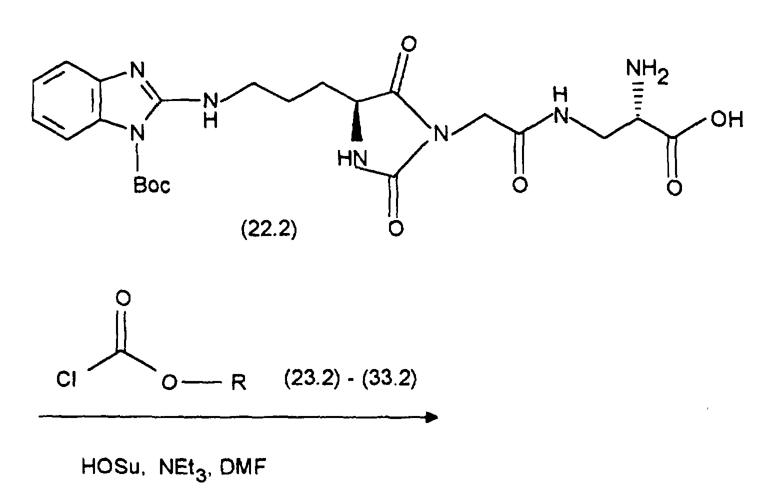

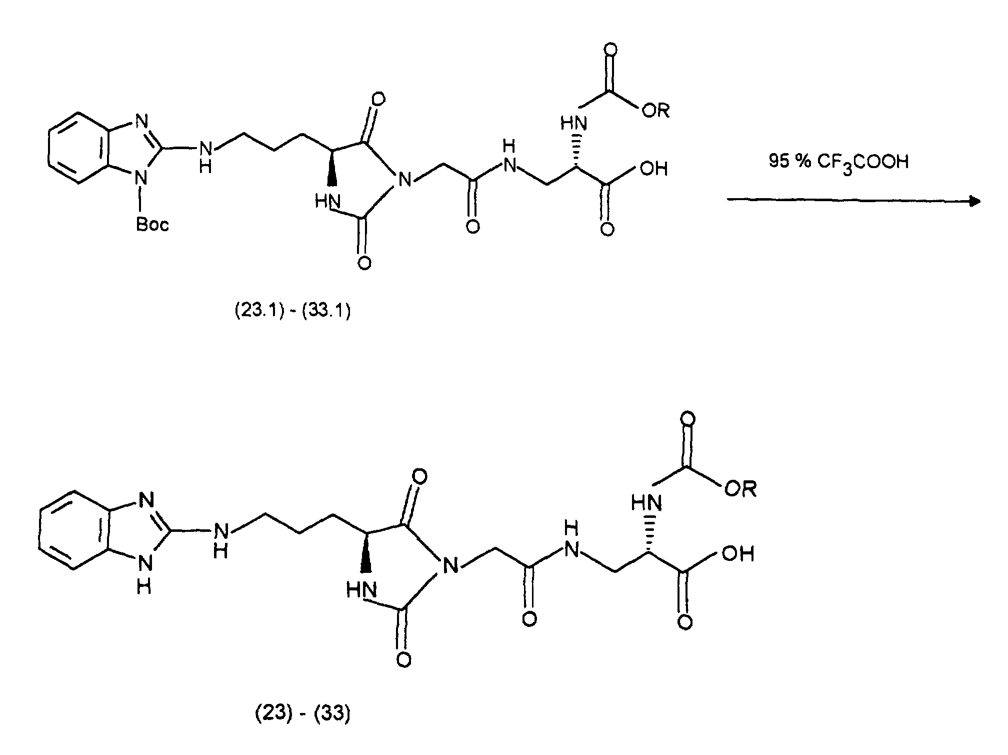

(Synthesebeschreibung siehe nach Beispiel 33)

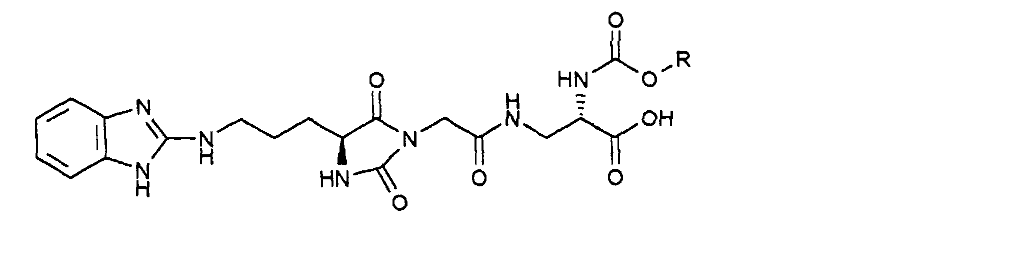

### Beispiel 23

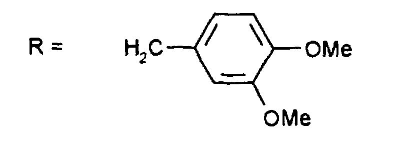

### 3-[2-((4S)-3-(1H-Benzimidazol-2-ylamino)-propyl)-2,5-dioxo-imidazolidin-1-yl)acetylamino)-(2S)-(3,4-dimethoxy-benzyloxycarbonylamino)-propionsäure (23)

ES(+)-MS: 612 (M + 1)⁺

### Beispiel 24

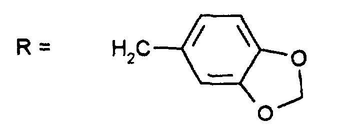

### 3-[2-((4S)-(3-(1H-Benzimidazol-2-ylamino)-propyl)-2,5-dioxo-imidazolidin-1-yl)-acetylamino)-(2S)-(3,4-methylendioxy-benzyloxycarbonyl)-propionsäure (24)

ES(+)-MS: 624 (M + 1)⁺

### Beispiel 25

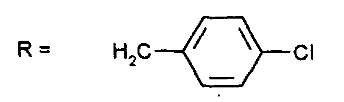

### 3-[2-((4S)-(3-(1H-Benzimidazol-2-ylamino)-propyl)-2,5-dioxo-imidzaolidin-1-yl)-acetylamino)-(2S)-(4-chloro-benzyloxycarbonylamino]-propionsäure (25)

ES(+)-MS: 586 (M + 1)⁺

### Beispiel 26

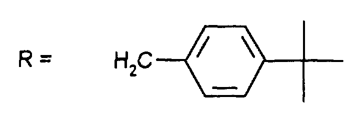

### 3-[2-((4S)-(3-(1H-Benzimidazol-2-ylamino)-propyl)-2,5-dioxo-imidazolidin-1-yl)-acetylamino)-(2S)-(4-tert.-butyl-benzyloxycarbonylamino]propionsäure (26)

ES(+)-MS: 608 (M + 1)⁺

### Beispiel 27

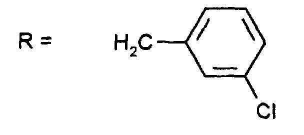

### 3-[2-((4S)-(3-(1H-Benzimidazol-2-ylamino)-propyl)-2,5-dioxo-imidazolidin-1-yl)-acetylamino)-(2S)-(3-chloro-benzyloxycarbonylamino]-propionsäure (27)

ES(+)-MS: 586 (M + 1)⁺

### Beispiel 29

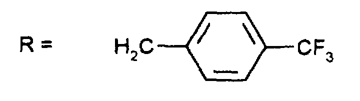

### 3-[2-((4S)-3-(1H-Benzimidazol-2-ylamino)-propyl)-2,5-dioxo-imidazolidin-1-yl)-acetylamino)-(2S)-(4-trifluormethyl-benzyloxycarbonylamino]-propionsäure (29)

ES(+)-MS: 620 (M + 1)⁺

### Beispiel 30

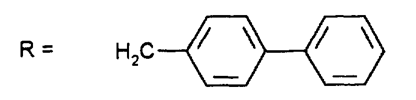

### 3-[2-((4S)-(3-(1H-Benzimidazol-2-ylamino)-propyl)-2,5-dioxo-imidazolidin-1-yl)-acetylamino]-(2S)-(4-phenyl-benzyloxycarbonylamino)-propionsäure (30)

ES(+)-MS: 628 (M + 1)⁺

### Beispiel 31

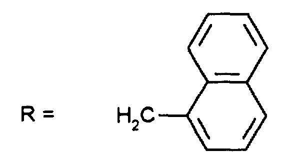

### 3-[2-((4S)-(3-(1H-Benzimidazol-2-ylamino)-propyl)-2,5-dioxo-imidazolidin-1-yl)-acetylamino]-(2S)-(naphthalin-1-ylmethoxycarbonylamino)-propionsäure (31)

ES(+)-MS: 602 (M + 1)⁺

### Beispiel 32

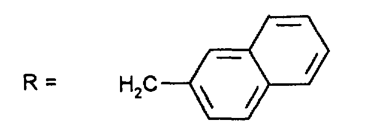

### 3-[2-(4S)-(3-(1H-Benzimidazol-2-ylamino)-propyl)-2,5-dioxo-imidazolidin-1-yl)-acetylamino]-(2S)-(naphthalin-2-yl-methoxycarbonylamino)-propionsäure (32)

ES(+)-MS: 602 (M + 1)⁺

### Beispiel 33

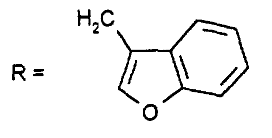

### (2S)-(Benzofuran-3-ylmethoxycarbonylamino)-3-[2-((4S)-(3-(1H-benzimidazol-2-ylamino)-propyl)-2,5-dioxo-imidazolidin-1-yl)-acetylamino]-propionsäure (33)

ES(+)-MS: 592 (M + 1)⁺

### a) Herstellung der Chlorameisensäureester (23.2) - (33.2)

Die Synthese erfolgt nach folgender allgemeiner Herstellvorschrift:

Zu einer Lösung von 2,2 Äquivalenten Bis-trichlormethylcarbonat in absolutem Dichlormethan tropft man bei 0 °C eine Lösung von 6 Äquivalenten des entsprechenden Alkohols (HO-R) und 6 Äquivalente Pyridin in absolutem Dichlormethan. Anschließend läßt man das Gemisch 2 h bei Raumtemperatur rühren, entfernt das Lösungsmittel im Vakuum, nimmt den Rüchstand in Essigester oder Ether auf, läßt das Gemisch 30 min. bei Raumtemperatur stehen und filtriert den eventuell anfallenden Niederschlag ab. Nach Entfernen des Lösungsmittels im Vakuum wird der Rückstand im Hochvakuum getrocknet und direkt zur Synthese von (23.1) - (33.1) eingesetzt.

### b) Herstellung der Verbindungen (23.1) - (33.1)

Die Synthese erfolgt nach folgender allgemeinen Herstellvorschrift:

Man gibt zu einer Lösung von 1 Äquivalent N-Hydroxysuccinimid in THF bei O °C 1 Äquivalent des entsprechenden Chlorameisensäureesters (23.2) - (33.2) und 1 Äquivalent Diisopropyl-ethylamin (DIPEA). Man läßt 30 min. bei 0 °C und 45 min. bei Raumtemperatur rühren und gibt diese Lösung zu einer Lösung von 1 Äquivalent (22.2) in DMF. Man läßt das Gemisch bei Raumtemperatur rühren bis die Reaktion beendet ist und entfernt das Lösungsmittel im Vakuum. Der Rückstand wird in Essigester aufgenommen und die Essigester-Phase 2 mal mit einer wäßrigen Zitronensäure-Lösung (pH 3) und gesättigter NaCI-Lösung gewaschen. Nach Trocknen über Natriumsulfat, Filtration und Entfernen des Lösungsmittels im Vakkum wird der Rückstand über Kieselgel chromatographiert. Nach Einengen der Produktfraktionen erhält man die Verbindungen (23.1) - (33.1).

### c) Herstellung der Verbindungen (23) - (33)

Die Herstellung erfolgt aus (23.1) - (33.1) durch Spaltung der tert.-Butoxycarbonylgruppe mit 95 %-iger Trifluoressigsäure wie bei der Herstellung von (22.4) aus (22.3) beschrieben.

### Beispiel 34

### 3-[8-(3-(1H-Benzimidazol-2-ylamino)-propionyl)-2,4-dioxo-1,3,8-triaza-spiro[4,5]dec-3-yl]-(2S)-benzyloxycarbonylamino-propionsäure (34.12)

Die Synthese erfolgte nach folgender Reaktionssequenz:

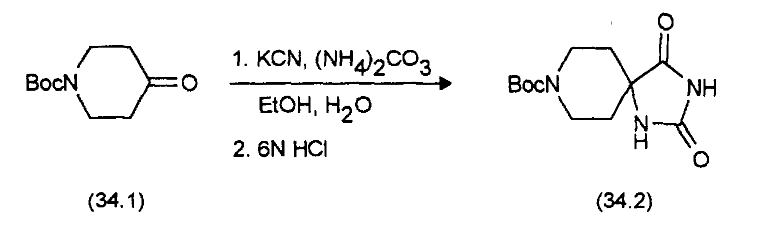

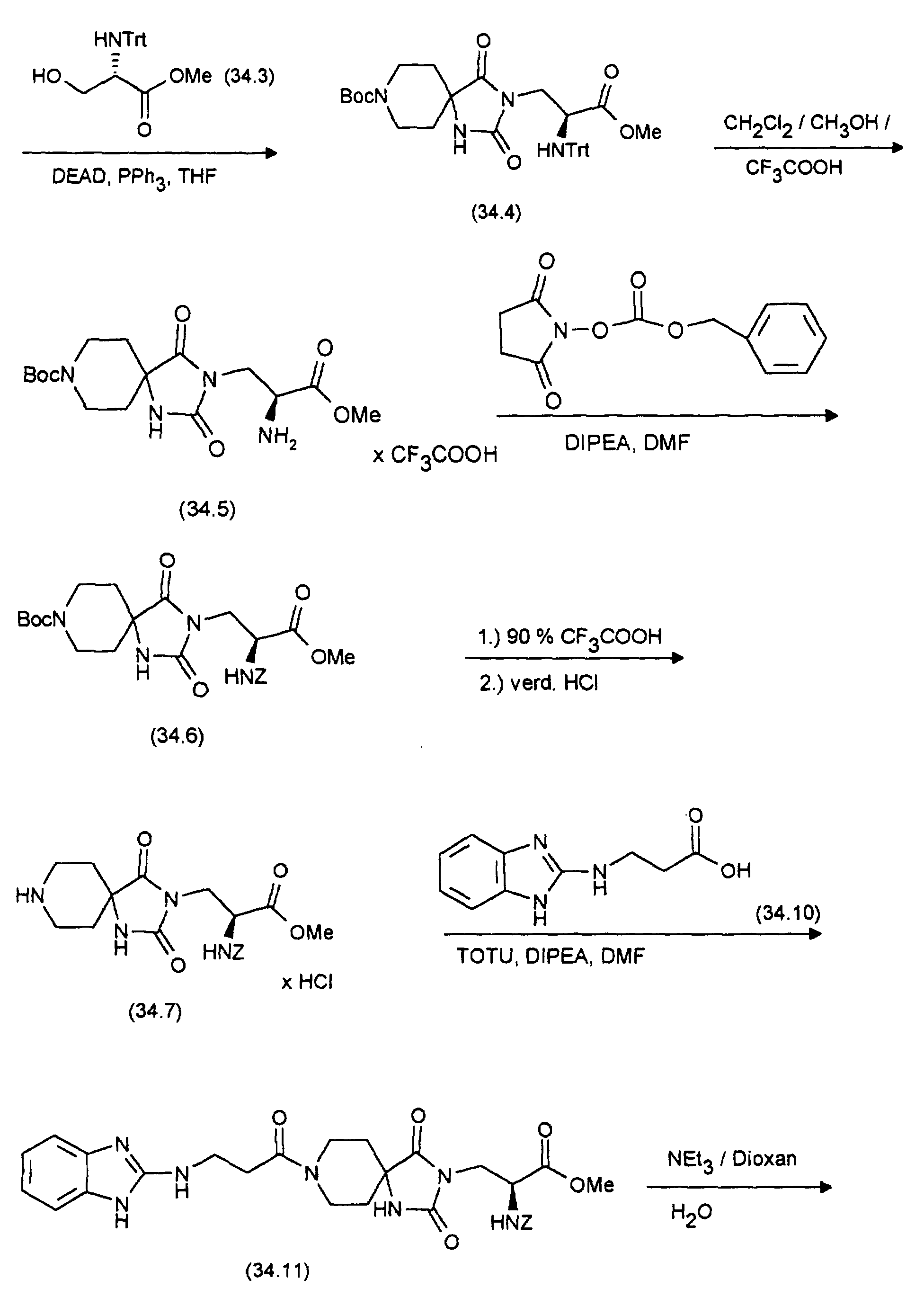

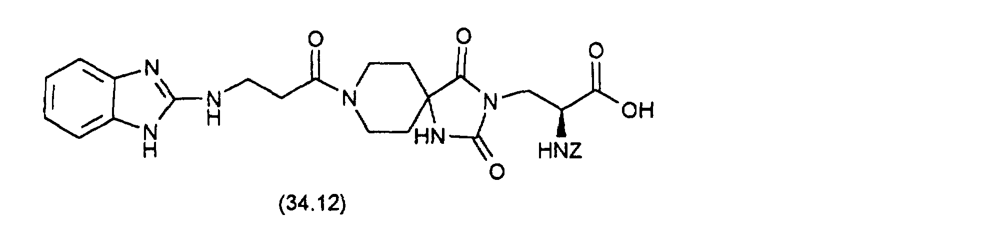

### 34a) 2,4-Dioxo-1,3,8-triaza-spiro[4.5]decan-8-carbonsäure-tert.-butylester (34.2)

Man gibt zu einer Lösung von 5 g (25,1 mMol) N-tert.-Butoxycarbonyl-4-piperidon (34.1) und 24,01 g (250 mMol) Ammoniumcarbonat in 80 ml EtOH/Wasser = 1/1 2,12 g (32,6 mMol) Kaliumcyanid und läßt das Gemisch 5 h bei 60 °C rühren. Man stellt anschließend durch Zugabe von 6N HCI pH 6,3 ein und rührt weitere 1,5 h bei 60 °C. Der Niederschlag wird abgesaugt und im Hochvakuum getrocknet. Man erhält 3,43 g an (34.2) als farblosen Feststoff. Durch Extraktion des Filtrats mit Dichlormethan, Trocknen der organischen Phase über Natriumsulfat, Filtration und Entfernen des Lösungsmittels im Vakuum erhält man weitere 1,0 g an (34.2). Gesamtausbeute an (34.2): 4,43 g (66 %), farbloser Feststoff.

### 34 b) N-Trityl-L-Serin-methylester (34.3)

Man gibt zu einer Lösung von 6 g (38,56 mMol) L-Serin-methylester-hydrochlorid und 7,8 g (77,12 mMol) Triethylamin in absolutem THF bei 0 °C eine Lösung von 10,75 g (38,56 mMol) Tritylchlorid und läßt die Lösung 4h bei 0 °C und 2 Tage bei Raumtemperatur rühren. Das Lösungsmittel wird im Vakuum entfernt und der Rückstand zwischen Dichlormethan und 10 %-iger wäßriger Zitronensäure-Lösung verteilt Die Phasen werden getrennt, die organische Phase mit Wasser gewaschen und über Natriumsulfat getrocknet. Nach Filtration wird das Lösungsmittel im Vakuum entfernt und der Rückstand mit Heptan/Essigester = 6/4 über Kieselgel chromatographiert. Nach Einengen der Produktfraktionen erhält man 8 g (58 %) an (34.3).

### 34 c) 3-[2-Methoxycarbonyl-(2S)-(trityl-amino)-ethyl]-2,4-dioxo-1,3,8-triaza-spiro[4.5]decane-8-carbonsäure-tert.-butylester (34.4)

Man gibt zu einer Lösung von 3,72 g (10,3 mMol) an (34.3) und 3,5 g (13,34 mMol) Triphenylphosphin in 20 ml absolutem THF eine Lösung von 3,6 g (13,37 mMol) an (34.2) in 15 ml absolutem THF. Zu dieser Lösung gibt man 2,11 ml (13,37 mMol) Diethyl-azo-dicarboxylat (DEAD) und rührt das Reaktionsgemisch bei Raumtemperatur bis die Reaktion beendet ist. Das Lösungsmittel wird im Vakuum entfernt und der Rückstand mit Heptan/Essigester über Kieselgel chromatographiert. Nach Einengen der Produktfraktionen erhält man 6 g (95 %) an (34.4).

### 34 d) 3-((2S)-Amino-2-methoxycarbonyl-ethyl)-2,4-dioxo-1,3,8-triaza-spiro[4.5]decane-8-carbonsäure-tert.-butyl ester (34.5)

Eine Lösung von 4,6 g (7,5 mMol) an (34.4) in 180 ml Dichlormethan/Methanol/Trifluoressigsäure = 95,5/3/1,5 wird 10 min. bei Raumtemperatur gerührt. Das Reaktionsgemisch wird eingeengt und der Rückstand über Kieselgel chromatographiert. Nach Einengen der Produktfraktionen erhält man 3,14 g (87 %) an (34.5).

### 34 e) 3-((2S)-Benzyloxycarbonylamino-2-methoxycarbonyl-ethyl)-2,4-dioxo-1,3,8-triaza-spiro[4.5]decane-8-carbonsäure-tert.-butyl ester (34.6)

Man gibt zu einer Lösung von 3,14 g (8,48 mmol) an (34.5) und 2,11 g (8,48 mMol) N-Benzyloxycarbonyloxy-succinimid in 80 ml absolutem DMF 2,08 g (16,1 mMol) Diisopropyl-ethylamin in 25 ml absolutem DMF und läßt das Gemisch 3 h bei Raumtemperatur rühren. Das Lösungsmittel wird im Vakuum entfernt und der Rückstand mit Heptan/Essigester = 6/4 über Kieselgel chromatographiert. Nach Einengen der Produktfraktionen erhält man (34.6).

### 34 f) (2S)-Benzyloxycarbonylamino-3-(2,4-dioxo-1,3,8-triaza-spiro[4.5]dec-3-yl)-propionsäure-methylester-hydrochlorid (34.7)

Eine Lösung von 4,7 g (9,3 mMol) an (34.6) in 100 ml 90 %-iger Trifluoressigsäure läßt man 45 min. bei Raumtemperatur rühren. Die Trifluoressigsäure wird im Vakuum entfernt und der Rückstand mit Wasser/Butanol/Essigsäure = 43/4,3/3,5 über Sephadex LH 20 chromatographiert. Die Produktfraktionen werden eingeengt und gefriergetrocknet. Nach Zugabe von 1 N HCI und erneuter Gefriertrocknung erhält man 1,99 g (53 %, ausgehend von (34.5)) an (34.7) als farblosen Feststoff.

### 34 g) 3-[8-(3-(1H-Benzimidazol-2-ylamino)-propionyl)-2,4-dioxo-1,3,8-triaza-spiro[4.5]dec-3-yl]-(2S)-benzyloxycarbonylamino-propionsäure-methylester (34.11)

Die Synthese von (34.11) erfolgt durch Kopplung von (34.7) mit (34.10) wie in Beispiel 15 bei der Synthese von (15.3) aus (15.2) beschrieben.

### 34 h) 3-[8-(3-(1H-Benzimidazol-2-ylamino)-propionyl)-2,4-dioxo-1,3,8-triaza-spiro[4.5]dec-3-yl]-(2S)-benzyloxycarbonylamino-propionsäure (34.12)

Die Synthese von (34.12) durch Esterspaltung von (34.11) erfolgt wie in Beispiel 3 bei der Synthese von (3.11) aus (3.10) beschrieben.

ES(+)-MS: 578 (M + H)⁺

### 34 i) Synthese von (34.10)

### 3-(1H-Benzoimidazol-2-ylamino)-propionsäure (34.10)

(34.10) wird gemäß nachfolgendem Reaktionsschema hergestellt:

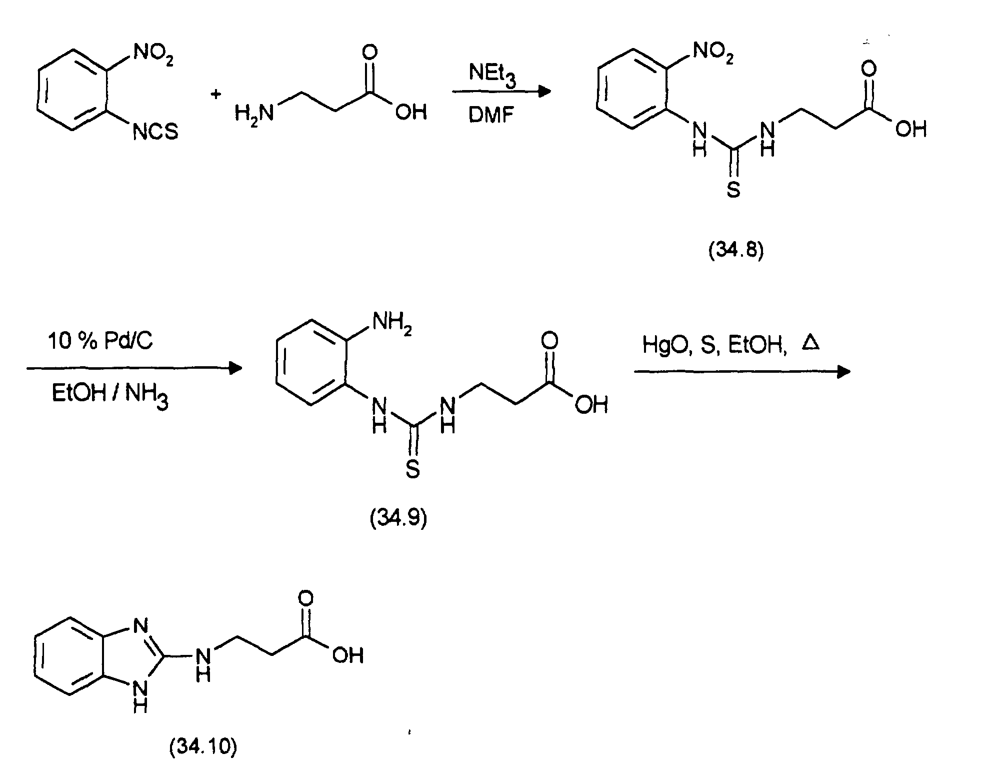

### a) 3-[3-(2-Nitro-phenyl)-thioharnstoff]-propionsäure (34.8)

Man gibt zu einer Lösung von 5 g (27,75 mMol) 2-Nitrophenyl-isothiocyanat und 2,5 g (27,75 mMol) b-Alanin in 80 ml DMF 3,8 ml (27,75 mMol) Triethylamin und rührt bis die Reaktion beendet ist. Das Lösungsmittel wird im Vakuum entfernt und der Rückstand zwischen Essigester und KHSO₄/K₂SO₄-Lösung verteilt. Die Phasen werden getrennt und die organische Phase über Natriumsulfat getrocknet. Nach Filtration wird das Lösungsmittel im Vakuum entfernt und der Rückstand über Kieselgel chromatographiert. Nach Einengen der Produktfraktionen erhält man 5,3 g (71 %) an (34.8).

### b) 3-[3-(2-Amino-phenyl)-thioharnstoff]-propionsäure (34.9)

5,3 g (19,7 mMol) an (34.8) in 410 ml mit Ammoniak gesättigtem EtOH werden über 6,44 g 10 % Pd/C 3 h bei Raumtemperatur hydriert. Der Katalysator wird abfiltriert, das Filtrat im Vakuum eingeengt und der Rückstand [1,9g (34.9), Rohprodukt] direkt zur Synthese von (34.10) eingesetzt.

### c) 3-(1H-Benzoimidazol-2-ylamino)-propionsäure (34.10)

Ein Gemisch aus (34.9), 3,44 g (14,79 mMol) Quecksilberoxid und 50 mg Schwefel in 40 ml Ethanol wird 5 h unter Rückfluß erhitzt. Nach Filtration wird das Filtrat eingeengt und der Rückstand mit 6N HCI 1 h zum Rückfluß erhitzt und anschließend gefriergetrocknet. Man erhält die Fraktion 1 an (34.9). Der Filterrückstand wird mehrmals mit Wasser ausgekocht und die vereinigten Wasser-Phasen werden gefriergetrocknet. Man erhält die Fraktion 2 an (34.9). Fraktion 1 und 2 werden vereinigt und mit Wasser/Butanol/Essigsäure = 43/4,3/3,5 über Sephadex LH 20 chromatographiert. Die Produktfraktionen werden eingeengt und gefriergetrocknet. Man erhält 340 mg (8 %, ausgehend von (34.8)) an (34.10) als farblosen Feststoff.

### Beispiel 35

### 3-[2-(4(E bzw. Z)-(3-(1H-Benzimidazol-2-ylamino)-propylidene)-2,5-dioxo-imidazolidin-1-yl)-acetylamino]-(2S)-benzyloxycarbonylamino-propionsäure (E-35.8) bzw. (Z-35.8)

Die Synthese erfolgte nach folgender Reaktionssequenz:

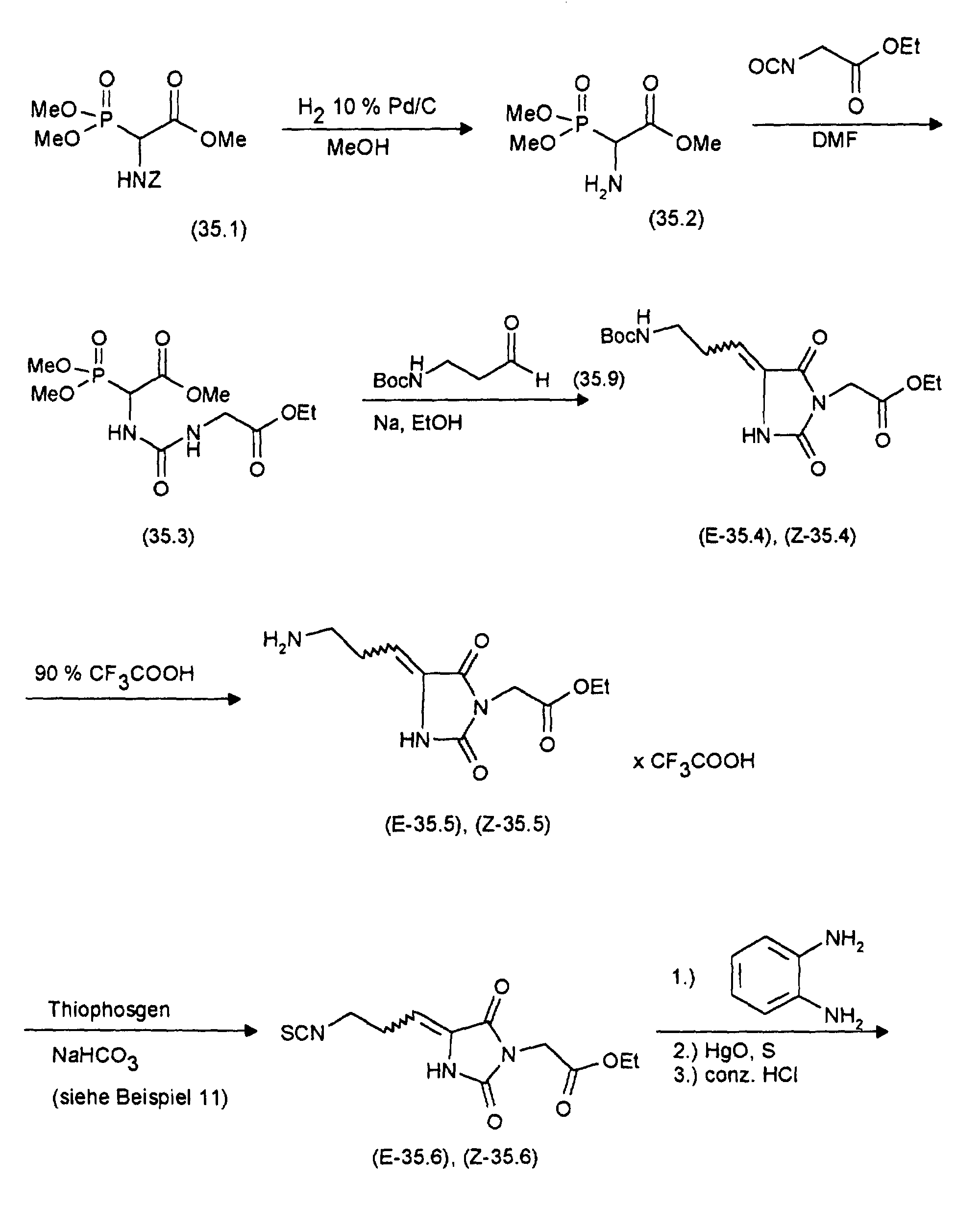

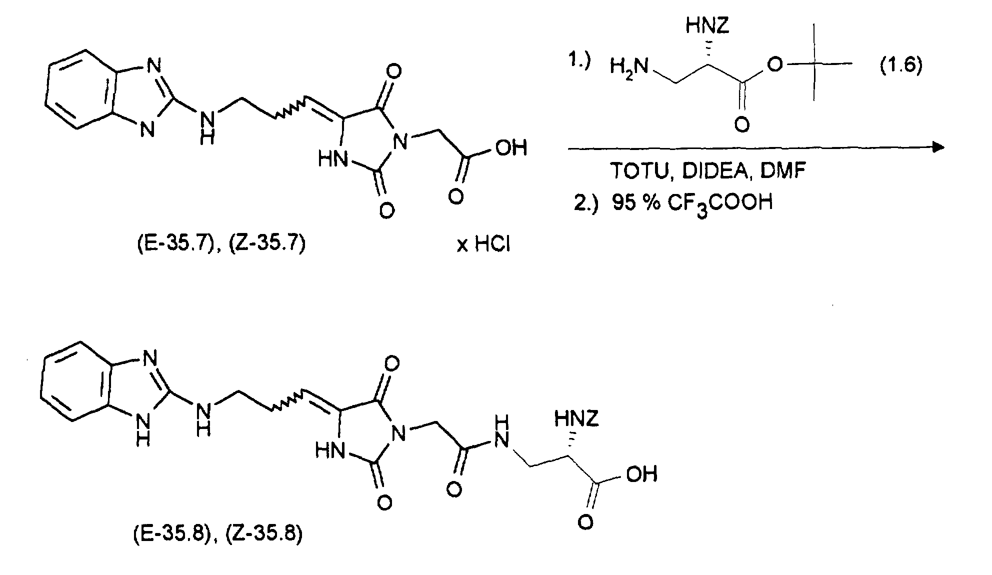

### 35 a) 3-Amino-3-(dimethoxy-phosphoryl)-prcpionsäure-methyl ester (35.2)

10 g (30,19 mMol) Z-Phosphonoglycin-trimethylester (35.1) in 300 ml Methanol werden über 10 % Pd/C hydriert. Nach 1 h wird der Katalysator abfiltriert und das Filtrat im Vakuum eingeengt.Man erhält 4,6 g an (35.2) als Rohprodukt, das direkt zur Synthese von (35.3) eingesetzt wird.

### 35b) 3-(Dimethoxy-phosphoryl)-3-(3-ethoxycarbonylmethyl-harnstoff)-propionsäure-methylester (35.3)

Man gibt zu einer Lösung von (35.2) in 20 ml DMF 2,62 ml (23,4 mMol) lsocyanato-essigsäureethylester. Nach 16 h bei Raumtemperatur werden weitere 0,262 ml (2,34 mMol) lsocyanato-essigsäureethylester zugegeben und das Reaktionsgemisch weitere 2 h bei Raumtemperatur gerührt. Das Lösungsmittel wird im Vakuum entfernt und der Rückstand zwischen Dichlormethan und KHSO₄/K₂SO₄-Lösung verteilt. Die Phasen werden getrennt und die organische Phase mit Wasser gewaschen. Nach Trocknen der organischen Phase über Natriumsulfat, Filtration und Einengen des Filtrats im Vakuum erhält man 6,2 g [63 %, ausgehend von (35.1)] an (35.3).

### 35 c) [4(E bzw. Z)-(3-tert-Butoxycarbonylamino-propylidene)-2,5-dioxo-imidazolidin-1-yl]-essigsäure-ethylester (E-35.4) bzw. (Z-35.4)

Man gibt zu einer Lösung von 451 mg (18,8 mMol) Natrium in absolutem EtOH eine Lösung von 5,8 g (17,84 mMol) an (35.3) in 30 ml absolutem EtOH. Zu dieser Lösung gibt man eine Lösung von (35.9), hergestellt aus b-Alanin analog O. P. Goel et al.,Org. Synth. 1988, 67, 69. Nach 3 h Rühren bei Raumtemperatur wird das Lösungsmittel im Vakuum entfernt und der Rückstand zwischen Wasser und Diethylether verteilt. Die Phasen werden getrennt und die organische Phase über Natriumsulfat getrocknet. Nach Filtration wird der Rückstand mit Dichlormethan/Methanol = 99/1 über Kieselgel chromatographiert. Man erhält 2,25 g an (E- oder Z-35.4) und 1,95 g an (Z-oder E-35.4). Gesamtausbeute: 4,2 g (69 %) an (35.4).

### 35 d) [4(E bzw. Z)-(3-Amino-propylidene)-2,5-dioxo-imidazolidin-1-yl]-essigsäure-ethylester-trifluoressigsäuresalz (E-35.5) bzw. (Z-35.5)

Eine Lösung von 2,25 g (6,6 mMol) an (Z- oder E-35.4) in 25 ml 90 %-iger Trifluoressigsäure wird 1 h bei Raumtemperatur gerührt. Die Trifluoressigsäure wird im Vakuum entfernt, der Rückstand mit Wasser verdünnt und gefriergetrocknet. Man erhält 2,2 g (94 %) an (Z- oder E-35.5). Analog erhält man aus 1,95 g (5,71 mMol) an (E-oder Z-35.4) 1,95 g (97 %) an (E-oder Z-35.5).

### 35e) [4-(E bzw. Z)-(3-lsothiocyanato-propylidene)-2,5-dioxo-imidazolidin-1-yll-essigsäure-ethyester (E-35.6) bzw. (Z-35.6)

Die Synthese von (E-35.6) bzw. (Z-35.6) aus (E-35.5) bzw. (Z-35.5) erfolgt wie in Beispiel 11 bei der Synthese von (11.2) aus (11.1) beschrieben

### 35f) [4(E bzw. Z)-(3-(1H-Benzoimidazol-2-ylamino)-propylidene)-2,5-dioxo-imidazolidin-1-yl]-essigsäure-hydrochlorid (E-35.7) bzw. (Z-35.7)

Die Synthese von (E-35.7) bzw. (Z-35.7) aus (E-35.6) bzw. (Z-35.6) erfolgt wie in Beispiel 15 bei der Synthese von (15.2) aus (11.2) beschrieben.

### 35g) 3-[2-(4(E bzw. Z)-(3-(1H-Benzimidazol-2-ylamino)-propylidene)-2,5-dioxo-imidazolidin-1-yl)-acetylamino]-(2S)-benzyloxycarbonylamino-propionsäure (E-35.8) bzw. (Z-35.8)

Die Synthese von (E-35.8) bzw. (Z-35.8) aus (E-35.7) bzw. (Z-35.7) erfolgt wie in Beispiel 15 bei der Synthese von (15.3) aus (15.2) beschrieben.

(E-35.8) bzw. (Z-35.8): ES(+)-MS: 550 (M + H)⁺

### Beispiel 36

### 3-[2-(4(Z bzw. E)-[3-(1 H-Benzimidazol-2-ylamino)-propylidene)-5-oxo-2-thioxo-imidazolidin-1-yl)-acetylamino]-(2S)-benzyloxycarbonylamino-propionsäure (Z-36) bzw (E-36)

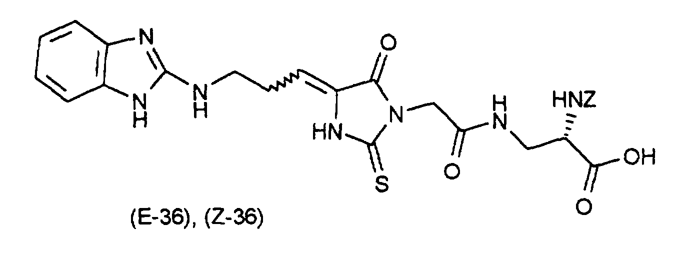

Die Synthese von (Z-36) bzw (E-36) erfolgt wie in Beispiel 35 beschrieben, wobei hier (35.2) mit lsothiocyanato-essigsäuremethylester anstelle von Isocyanato-essigsäureethylester umgesetzt wird. Die weitere Synthese erfolgt analog Beispiel 35.

(Z-36) bzw (E-36): ES(+)-MS: 566 (M + H)⁺

### Beispiel 37

### 3-(4-E/Z-(4-Guanidinocarbonylbenzyliden)-2,5-dioxo-imidazolidin-1-yl)-(2S)-N-benzyloxycarbonyl-alanin (E/Z-37.5)

Die Synthese erfolgte nach folgender Reaktionssequenz:

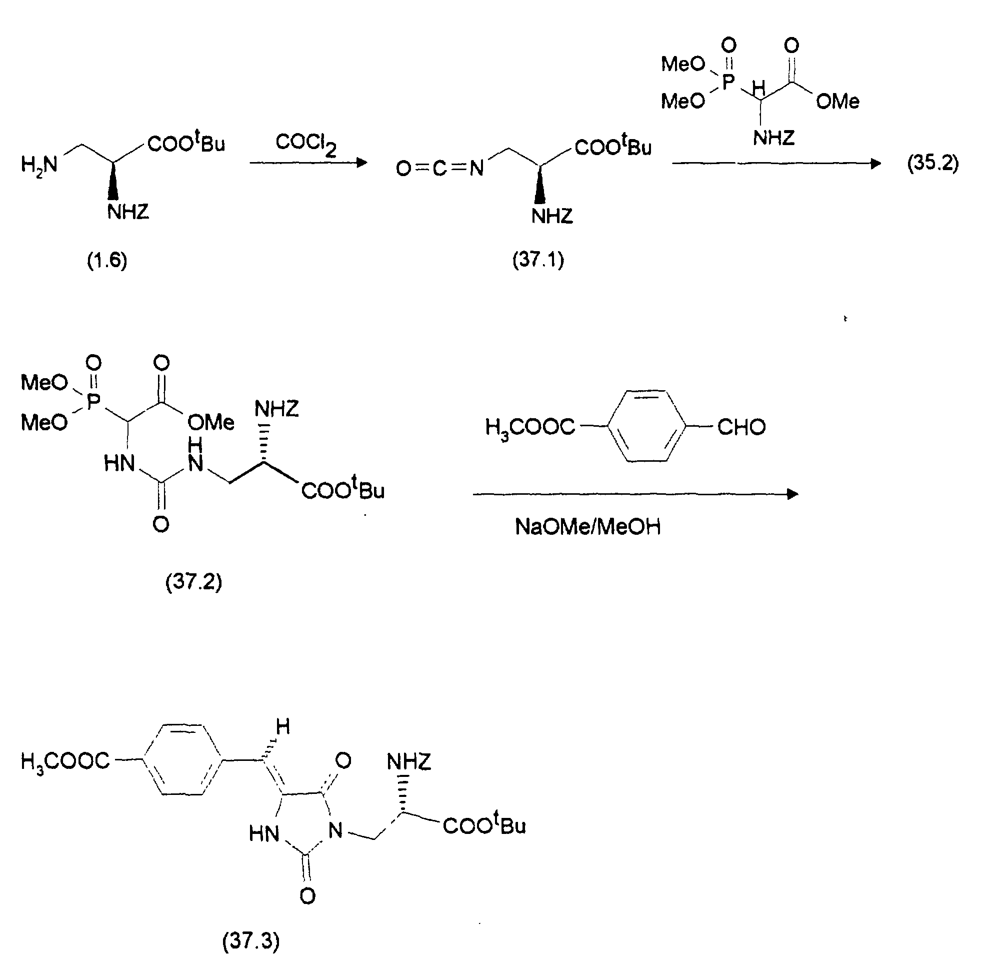

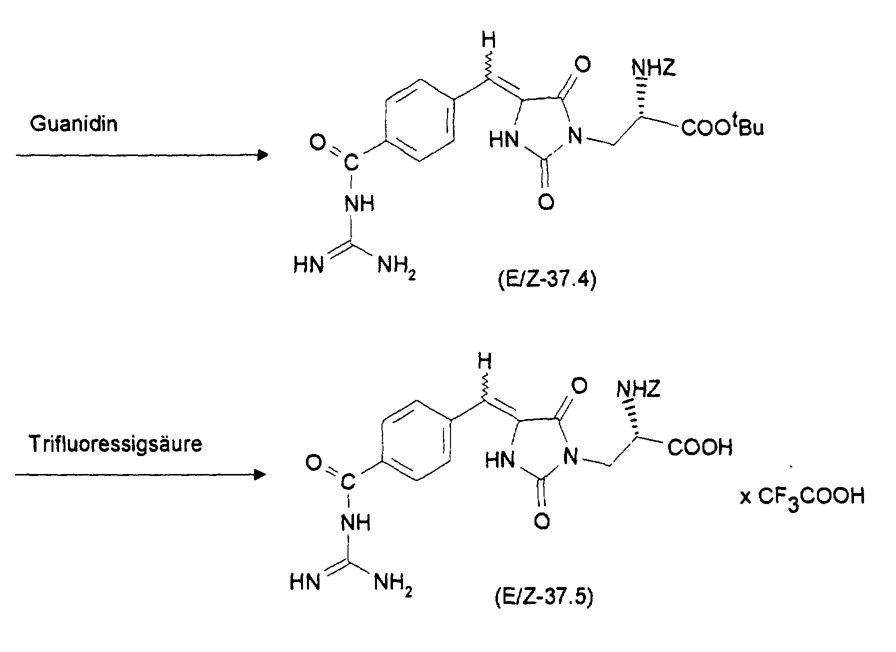

### 37a) 2-lsocyanato-Z-alanin-tert.-butylester (37.1)

Eine Lösung von 2,94 g (10 mmol) Z-Dap-O-t-Bu (1.6) in 80 ml Methylenchlorid wird mit 80 ml ges. Natriumbicarbonat-Lsg. im Eisbad unter heftigem Rühren auf ca. 0°C gekühlt. Nach Einstellen des Rührvorganges und Auftrennung der Phasen trägt man 10,4 ml einer 1,93 molaren Lösung von Phosgen in Toluol rasch in die organische Phase ein und läßt erneut unter wirksamen Rühren und externer Kühlung ausreagieren. Nach weiteren 10 min. werden die Phasen getrennt, die wässrige noch zweimal mit jeweils 40 ml methylenchlorid extrahiert. Die vereinigten organiscehn Extrakte werden mit wasserfreiem magnesiumsulfat getrocknet, filtriert und im Vakuum vom Lösungsmittel befreit. Man erhält ca. 3,0 g eines farblosen Öls, das ohne weitere Reinigung zur Synthese von (37.2) eingesetzt wurde.

### 37b) N'-(Dimethoxy-phosphoryl)-(methoxycarbonyl)-methyl-ureido-N-(2S)-benzyloxycarbonylamino-3-propionsäure (t)-butylester (37.2)

Unter Argonschutzgasatmosphäre löst man die aus 37a) erhaltene Menge an (37.1) in ca. 30 ml Dichlormethan und fügt unter Rühren bei Raumtemperatur 1,97 g (35.2), gelöst in ca. 50 ml Dichlormethan hinzu. Nach ca. 2 h ist die Reaktion beendet. Die Lösung wird zunächst mit 100 ml 2N Kaliumhydrogensulfatlösung, dann mit 100 ml Waser gewaschen, die organische Phase mit wasserfreiem Magnesiumsulfat getrocknet und filtriert. Nach Entfernen des Lösungsmittels im Vakuum verbleiben ca. 4,6 g reines (37.2), (89 %).

### 37c) E- bzw. Z-3-(4-(4-Methoxycarbonyl-benzyliden)-2,5-dioxo-imidazolidin-1-yl)-2-N-benzyloxycarbonylamino-propionsäure-(t)-butylester (37.3)

1,55 g (3 mmol) an (37.2) werden in ca. 8 ml Methanol gelöst und mit 0,6 ml (ca. 3,3 mmol) einer 30 %igen natriummethanolat-Lösung in Methanol versetzt. Dazu tropft man langsam (unter Argonschutzgas und unter Rühren bei Raumtemperatur) 8 ml einer methanolischen Lösung, die 0,59 g (3,6 mmol) 4-Formylbenzoesäuremethylester enthält. Nach ca. 1 h ist die Reaktion beendet. Die Lösung wird im Vakuum eingeengt, mit Essigester aufgenommen, mit Wasser gewaschen, getrocknet, filtriert und eingeengt. Man erhält 1,6 g eines E/Z-Gemisches von (37.3).
Durch Chromatographie mit Essigester/n-Heptan (2:1) als Eluens und Kieselgel als Trägermaterial sind die Diastereomeren auftrennbar: man erhält 0,85 g (Z-37.3) und 0,7 g (E-37.3)

### 37d) E- bzw. Z-3-(4-(4-Guanidinocarbonylbenzyliden)-2,5-dioxo-imidazolidin-1-yl)-(2S)-N-benzyloxycarbonylamino-propionsäure-(t)-butylester (E- bzw. Z-37.4)

Man löst 0,262 g (0,5 mmol) an (Z-37.3) in 5 ml abs. Tetrahydrofuran, gibt 0,148 g (2,5 mmol) Guanidin zu und erhitzt das Gemisch 20 h unter Rückfluß. Nach Entfernen des Lösungsmittels im Vakuum wird der Rückstand über Kieselgel mit Methanol/Methylenchlorid (1:10) chromatographiert. Man erhält 0,05 g (Z-37.4). Analog setzt man (E-37.3) um, wobei man als Lösungsmittel 1,2-Dimethoxyethan verwendet. Man erhält 0,12 g (E-37.4).

### 37e) E- bzw. Z-3-(4-(4-Guanidinocarbonylbenzyliden)-2,5-dioxo-imidazolidin-1-yl)-(2S)-N-benzyloxycarbonylamino-propionsäure-trifluoressigsäuresalz (E- bzw. Z-37.5)

0,12 g (E-37.4) werden in eiskalter 90 %iger Trifluoressigsäure (ca. 10 ml) gelöst und 1,5 h unter Argonschutzgas bei Raumtemperatur gerührt. Nach Entfernen des Lösungsmittels im Vakuum wird der Rückstand mit Methanol/Wasser über RP 18 chromatographiert. Die Produktfraktionen werden eingeengt und man erhält 0,06 g (E-37.5). Analog erhält man aus 0,05 g (Z-37.4) 0,02 g (Z-37.5).

### Beispiel 38

### 3-(4-E/Z-(4-(3,4,5,6-Tetrahydropyrimidin-2-yl)-aminocarbonylbenzyliden)-2,5-dioxo-imidazolidin-1-yl)-(2S)-N-benzyloxycarbonyl-alanin (E/Z-38.4)

Die Synthese erfolgt nach folgender Reaktionssequenz:

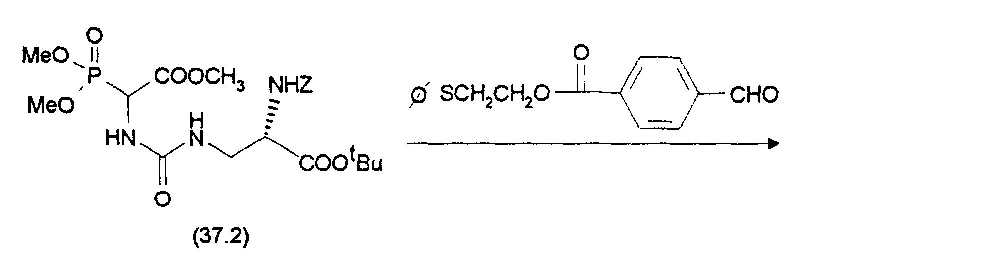

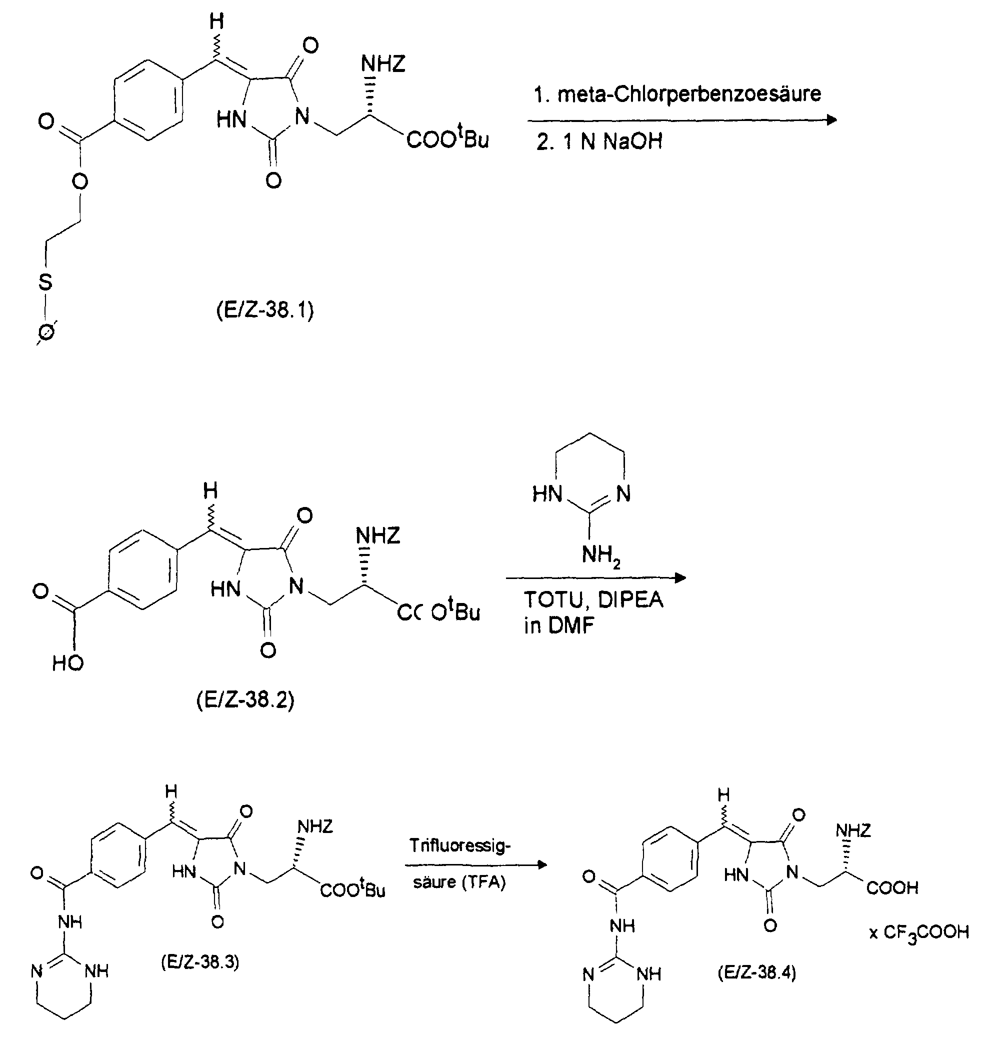

### 38a) E-bzw.Z-3-(4-((2-Phenylthio)-ethyloxycarbonylbenzyliden) 2,5-dioxo-imidazolidin-1-yl)-(2S)-N-benzyloxycarbonylamino-propionsäure-tert.-butylester (E-bzw. Z-38.1)

Analog der Umsetzung 37c) erhält man aus 0,259g (0,5mmol) an (37.2) 0,172g 4-Formylbenzoesäure-(2-phenylthio) ethylester (0,6mmol) 0,157g Z-38.1 und 0,153g (E-38.1) (Gesamtausbeute:ca. 91%), die durch Chromatographie einzeln isolierbar sind.

### 38b) E-bzw.Z-3-(4-(Carboxylbenzyliden) 2,5-dioxo-imidazolidin-1-yl)-2-N-benzyloxycarbonylamino-propionsäure-tert.-butylester (E-bzw. Z-38.2)

0,155g (0,24mmol) an (Z-38.1) werden in 5ml trockenem Methylenchlorid gelöst und mit 0,098g (0,4mmol) 65%iger meta-Chlorperbenzoesäure versetzt. Nach 1,5h ist die Reaktion beendet. Man wäscht die Methylenchloridlösung mit Natriumbisulfit (ca. 10%ig) und anschliessend mit Wasser. Nach Trocknen über Magnesiumsulfat, Filtration und Entfernen des Lösungsmittels verbleiben 0,15g des Phenylsulfonylethylesters, der ohne weitere Behandlung in ein Gemisch von 8ml Dioxan, 1,4ml Methanol sowie 0,19ml 1N NaOH eingetragen wird. Nach 16h werden die Lösungsmittel im Vak. entfernt und der Rückstand mit Methylenchlorid/Methanol (95:5) über Kieselgel chromatographiert. Ausbeute: 0,033g (Z-38.2). Analog werden aus 0,157g (E-38.1) (0,24mmol) 0,051g (E-38.2) erhalten.

### 38c) 3-(4-E/Z-(4-(3,4,5,6-Tetrahdropyrimidin-2-yl)-aminocarbonylbenzyliden)-2,5-dioxo-imidazolidin-1-yl)-(2S)-N-benzyloxycarbonylamino-propionsäure-tert.-butylester (E/Z-38.3)

Analog der Vorschrift zur Synthese von 11.6 (Vorschrift 11f) werden 0,05g (E-38.2) (0,1 mmol) und 0,01g (0,1mmol) 2-Amino-3,4,5,6-tetrahydropyrimidin mit der äquivalenten Menge TOTU (0,033g) in Dimethylformamid in Gegenwart von Diisopropylethylamin zur Reaktion gebracht. Die übliche Aufarbeitung liefert 0,06g (E-38.3), das ohne weitere Reinigung umgesetzt wird. Auf die gleiche Weise erhält man aus 0,03 g an (Z-38.2) 0,025g des Rohprodukts von (Z-38.3).

### 38d) 3-(4-E/Z-(4-(3,4,5,6-Tetrahdropyrimidin-2-yl)-aminocarbonylbenzyliden)-2,5-dioxo-imidazolidin-1-yl)-(2S)-N-benzyloxycarbonylamino-propionsäure (E/Z-38.4)

0,055g (E-38.3) werden in 2ml 90%iger Trifluoressigsäure 2h unter Eiskühlung gerührt. Entfernen des Lösungsmittels im Vak. und Gefriertrocknung des Rückstandes liefert 0,02g an (E-38.4). Analog erhält man aus 0,02g an (Z-38.3) 0,012g an (Z-38.4).

Die Hemmung der Knochenresorption durch die erfindungsgemäßen Verbindungen kann beispielsweise mit Hilfe eines Osteoclasten-Resorptions-Tests ("PIT ASSAY") beispielsweise analog WO 95/32710 bestimmt werden. Die Testmethoden, nach denen die antagonistische Wirkung der erfindungsgemäßen Verbindungen auf den Vitronectinrezeptor αᵥβ₃ bestimmt werden können, sind nachfolgend beschrieben.

### Testmethode 1:

### Inhibierung der Bindung von humanem Vitronectin (Vn) an humanen Vitronectinrezeptor (VnR) αᵥβ₃ : ELISA-Test.

### (Testmethode 1 wird bei der Auflistung der Testergebnisse mit Vn/VnR abgekürzt)

1. Reinigung von humanem Vitronectin
   Humanes Vitronectin wird aus menschlichem Plasma isoliert und durch Affinitätschromatographie nach der Methode von Yatohyo et al., Cell Structure and Function, 1988, 23, 281-292 gereinigt.
2. Reinigung von humanem Vitronectinrezeptor (αᵥβ₃)
   Humaner Vitronectinrezeptor wird aus der menschlichen Plazenta nach der Methode von Pytela et al., Methods Enzymol. 1987, 144, 475 gewonnen. Humaner Vitronectinrezeptor αᵥβ₃ kann auch aus einigen Zellinien (z.B. aus 293 Zellen, einer humanen embryonalen Nierenzellinie), die mit DNA Sequenzen für beide Untereinheiten αᵥ und β₃ des Vitronectinrezeptors co-transfiziert sind, gewonnen werden. Die Untereinheiten werden mit Octylglycosid extrahiert und anschließend über Concanavalin A, Heparin-Sepharose und S-300 chromatographiert.
3. Monoklonale Antikörper
   Murine monoklonale Antikörper, spezifisch für die β₃ Untereinheit des Vitronectinrezeptors, werden nach der Methode von Newman et al., Blood, 1985, 227-232, oder nach einem ähnlichen Verfahren hergestellt. Das Kaninchen Fab 2 anti-Maus Fc Konjugat an Meerrettich Peroxidase (anti-Maus Fc HRP) wurde von Pel Freeze (Katalog Nr. 715 305-1) bezogen.
4. ELISA Test
   Nunc Maxisorb 96 well Mikrotiter Platten werden mit einer Lösung von humanem Vitronectin (0,002 mg/ml, 0,05 ml/well) in PBS (Phosphat gepufferte Kochsalzlösung) über Nacht bei 4°C belegt. Die Platten werden zweimal mit PBS/0,05 % Tween 20 gewaschen und durch Inkubieren (60 min) mit Rinderserum Albumin (BSA, 0,5 %, RIA Güteklasse oder besser) in Tris-HCI (50 mM), NaCI (100 mM), MgCl₂ (1 mM), CaCl₂ (1 mM), MnCl₂ (1 mM), pH 7, geblockt. Man stellt Lösungen von bekannten Inhibitoren und von den Testsubstanzen in Konzentration von 2x10⁻¹² - 2x10⁻⁶ Mol/l in Assay Puffer [BSA (0,5 %, RIA-Güteklasse oder besser) in Tris-HCI (50 mM/l), NaCl (100 mM), MgCl₂ (1 mM), CaCl₂ (1 mM), MnCl₂ (1 mM), pH 7] her.
   Die geblockten Platten werden entleert, und jeweils 0,025 ml dieser Lösung, die eine definierte Konzentration (2x10⁻¹² bis 2x10⁻⁶) entweder an einem bekannten Inhibitor oder an einer Testsubstanz enthält, in jedes well gegeben. 0,025 ml einer Lösung des Vitronectinrezeptors im Testpuffer (0,03 mg/ml) werden in jedes well der Platte pipettiert und die Platte wird auf einem Schüttler 60-180 min bei Raumtemperatur inkubiert. In der Zwischenzeit wird eine Lösung (6 ml/Platte) eines für die β₃-Untereinheit des Vitronectinrezeptors spezifischen murinen monoklonalen Antikörpers im Assay Puffer (0,0015 mg/ml) hergestellt. Zu dieser Lösung gibt man einen zweiten Kaninchen Antikörper (0,001 ml Stammlösung/6 ml der murinen monoklonalen anti-β₃-Antikörper Lösung), der ein anti-Maus Fc HRP Antikörper Konjugat darstellt, und dieses Gemisch aus murinem anti-β₃ Antikörper und Kaninchen anti-Maus Fc HRP Antikörper Konjugat läßt man während der Zeit der Rezeptor-Inhibitor Inkubation inkubieren.
   Die Testplatten werden 4 mal mit PBS-Lösung, die 0,05 % Tween-20 enthält, gewaschen und man pipettiert jeweils 0,05 ml/well der Antikörpermischung in jedes well der Platte und inkubiert 60-180 min.. Die Platte wird 4 mal mit PBS/0,05 % Tween-20 gewaschen und anschließend mit 0,05 ml/well einer PBS-Lösung, die 0,67 mg/ml o-Phenylendiamin und 0,012 % H₂O₂ enthält, entwickelt. Alternativ hierzu kann o-Phenylendiamin in einem Puffer (pH 5) eingesetzt werden, der Na₃PO₄ (50 mM) und Zitronensäure (0,22 mM) enthält. Die Farbentwicklung wird mit 1N H₂SO₄ (0,05 ml/well) gestoppt.
   Die Absorption für jedes well wird bei 492-405 nm gemessen und die Daten werden nach Standardmethoden ausgewertet.

### Testmethode 2:

### Inhibierung der Bindung von Kistrin an humanen Vitronectinrezeptor (VnR) αᵥβ₃: ELISA-Test

### (Testmethode 2 wird bei der Auflistung der Testergebnisse mit Kistrin/VnR abgekürzt)

1. Reinigung von Kistrin
   Kistrin wird nach den Methoden von Dennis et al., wie in Proc. Natl. Acad. Sci. USA 1989, 87, 2471-2475 und PROTEINS: Structure, Function and Genetics 1993, 15, 312-321, beschrieben, gereinigt.
2. Reinigung von humanem Vitronectinrezeptor (αᵥβ₃)
   siehe Testmethode 1.
3. Monoklonale Antikörper
   siehe Testmethode 1.
4. ELISA-Test
   Die Fähigkeit von Substanzen die Bindung von Kistrin an den Vitronectinrezeptor zu inhibieren, kann mit einem ELISA-Test ermittelt werden. Zu diesem Zweck werden Nunc 96 well Mikrotiterplatten mit einer Lösung von Kistrin (0,002 mg/ml) nach der Methode von Dennis et al., wie in PROTEINS: Structure, Function and Genetics 1993, 15, 312-321, beschrieben, belegt. Die weitere experimentelle Durchführung des ELISA-Test erfolgt wie bei Testmethode 1, Punkt 4, beschrieben.

| Testergebnisse: | | |
|---|---|---|
| Beispiel | Vn/VnR IC₅₀ (µM) | Kistrin/VnR IC₅₀ (µM) |
| 1 | 0,008 | 0,02 |
| 3 | | 0,36 |
| 4 | | 1,66 |
| 5 | | 0,04 |
| 7 | | 0,58 |
| 8 | | 0,13 |
| 18 | | 0,81 |
| 19 | | 0,02 |

## Patentansprüche

1. Verbindung der Formel I, 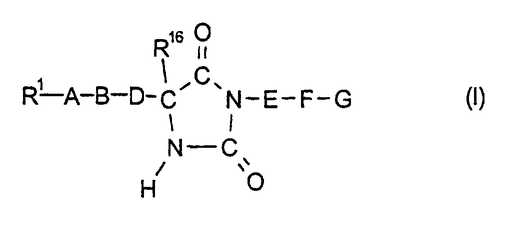 worin bedeuten:
A eine direkte Bindung, (C₁-C₄)-Alkandiyl, -NR²-N=CR²-, -NR²-C(O)-NR²-, -NR²-C(O)O-, -NR²-S(O)ₙ-, -NR²-S(O)ₙ-NR²-, -NR²-CO-, -NR²- oder -N=CR², die jeweils durch NH und/oder einfach oder zweifach durch (C₁-C₄)-Alkandiyl substituiert sein können;
B eine direkte Bindung, (C₁-C₄)-Alkandiyl, Phenylen, einen zweiwertigen Rest des Pyridins, Thiophens oder Furans, Cyclohexandiyl, -C≡C-, -CR²=CR³-, -C(O)-NR²- oder -NR²-C(O)-, die jeweils einfach oder zweifach durch (C₁-C₄)-Alkandiyl substituiert sein können;
D eine direkte Bindung, (C₁-C₄)-Alkandiyl, Phenylen, -O-, -NR²-, -NR²-CO-, -NR²-C(O)-NR²-, -R²N-S(O)₂-NR²-, -NR²-S(O)₂-, -NR²-S(O)-, -N=CR²- oder
-R²C=N-, die jeweils einfach oder zweifach durch (C₁-C₄)-Alkandiyl substituiert sein können;
E eine direkte Bindung oder (C₁-C₄)-Alkandiyl;
F eine direkte Bindung, (C₁-C₆)-Alkandiyl, -O-, -CO-NR²-, -NR²-CO-, -NR²-C(O)-NR²-, -S(O)₂-NR²-, -NR²-S(O)₂-, -CR² = CR³-, -C≡C-,-N=CR²- oder -R²C = N-, die jeweils einfach oder zweifach durch (C₁-C₄)-Alkandiyl substituiert sein können;
G 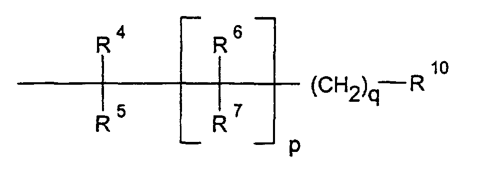
R¹ R²R³N-C(=NR²)-, 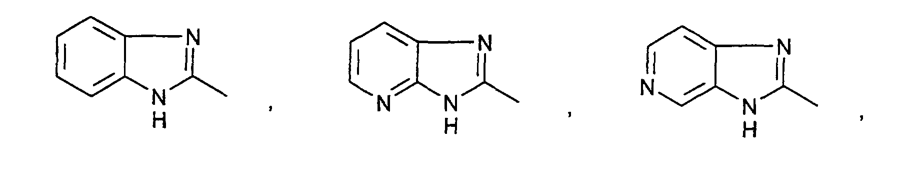 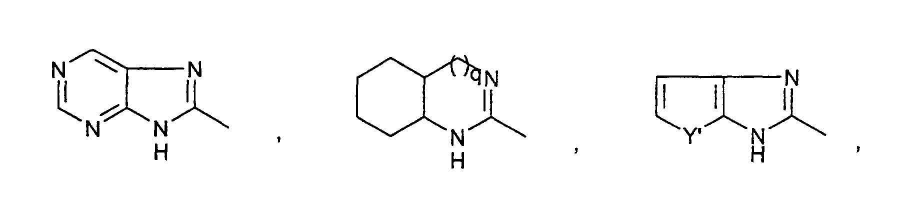 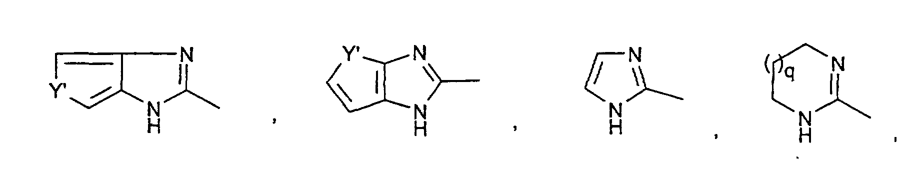 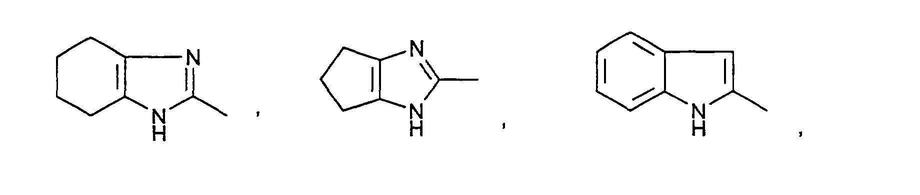 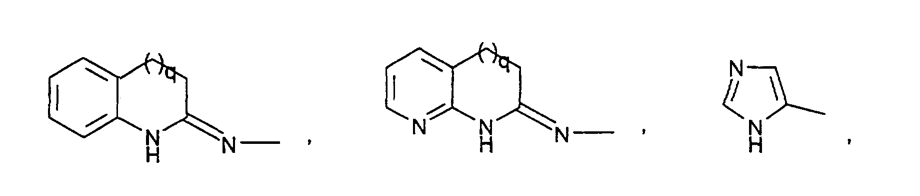 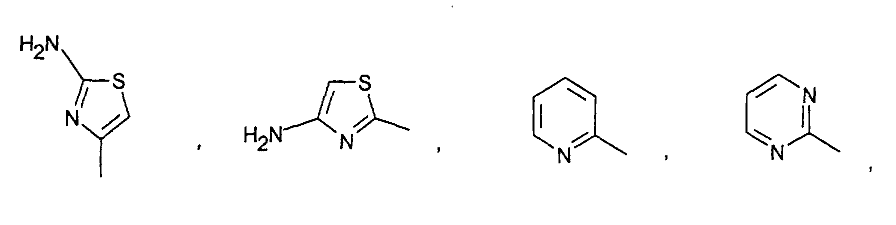 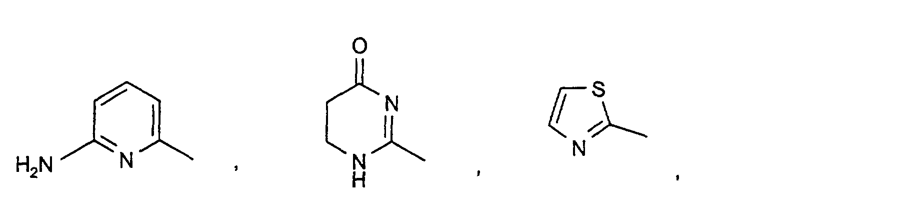 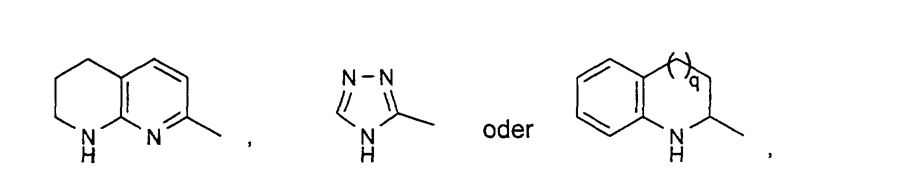 wobei Y' NH, O oder S bedeutet.
R², R³ unabhängig voneinander H, (C₁-C₆)-Alkyl, Trifluormethyl, Pentafluorethyl, (C₅-C₆)-Cycloalkyl, (C₅-C₆)-Cycloalkyl-(C₁-C₂)-Alkyl, Phenyl, Benzyl, H₂N, R⁸OR⁹, R⁸-(C₅-C₁₀)-ArylR⁹, R⁸NHR⁹, R⁸R⁸NR⁹, R⁸NHC(O)R⁹, H₂N-C(=NH) oder H₂N-C(= NH)-NH-;
R⁴, R⁵, R⁶, R⁷ unabhängig voneinander H, Fluor, OH, (C₁-C₆)-Alkyl, (C₁₀-C₁₂)-Cycloalkyl, (C₁₀-C₁₂)-Cycloalkyl-(C₁-C₆)-alkyl, R⁸OR⁹, R⁸-(C₅-C₁₀)-ArylR⁹, R⁸R⁸NR⁹, R⁸NHC(O)OR⁹, R⁸S(O)ₙNHR⁹, R⁸OC(O)NHR⁹ oder R⁸C(O)NHR⁹;
R⁸ H, (C₁-C₆)-Alkyl, (C₁₀-C₁₂)-Cycloalkyl, (C₁₀-C₁₂)-Cycloalkyl-(C₁-C₂)-Alkyl, (C₅-C₁₀)-Aryl oder (C₅-C₁₀)-Aryl-(C₁-C₂)-alkyl;
R⁹ eine direkte Bindung oder (C₁-C₆)-Alkandiyl;
R¹⁰ C(O)R¹¹;
R¹¹ OH, (C₁-C₆)-Alkoxy, Phenoxy, Benzyloxy, (C₁-C₄)-Alkylcarbonyloxy-(C₁-C₄)-alkoxy, NH₂, Mono- oder Di-(C₁-C₆-alkyl)-amino;
R¹⁶ H, (C₁-C₄)-Alkyl, Trifluormethyl, Pentafluorethyl, (C₅-C₆)-Cycloalkyl, (C₅-C₆)-Cycloalkyl-(C₁-C₂)-alkyl, Phenyl oder Benzyl;
n 1 oder 2; und
p, q unabhängig voneinander 0 oder 1;
und ihre physiologisch verträgliche Salze, wobei Verbindungen ausgenommen sind, in denen R¹-A-B-D-C(R¹⁶) gleich R¹-K-C(R¹⁶) ist, wobei hier
R¹ für X-NH-C(=NH)-(CH₂)ₚ, X¹-NH-(CH₂)ₚ oder 4-Imidazolyl-CH₂- steht, wobei p für eine ganze Zahl von 0 bis 3 stehen kann,
X Wasserstoff, (C₁-C₆)-Alkyl, Hydroxy, (C₁-C₆)-Alkoxy, (C₆-C₁₄)-Aryl-(C₁-C₆)-alkoxy oder Amino bedeutet, wobei die Arylgruppen in X reine, gegebenenfalls einfach oder mehrfach substituierte, Carbocyclen darstellen,
X¹ (C₄-C₁₄)-Aryl-(C₁-C₆)-alkoxy oder R'-NH-C(=N-R") bedeutet, wobei R' und R" unabhängig voneinander die Bedeutungen von X haben und wobei die Arylgruppen in X¹ reine, gegebenenfalls einfach oder mehrfach substituierte, Carbocyclen darstellen,
K (C₁-C₆)-Alkandiyl, Cyclohexandiyl, Phenylen, Phenylen-(C₁-C₆)-Alkandiyl, (C₁-C₆)-Alkandiyl-Phenylen, Phenylen-(C₂-C₆)-Alkendiyl oder einen zweiwertigen Rest eines 6-gliedrigen gesättigten oder ungesättigten Ringes, der 1 Stickstoffatom enthalten und einfach oder zweifach durch (C₁-C₆)-Alkyl oder doppelt gebundenen Sauerstoff oder Schwefel substituiert sein kann, bedeutet.

2. Verbindung der Formel I gemäß Anspruch 1, worin F für -CO-NR²- steht, und ihre physiologisch verträglichen Salze.

3. Verbindung der Formel I gemäß den Ansprüchen 1 und/oder 2, worin G für 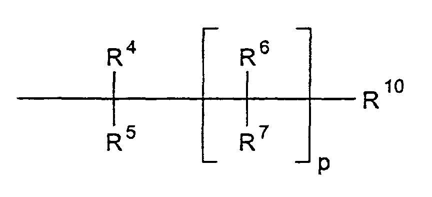 steht, und ihre physiologisch verträglichen Salze.

4. Verfahren zur Herstellung einer Verbindung der Formel I gemäß den Ansprüchen 1 bis 3, worin F für -C(O)-NR²- steht, **dadurch gekennzeichnet, daß** man eine Verbindung der Formel II 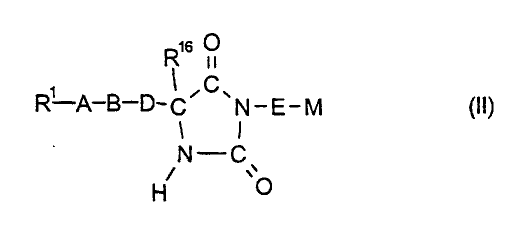 mit einer Verbindung der Formel HNR²-G kondensiert, wobei A, B, D, R¹, R¹⁶, E und G die in den Ansprüchen 1 bis 3 angegebenen Bedeutungen haben oder auch funktionelle Gruppen in geschützter Form vorliegen können, und M für Hydroxycarbonyl, (C₁-C₆)-Alkoxycarbonyl oder ein aktiviertes Carbonsäurederivat steht.

5. Verbindung der Formel I gemäß den Ansprüchen 1 bis 3 oder ein physiologisch verträgliches Salz davon zur Verwendung als Heilmittel.

6. Verbindung der Formel I gemäß den Ansprüchen 1 bis 3 oder ein physiologisch verträgliches Salz davon als Inhibitor der Knochenresorption durch Osteoclasten, als Inhibitor von Tumorwachstum oder Tumormetastasierung, als Entzündungshemmer, zur Behandlung oder Prophylaxe von cardiovaskulären Erkrankungen, zur Behandlung oder Prophylaxe von Nephropatien oder Retinopathien oder als Vitronectinrezeptor-Antagonist zur Behandlung und Prophylaxe von Krankheiten, die auf der Wechselwirkung zwischen Vitronectinrezeptoren und deren Liganden bei Zell-Zell- oder Zell-Matrix-Interaktionsprozessen beruhen.

7. Pharmazeutische Zubereitung, enthaltend mindestens eine Verbindung der Formel I gemäß den Ansprüchen 1 bis 3 oder ein physiologisch verträgliches Salz davon neben pharmazeutisch einwandfreien Trägerstoffen und/oder Zusatzstoffen.

## Claims

1. , A compound of the formula I: 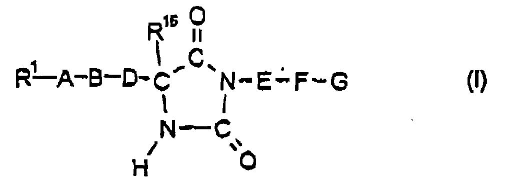 in which:
A is a direct linkage, (C₁-C₄)-alkanediyl, -NR²-N=CR²-, -NR²-C(O)-NR²-, -NR²-C(O)O-, -NR²-S(O)ₙ-, -NR²-S(O)ₙ-NR²-, -NR²-CO-,-NR²- or -N=CR², which in each case can be substituted by NH and/or be substituted, once or twice, by (C₁-C₄)-alkanediyl;
B is a direct linkage, (C₁-C₄)-alkanediyl, phenylene, a divalent radical of pyridine, thiophene or cyclohexanediyl, cyclohexanediyl, -C≡C-, -CR²=CR³-, -C(O)-NR²- or -NR²-C(O)-, which in each case can be substituted, once or twice, by (C₁-C₄)-alkanediyl;
D is a direct linkage, (C₁-C₄)-alkanediyl, phenylene, -O-, -NR²-, -NR²-CO-, -NR²-C(O)-NR²-, -R²N-S(O)₂-NR²-, -NR²-S(O)₂-, -NR²-S(O)-, -N=CR²- or -R²C=N-, which in each case can be substituted, once or twice, by (C₁-C₄)-alkanediyl;
E is a direct linkage or (C₁-C₄)-alkanediyl;
F is a direct linkage, (C₁-C₆)-alkanediyl, -O-, -CO-NR²-, -NR²-CO-, -NR²-C(O)-NR²-, -S(O)₂-NR²-, -NR²-S(O)₂-, -CR²=CR³-, -C=C-, -N=CR²- or -R²C=N-, which in each case can be substituted, once or twice, by (C₁-C₄)-alkanediyl;
G is 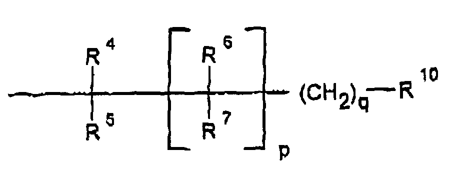
R¹ is R²R³N-C(=NR²)-, 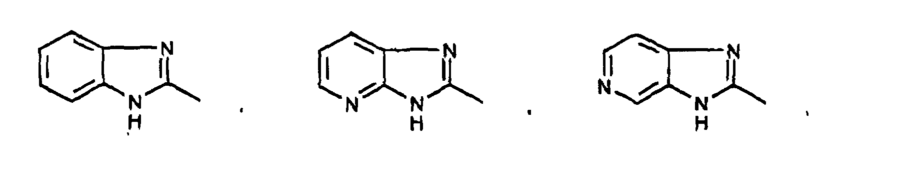 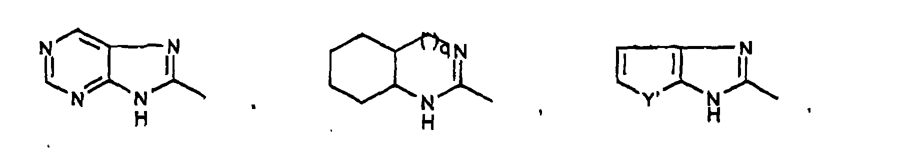 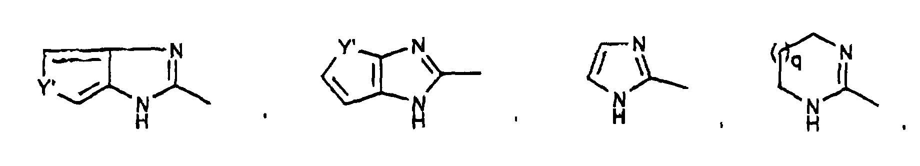 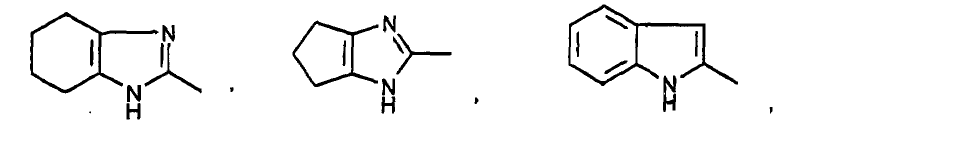 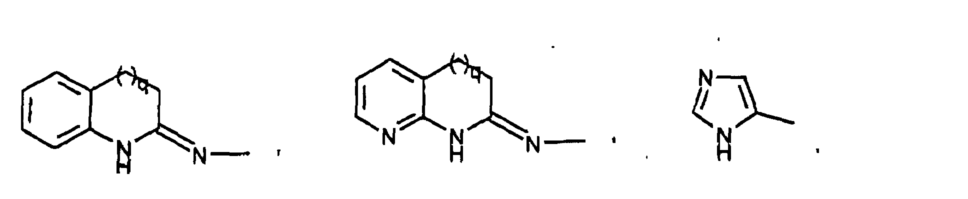 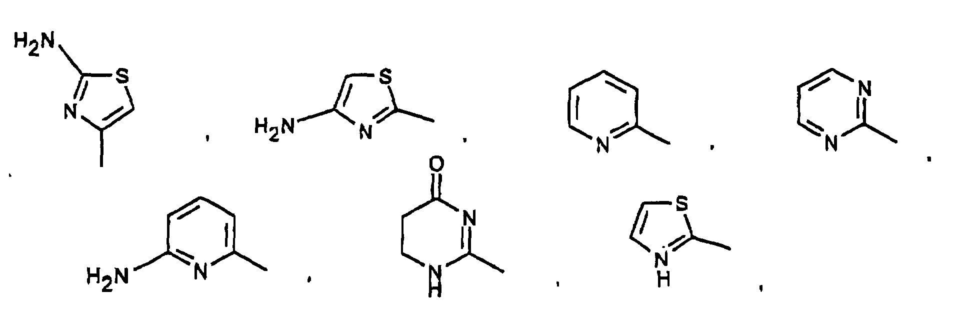 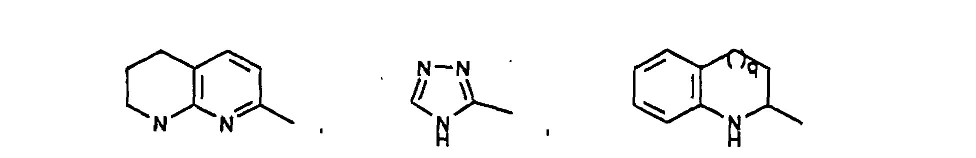 with Y' being NH, O or S.
R² and R³ are, independently of each other, H, (C₁-C₆)-alkyl, trifluoromethyl, pentafluoroethyl, (C₅-C₆)-cycloalkyl, (C₅-C₆)-cycloalkyl-(C₁-C₂)-alkyl, phenyl, benzyl, H₂N, R⁸OR⁹, R⁸-(C₅-C₁₀)-aryl-R⁹, R⁸NHR⁹, R⁸R⁸NR⁹, R⁸NHC(O)R⁹, H₂N-C(=NH) or H₂N-C(=NH)-NH-;
R⁴, R⁵, R⁶ and R⁷ are, independently of each other, H, fluorine, OH, (C₁-C₆)-alkyl, (C₁₀-C₁₂)-cycloalkyl, (C₁₀-C₁₂)-cycloalkyl-(C₁-C₆)-alkyl, or R⁸OR⁹, R⁸-(C₅-C₁₀)-aryl-R⁹, R⁸R⁸NR⁹, R⁸NHC(O)OR⁹, R⁸S(O)ₙNHR⁹, R⁸OC(O)NHR⁹ or R⁸C(O)NHR⁹;
R⁸ is H, (C₁-C₆)-alkyl, (C₁₀-C₁₂)-cycloalkyl, (C₁₀-C₁₂)-cycloalkyl-(C₁-C₂)-alkyl, (C₅-C₁₀)-aryl or (C₅-C₁₀)-aryl-(C₁-C₂)-alkyl;
R⁹ is a direct linkage or (C₁-C₆)-alkanediyl;
R¹⁰ is C(O)R¹¹;
R¹¹ is OH, (C₁-C₆)-alkoxy, phenoxy, benzyloxy, (C₁-C₄)-alkylcarbonyloxy-(C₁-C₄)-alkoxy, NH₂, mono- or di(C₁-C₆-alkyl)amino;
R¹⁶ is H, (C₁-C₄)-alkyl, trifluoromethyl, pentafluoroethyl, (C₅-C₆)-cycloalkyl, (C₅-C₆)-cycloalkyl-(C₁-C₂)-alkyl, phenyl or benzyl;
n is 1 or 2; and
p and q are, independently of each other, 0 or 1,
and the physiologically tolerated salts thereof,
with compounds being excepted in which R¹-A-B-D-C(R¹⁶) is R'-K-C(R¹⁶), where, in this case,
R¹ is X-NH-C(=NH)-(CH₂)p, X¹-NH-(CH₂)ₚ or 4-imidazolyl-CH₂-, with it being possible for p to be an integer from 0 to 3,
X is hydrogen, (C₁-C₆)-alkyl, hydroxyl, (C₁-C₆)-alkoxy, (C₆-C₁₄)-aryl-(C₁-C₆)-alkoxy or amino, with the aryl groups in X being pure carbocycles which are optionally substituted once or more than once.
X¹ is (C₄-C₁₄)-aryl-(C₁-C₆)-alkoxy or R'-NH-C(=N-R"), where R' and R" have, independently of each other, the meanings of X and where the aryl groups in X, are pure carbocycles which are optionally substituted once or more than once,
K is (C₁-C₆)-alkanediyl, cyclohexanediyl, phenylene, phenylene-(C₁-C₆)-alkanediyl, (C₁-C₆)-alkanediylphenylene, phenylene-(C₂-C₆)-alkenediyl or a divalent radical of a 6-membered, saturated or unsaturated ring which contains 1 nitrogen atom and can be substituted, once or twice, by (C₁-C₆)-alkyl or doubly bonded oxygen or sulfur.

2. A compound of the formula I as claimed in claim 1 in which F is -CO-NR²-, and its physiologically tolerated salts.

3. A compound of the formula I as claimed in claims 1 and/or 2, in which G is 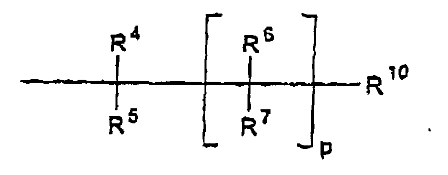 and its physiologically tolerated salts.

4. A process for preparing a compound of the formula I as claimed in claims 1 to 3, in which F is -C(O)-NR²-, which comprises condensing a compound of the formula II 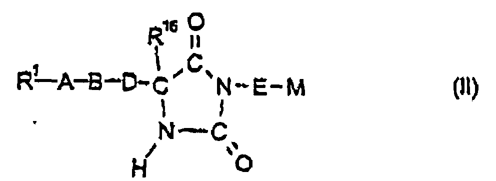 with a compound of the formula HNR²-G, where A, B, D, R¹, R¹⁶, E and G have the meanings given in claims 1 to 3 or else functional groups can be present in protected form, and M is hydroxycarbonyl, (C₁-C₆)-alkoxycarbonyl or an activated carboxylic acid derivative.

5. A compound of the formula I as claimed in claims 1 to 3 or a physiologically tolerated salt thereof as a medicament.

6. A compound of the formula I as claimed in claims 1 to 3 or a physiologically tolerated salt thereof as an inhibitor of bone reabsorption by osteoclasts, as an inhibitor of tumor growth and tumor metastasis, as an antiinflammatory agent, for the treatment or prophylaxis of cardiovascular diseases, for the treatment or prophylaxis of nephropathies and retinopathies and as a vitronectin receptor antagonist for the treatment and prophylaxis of diseases which are based on the interaction between vitronectin receptors and their ligands in cell-cell or cell-matrix interaction processes.

7. A pharmaceutical preparation which comprises at least one compound of the formula I as claimed in claims 1 to 3, or a physiologically tolerated salt thereof, in addition to pharmaceutically unobjectionable carrier substances and/or additives.

## Revendications

1. Composé de formule I, 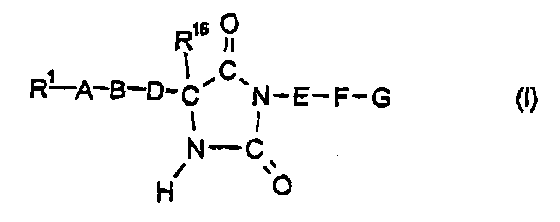 dans laquelle
A représente une liaison directe, un groupe alcanediyle en C₁-C₄, -NR²-N=CR²-, -NR²-C(O)-NR²-, -NR²-C(O)O-, -NR²-S(O)ₙ-, -NR²-S(O)ₙ-NR²-, -NR²-CO-, -NR²- ou -N=CR², qui peuvent être substitués chacun par NH et/ou une ou plusieurs fois par un groupe alcanediyle en C₁-C₄ ;
B représente une liaison directe, un groupe alcanediyle en C₁-C₄, phénylène, un reste bivalent de la pyridine, du thiophène ou du furanne, un groupe cyclohexanediyle, -C≡C-, -CR²=CR³-, -C(O)-NR²- ou -NR²-C(O)-, qui peuvent être substitués chacun une ou plusieurs fois par un groupe alcanediyle en C₁-C₄ ;
D représente une liaison directe, un groupe alcanediyle en C₁-C₄, phénylène, -O-, -NR²-, -NR²-CO-, -NR²-C(O)-NR²-; -R²N-S(O)₂-NR²-, -NR²-S(O)₂-, -NR²-S(O)-, -N=CR²- ou -R²C=N-, qui peuvent être substitués chacun une ou plusieurs fois par un groupe alcanediyle en C₁-C₄ ;
E représente une liaison directe ou un groupe alcanediyle en C₁-C₄ ;
F représente une liaison directe, un groupe alcanediyle en C₁-C₆, -O-, -CO-NR²-, -NR²-CO-, -NR²-C(O)-NR²-, -S(O)₂-NR²-, -NR²-S(O)₂-, -CR²=CR³-, -C≡C-, -N=CR²- ou -R²C=N-, qui peuvent être substitués chacun une ou plusieurs fois par un groupe alcanediyle en C₁-C₄ ; 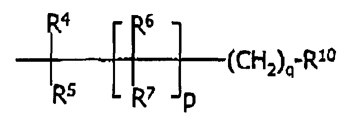
G représente
R¹ représente R²R³N-C(=NR²)-, 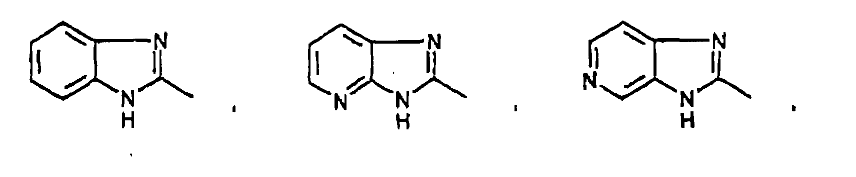 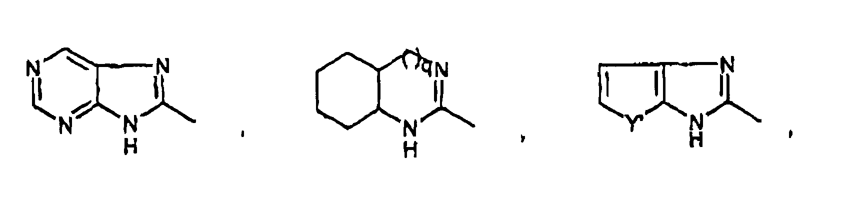 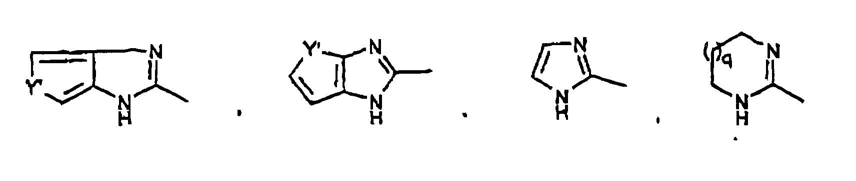 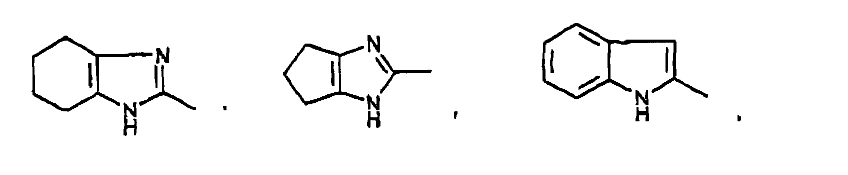 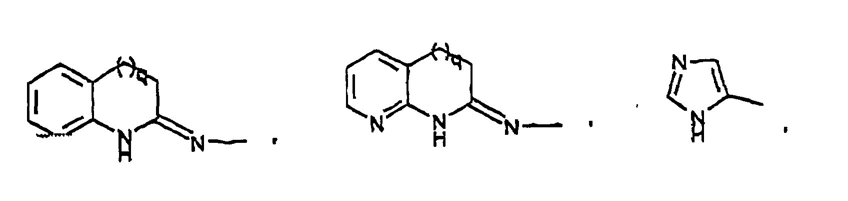 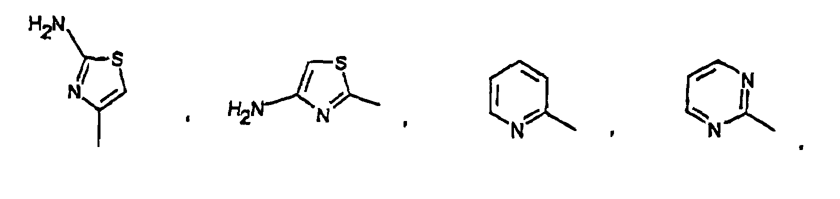 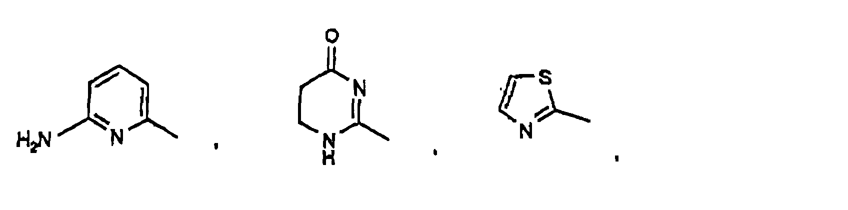 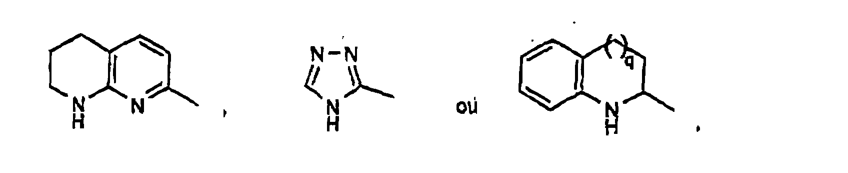
Y' représentant NH, O ou S,
R², R³ représentent, indépendamment l'un de l'autre, H, un groupe alkyle en C₁-C₆, trifluorométhyle, pentafluoroéthyle, cycloalkyle en C₅-C₆, cycloalkyl(C₅-C₆)-alkyle(C₁-C₂), phényle, benzyle, H₂N, R⁸OR⁹, R⁸-aryl(C₅-C₁₀)-R⁹, R⁸NHR⁹, R⁸R⁸NR⁹, R⁸NHC(O)R⁹, H₂N-C(=NH) ou H₂N-C(=NH)-NH- ;
R⁴, R⁵, R⁶, R⁷ représentent, indépendamment les uns des autres, un atome d'hydrogène ou de fluor, OH, un groupe alkyle en C₁-C₆, cycloalkyle en C₁₀-C₁₂, cycloalkyl (C₁₀-C₁₂)-alkyle(C₁-C₆), R⁸OR⁹, R⁸-(C₅-C₁₀)-aryl-R⁹, R⁸R⁸NR⁹, R⁸NHC(O)OR⁹, R⁸S(O)ₙNHR⁹, R⁸OC(O)NHR⁹ ou R⁸C(O)NHR⁹ ;
R⁸ représente H, un groupe alkyle en C₁-C₆, cycloalkyle en C₁₀-C₁₂, cycloalkyl (C₁₀-C₁₂)-alkyle (C₁-C₂), aryle en C₅-C₁₀ ou aryl (C₅-C₁₀)-alkyle (C₁-C₂) ;
R⁹ représente une liaison directe ou un groupe alcanediyle en C₁-C₆ ;
R¹⁰ représente C(O)R¹¹ ;
R¹¹ représente un groupe OH, alcoxy en C₁-C₆, phénoxy, benzyloxy, alkyl(C₁-C₄)-carbonyloxyalcoxy(C₁-C₄), NH₂, mono- ou dialkyl(C₁-C₆)amino ;
R¹⁶ représente H, un groupe alkyle en C₁-C₄, trifluorométhyle, pentafluoroéthyle, cycloalkyle en C₅-C₆, cycloalkyl(C₅-C₆)-alkyle(C₁-C₂), phényle ou benzyle ;
n est 1 ou 2 ; et
p, q représentent, indépendamment l'un de l'autre, 0 ou 1 ;
et leurs sels physiologiquement acceptables,
à l'exclusion des composés dans lesquels R¹-A-B-D-C(R¹⁶) est R¹-K-C(R¹⁶), en ce cas,
R¹ représentant un groupe X-NH-C(=NH)-(CH₂)ₚ, X¹-NH-(CH₂)ₚ ou 4-imidazolyl-CH₂-, p pouvant être un nombre entier allant de 0 à 3,
x représentant un atome d'hydrogène ou un groupe alkyle en C₁-C₆, hydroxy, alcoxy en C₁-C₆, aryl(C₆-C₁₄)-alcoxy(C₁-C₆) ou amino, les groupes aryle dans X représentant des carbocycles purs, éventuellement une ou plusieurs fois substitués ;
X¹ représentant un groupe aryl(C₄-C₁₄)-alcoxy(C₁-C₆) ou R'-NH-C(=N-R-"), R' et R" ayant indépendamment l'un de l'autre les significations de X et les groupes aryle dans X' représentant des carbocycles purs, éventuellement une où plusieurs fois substitués ;
K représentant un groupe alcanediyle en C₁-C₆, cyclohexanediyle, phénylène, phénylènealcanediyle(C₁-C₆), alcanediyl(C₁-C₆)-phénylène, phénylène-alcènediyl (C₂-C₆) ou un reste bivalent d'un cycle à 6 chaînons, saturé ou insaturé, qui peut contenir 1 atome d'azote et peut être une ou deux fois substitué par un groupe alkyle en C₁-C₆ ou un atome d'oxygène ou de soufre doublement lié.

2. Composé de formule I selon la revendication 1, dans lequel F représente -CO-NR²-, et sels physiologiquement acceptables d'un tel composé.

3. Composé de formule I selon la revendication 1 et/ou la revendication 2, dans lequel G représente 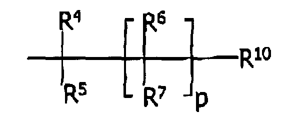 et sels physiologiquement acceptables d'un tel composé.

4. Procédé pour la préparation d'un composé de formule I selon les revendications 1 à 3, dans lequel F représente -C(O)-NR²-, **caractérisé en ce qu'**on condense un composé de formule II 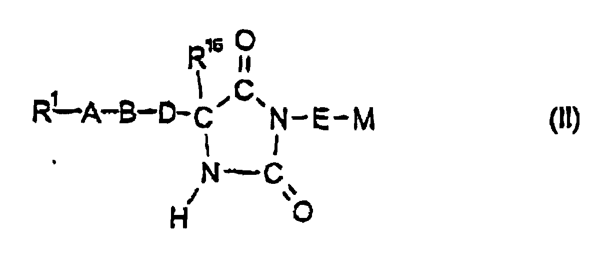 avec un composé de formule HNR²-G, A, B, D, R¹, R¹⁶, E et G ayant les significations données dans les revendications 1 à 3, ou des groupes fonctionnels pouvant également être présents sous forme protégée, et M représente ' un groupe hydroxycarbonyle, alcoxy(C₁-C₆)carbonyle ou un dérivé activé d'acide carboxylique.

5. Composé de formule I selon les revendications 1 à 3, ou sel physiologiquement acceptable de celui-ci, pour utilisation en tant que médicament.

6. Composé de formule I selon les revendications 1 à 3, ou sel physiologiquement acceptable de celui-ci, en tant qu'inhibiteur de la résorption osseuse par les ostéoclastes, en tant qu'inhibiteur de la croissance tumorale ou de la dissémination de métastases, en tant qu'anti-inflammatoire, pour le traitement ou la prophylaxie de maladies cardiovasculaires, pour le traitement ou la prophylaxie de néphropathies ou de rétinopathies, ou en tant qu'antagoniste des récepteurs de la vitronectine, pour le traitement et la prophylaxie de maladies qui reposent sur l'interaction entre les récepteurs de la vitronectine et leurs ligands dans des processus d'interaction cellule-cellule ou cellule-matrice.

7. Préparation pharmaceutique contenant au moins un composé de formule I selon les revendications 1 à 3, ou un sel physiologiquement acceptable de celui-ci, en plus de véhicules et/ou d'additifs pharmaceutiquement acceptables.
